# EUROPEAN PATENT APPLICATION

(11) **EP 3 885 365 A1**
(43) Date of publication of application: **29.09.2021**
(21) Application number: 19886429.0
(22) Date of filing: 19.11.2019
(51) Int. Cl.: C07K 16/28, C12N 15/13, C12N 15/85, A61K 39/395, A61P 35/00, A61P 37/00

(54) **OX40 ANTIBODY, PREPARATION METHOD THEREOF AND USE THEREOF**

(30) Priority: 20.11.2018 CN 201811386274
(71) Applicant: Shanghai Pharmaexplorer Co., Ltd., Shanghai 201210 (CN); Pharmaexplorer Limited, Road Town, Tortola (VG)
(72) Inventor: CHAI, Xiaojuan, Shanghai 201210 (CN); XIN, Wenfang, Shanghai 201210 (CN); CAO, Yanyun, Shanghai 201210 (CN); XU, Hui, Shanghai 201210 (CN); ZHANG, Yu, Shanghai 201210 (CN); XIA, Ke, Shanghai 201210 (CN); DUAN, Qing, Shanghai 201210 (CN); YANG, Tatchi Teddy, Shanghai 201210 (CN); LIU, Lile, Shanghai 201210 (CN)
(74) Representative: karo IP
(86) International application number: PCT/CN2019/119515
(87) International publication number: WO 2020/103836

(57) **Abstract**

Provided are a murine or human-mouse chimeric antibody targeting OX40 and a preparation method therefor, wherein the antibody binds to the OX40 antigen and has an anti-tumor activity and other activities.

## Description

### Technical field

The present invention belongs to the field of biomedicine, in particular to an OX40 antibody, preparation method thereof and use thereof.

### BACKGROUND

OX40 (also known as CD134, TNFRSF4 or ACT35) is a tumor necrosis factor receptor superfamily (TNFRSF) member and a type I transmembrane glycoprotein. OX40 is mainly expressed in activated CD4+ T cell and CD8+ T cell, regulatory T cell (Treg) and natural killer cell (NK). The ligand of OX40 in the body is OX40L (also known as CD152 or TNFSF4), which exists as a trimer. OX40/OX40L signal plays a very important role in the activation and proliferation of T cells. Studies have shown that OX40 antibody can effectively promote the activation and proliferation of T cells, block the immunosuppressive effect of Treg cells, and activate the immune system.

Clinical sample studies have found that OX40 is expressed in tumor infiltrating lymphocytes (TILs), such as breast cancer, melanoma, B-cell lymphoma, head and neck cancer, and colon cancer.

In mice tumor models such as CT26 colon cancer, N2C breast cancer, A20 lymphoma, and B16-F10 melanoma, the mouse OX40 antibody (OX86) can effectively control the growth of tumors and improve the survival rate of mice. In addition, the combination of OX40 antibody and other immune checkpoint-related antibodies, such as 4-1BB antibody, PD-L1 antibody or CTLA-4 antibody, has good anti-tumor activity and can significantly improve the survival rate. Therefore, the development of OX40 antibody has very important clinical significance.

At present, there is no better OX40 antibody that can properly regulate the immune system and be used in tumor immunotherapy, and achieves the effect of high antibody titer and low toxic side effects. Therefore, it is urgent to solve the above problems.

### Summary of the Invention

In order to overcome the shortcomings of current lack of superior OX40 antibodies, the present invention provides an OX40 antibody with high affinity and strong specificity, and a preparation method thereof and use thereof.

In a first aspect of the present invention, it provides a heavy chain variable region of an antibody having a complementarity determining region CDR selected from the group consisting of:
a VH-CDR1 as shown in SEQ ID NO.10n+3,
a VH-CDR2 as shown in SEQ ID NO.10n+4, and
a VH-CDR3 as shown in SEQ ID NO.10n+5;
wherein each n is independently 0, 1, 2, 3, 4, 5, 6, 7, 8 or 9;
wherein any one of the above amino acid sequences further includes a derivative sequence that is optionally added, deleted, modified, and/or substituted for at least one amino acid and can retain the binding affinity of OX40.

In another preferred embodiment, the heavy chain variable region has the amino acid sequence as shown in SEQ ID NO. 101 or 10n+1, wherein n is 0, 1, 2, 3, 4, 5, 6, 7, 8, or 9.

In another preferred embodiment, the heavy chain variable region has the amino acid sequence as shown in SEQ ID NO.101.

In another preferred embodiment, the heavy chain variable region has the amino acid sequence as shown in SEQ ID NO.1.

In another preferred embodiment, the heavy chain variable region has the amino acid sequence as shown in SEQ ID NO.11.

In another preferred embodiment, the heavy chain variable region has the amino acid sequence as shown in SEQ ID NO.21.

In another preferred embodiment, the heavy chain variable region has the amino acid sequence as shown in SEQ ID NO.31.

In a second aspect of the present invention, it provides a heavy chain of an antibody having the heavy chain variable region of claim 1.

In another preferred embodiment, the heavy chain further comprises a heavy chain constant region.

In another preferred embodiment, the heavy chain constant region is of human or murine origin.

In another preferred embodiment, the heavy chain constant region is a human antibody heavy chain IgG1 constant region.

In a third aspect of the present invention, it provides a light chain variable region of an antibody having a complementarity determining region CDR selected from the group consisting of:
a VL-CDR1 as shown in SEQ ID NO.10n+8,
a VL-CDR2 as shown in SEQ ID NO.10n+9, and
a VL-CDR3 as shown in SEQ ID NO.10n+10;
wherein each n is independently 0, 1, 2, 3, 4, 5, 6, 7, 8 or 9;
wherein any one of the above amino acid sequences further includes a derivative sequence that is optionally added, deleted, modified, and/or substituted for at least one amino acid and can retain the binding affinity of OX40.

In another preferred embodiment, the light chain variable region has the amino acid sequence as shown in SEQ ID NO. 103 or 10n+6, wherein n is 0, 1, 2, 3, 4, 5, 6, 7, 8, or 9.

In another preferred embodiment, the light chain variable region has the amino acid sequence as shown in SEQ ID NO.103.

In another preferred embodiment, the light chain variable region has the amino acid sequence as shown in SEQ ID NO.6.

In another preferred embodiment, the light chain variable region has the amino acid sequence as shown in SEQ ID NO.16.

In another preferred embodiment, the light chain variable region has the amino acid sequence as shown in SEQ ID NO.26.

In another preferred embodiment, the light chain variable region has the amino acid sequence as shown in SEQ ID NO.36.

In a fourth aspect of the present invention, it provides a light chain of an antibody having the light chain variable region of claim 3.

In another preferred embodiment, the light chain further comprises a light chain constant region.

In another preferred embodiment, the light chain constant region is of human or murine origin.

In another preferred embodiment, the light chain constant region is a human antibody light chain kappa constant region.

In a fifth aspect of the present invention, it provides an antibody having:
(1) a heavy chain variable region according to the first aspect of the present invention; and/or
(2) a light chain variable region according to the third aspect of the present invention;
or the antibody has the heavy chain according to the second aspect of the present invention; and/or the light chain according to the fourth aspect of the present invention,
wherein any one of the above amino acid sequences further includes a derivative sequence that is optionally added, deleted, modified, and/or substituted for at least one amino acid and can retain the binding affinity of OX40.

In another preferred embodiment, the amino acid sequence of any of the above-mentioned CDRs contains a derivative CDR sequence of 1, 2, or 3 amino acids that has been added, deleted, modified, and/or substituted, and so that the derivative antibody composed of VH and VL containing the derivative CDR sequence can retain the affinity for binding to OX40.

In another preferred embodiment, the ratio (F1/F0) of the affinity F1 of the derivatized antibody binding to OX40 and the affinity F0 of the corresponding non-derivatized antibody binding to OX40 is 0.5-2, preferably 0.7-1.5, and more preferably 0.8-1.2 .

In another preferred embodiment, the number of added, deleted, modified and/or substituted amino acids is 1-5 (such as 1-3, preferably 1-2, more preferably 1).

In another preferred embodiment, the derivative sequence that has been added, deleted, modified and/or substituted at least one amino acid and can retain OX40 binding affinity is an amino acid sequence with homology or sequence identity of at least 96%.

In another preferred embodiment, the antibody further comprises a heavy chain constant region and/or light chain constant region.

In another preferred embodiment, the heavy chain constant region is of human origin, and/or the light chain constant region is of human origin.

In another preferred embodiment, the heavy chain constant region is a human antibody heavy chain IgG1 constant region, and the light chain constant region is a human antibody light chain kappa constant region.

In another preferred embodiment, the heavy chain variable region of the antibody further comprises a human framework region, and/or the light chain variable region of the antibody further comprises a human framework region.

In another preferred embodiment, the heavy chain variable region of the antibody further comprises a framework region of murine origin, and/or the light chain variable region of the antibody further comprises a framework region of murine origin.

In another preferred embodiment, the antibody is selected from the group consisting of an animal-derived antibody, chimeric antibody, humanized antibody, fully human antibody, and a combination thereof.

In another preferred embodiment, the ratio (Z1/Z0) of the immunogenicity Z1 of the chimeric antibody in humans and the immunogenicity Z0 of non-chimeric antibody (such as murine antibodies) in humans is 0-0.5, preferably 0 -0.2, more preferably 0-0.05 (e.g., 0.001-0.05).

In another preferred embodiment, the antibody is a partially or fully humanized, or fully human monoclonal antibody.

In another preferred embodiment, the antibody is a double-chain antibody or a single-chain antibody.

In another preferred embodiment, the antibody is a full-length protein of an antibody, or an antigen binding fragment.

In another preferred embodiment, the antibody is a bispecific antibody or a multispecific antibody.

In another preferred embodiment, the antibody is in the form of a drug conjugate.

In another preferred embodiment, the antibody has one or more characteristics selected from the group consisting of:
(a) inhibiting the migration or metastasis of tumor cells;
(b) inhibiting tumor growth.

In another preferred embodiment, the antibody has a heavy chain variable region according to the first aspect of the present invention and a light chain variable region according to the third aspect of the present invention;
wherein the heavy chain variable region and the light chain variable region comprise a CDR selected from the group consisting of:

| VH-CDR1 Sequence Number | VH-CDR2 Sequence Number | VH-CDR3 Sequence Number | VL-CDR1 Sequence Number | VL-CDR2 Sequence Number | VL-CDR3 Sequence Number |
|---|---|---|---|---|---|
| 3 | 4 | 5 | 8 | 9 | 10 |
| 13 | 14 | 15 | 18 | 19 | 20 |
| 23 | 24 | 25 | 28 | 29 | 30 |
| 33 | 34 | 35 | 38 | 39 | 40 |
| 43 | 44 | 45 | 48 | 49 | 50 |
| 53 | 54 | 55 | 58 | 59 | 60 |
| 63 | 64 | 65 | 68 | 69 | 70 |
| 73 | 74 | 75 | 78 | 79 | 80 |
| 83 | 84 | 85 | 88 | 89 | 90 |
| 93 | 94 | 95 | 98 | 99 | 100 |

wherein any one of the above amino acid sequences also includes a derivative sequence that is optionally added, deleted, modified, and/or substituted for at least one amino acid and can retain the binding affinity of OX40.

In another preferred embodiment, the antibody has a heavy chain variable region according to the first aspect of the present invention and a light chain variable region according to the third aspect of the present invention;

Wherein the heavy chain variable region comprises the following three complementarity determining region CDRs:
a VH-CDR1 as shown in SEQ ID NO.3,
a VH-CDR2 as shown in SEQ ID NO. 4, and
a VH-CDR3 as shown in SEQ ID NO.5;
the light chain variable region comprises the following three complementarity determining region CDRs:
   a VL-CDR1 as shown in SEQ ID NO. 8,
   a VL-CDR2 as shown in SEQ ID NO. 9, and
   a VL-CDR3 as shown in SEQ ID NO. 10; or
   the heavy chain variable region comprises the following three complementarity determining region CDRs:
      a VH-CDR1 as shown in SEQ ID NO. 13,
      a VH-CDR2 as shown in SEQ ID NO. 14, and
      a VH-CDR3 as shown in SEQ ID NO.15;

The light chain variable region comprises the following three complementarity determining region CDRs:
a VL-CDR1 as shown in SEQ ID NO. 18,
a VL-CDR2 as shown in SEQ ID NO. 19, and
a VL-CDR3 as shown in SEQ ID NO. 20; or
the heavy chain variable region comprises the following three complementarity determining region CDRs:
   a VH-CDR1 as shown in SEQ ID NO.23,
   a VH-CDR2 as shown in SEQ ID NO. 24, and
   a VH-CDR3 as shown in SEQ ID NO.25;
   the light chain variable region comprises the following three complementarity determining region CDRs:
      a VL-CDR1 as shown in SEQ ID NO. 28,
      a VL-CDR2 as shown in SEQ ID NO. 29, and
      a VL-CDR3 as shown in SEQ ID NO. 30; or
      the heavy chain variable region comprises the following three complementarity determining region CDRs:
         a VH-CDR1 as shown in SEQ ID NO.33,
         a VH-CDR2 as shown in SEQ ID NO. 34, and
         a VH-CDR3 as shown in SEQ ID NO.35;

The light chain variable region comprises the following three complementarity determining region CDRs:
a VL-CDR1 as shown in SEQ ID NO. 38,
a VL-CDR2 as shown in SEQ ID NO. 39, and
a VL-CDR3 as shown in SEQ ID NO. 40.

In another preferred embodiment, the heavy chain variable region of the antibody contains the amino acid sequence as shown in SEQ ID NO. 101, 1, 11, or 21; and/or the light chain variable region of the antibody contains the amino acid sequence as shown in SEQ ID NO. 103, 6, 16 or 26.

In another preferred embodiment, the heavy chain variable region of the antibody comprises the amino acid sequence as shown in SEQ ID NO. 101; and the light chain variable region of the antibody comprises the amino acid sequence as shown in SEQ ID NO. 103.

In another preferred embodiment, the heavy chain variable region of the antibody comprises the amino acid sequence as shown in SEQ ID NO. 1; and the light chain variable region of the antibody comprises the amino acid sequence as shown in SEQ ID NO. 6.

In another preferred embodiment, the heavy chain variable region of the antibody comprises the amino acid sequence as shown in SEQ ID NO. 11; and the light chain variable region of the antibody comprises the amino acid sequence as shown in SEQ ID NO. 16.

In another preferred embodiment, the heavy chain variable region of the antibody comprises the amino acid sequence as shown in SEQ ID NO. 21; and the light chain variable region of the antibody comprises the amino acid sequence as shown in SEQ ID NO. 26.

In another preferred embodiment, the heavy chain variable region of the antibody comprises the amino acid sequence as shown in SEQ ID NO.31; and the light chain variable region of the antibody comprises the amino acid sequence as shown in SEQ ID NO.36.

In another preferred embodiment, the antibody is selected from the group consisting of:

| Antibody number | Clone | VH sequence Number | VL Sequence Number |
|---|---|---|---|
| 1 | Humab035-24 | 101 | 103 |
| 2 | Mab035 | 1 | 6 |
| 3 | Mab053 | 11 | 16 |
| 4 | Mab041 | 21 | 26 |
| 5 | Mab058 | 31 | 36 |
| 6 | Mab034 | 41 | 46 |
| 7 | Mab049 | 51 | 56 |
| 8 | Mab064 | 61 | 66 |
| 9 | Mab033 | 71 | 76 |
| 10 | Mab047 | 81 | 86 |
| 11 | Mab055 | 91 | 96 |

In another preferred embodiment, the amino acid sequence of the variable region of the heavy chain has at least 80%, 85%, 90%, 91%, 92%, 93%, 94%, 95%, 96%, 97%, 98% or 99% sequence homology or sequence identity with the amino acid sequence as shown in SEQ ID NO. 101, 1, 11 or 21 in the sequence listing.

In another preferred embodiment, the amino acid sequence of the light chain variable region has at least 80%, 85%, 90%, 91%, 92%, 93%, 94%, 95%, 96%, 97%, 98% or 99% sequence homology or sequence identity with the amino acid sequence as shown in SEQ ID NO. 103, 6, 16 or 26 in the sequence listing.

In a sixth aspect of the present invention, it provides a recombinant protein comprising:
(i) a heavy chain variable region according to the first aspect of the present invention, a heavy chain according to the second aspect of the present invention, a light chain variable region according to the third aspect of the present invention, a light chain according to the fourth aspect of the present invention, or an antibody according to the fifth aspect of the present invention; and
(ii) an optional tag sequence to assist in expression and/or purification.

In another preferred embodiment, the tag sequence comprises 6His tag.

In another preferred embodiment, the recombinant protein (or polypeptide) comprises a fusion protein.

In another preferred embodiment, the recombinant protein is a monomer, dimer, or multimer.

In another preferred embodiment, the recombinant protein comprises:
(i) an antibody selected from the group consisting of,

| Antibody number | Clone | VH Sequence Number | VL Sequence Number |
|---|---|---|---|
| 1 | Humab035-24 | 101 | 103 |
| 2 | Mab035 | 1 | 6 |
| 3 | Mab053 | 11 | 16 |
| 4 | Mab041 | 21 | 26 |
| 5 | Mab058 | 31 | 36 |
| 6 | Mab034 | 41 | 46 |
| 7 | Mab049 | 51 | 56 |
| 8 | Mab064 | 61 | 66 |
| 9 | Mab033 | 71 | 76 |
| 10 | Mab047 | 81 | 86 |
| 11 | Mab055 | 91 | 96 |

and
(ii) an optional tag sequence to assist in expression and/or purification.

In a seventh aspect of the present invention, it provides a polynucleotide encoding a polypeptide selected from the group consisting of:
(1) a heavy chain variable region according to the first aspect of the present invention, a heavy chain according to the second aspect of the present invention, a light chain variable region according to the third aspect of the present invention, a light chain according to the fourth aspect of the present invention, or an antibody according to the fifth aspect of the present invention; and
(2) the recombinant protein according to the sixth aspect of the present invention.

In another preferred embodiment, the polynucleotide encoding the variable region of the heavy chain is shown in SEQ ID NO. 102, 2, 12, 22, 32, 42, 52, 62, 72, 82, or 92; and/or, the polynucleotide encoding the variable region of the light chain is shown in SEQ ID NO. 104, 7, 17, 27, 37, 47, 57, 67, 77, 87 or 97.

In another preferred embodiment, the polynucleotide encoding the heavy chain variable region sequence and the polynucleotide encoding the light chain variable region sequence are selected from the group consisting of:

| Clone | Sequence numbering of polynucleotide encoding VH | Sequence numbering of polynucleotide encoding VL |
|---|---|---|
| Humab035-24 | 102 | 104 |
| Mab035 | 2 | 7 |
| Mab053 | 12 | 17 |
| Mab041 | 22 | 27 |
| Mab058 | 32 | 37 |
| Mab034 | 42 | 47 |
| Mab049 | 52 | 57 |
| Mab064 | 62 | 67 |
| Mab033 | 72 | 77 |
| Mab047 | 82 | 87 |
| Mab055 | 92 | 97 |

In an eighth aspect of the present invention, it provides a vector comprising the polynucleotide according to any one of the seventh aspects of the present invention.

In another preferred embodiment, the vector includes: a bacterial plasmid, bacteriophage, yeast plasmid, plant cell virus, mammalian cell virus such as adenovirus, retrovirus, or other vectors.

In a ninth aspect of the present invention, it provides a genetically engineered host cell comprising the vector according to the eighth aspect of the present invention or the genome integrated with the polynucleotide according to the seventh aspect of the present invention.

In a tenth aspect of the present invention, it provides an antibody conjugate comprising:
(a) an antibody portion, which is selected from the group consisting of a heavy chain variable region according to the first aspect of the present invention, a heavy chain according to the second aspect of the present invention, a light chain variable region according to the third aspect of the present invention, a light chain according to the fourth aspect of the present invention, or an antibody according to the fifth aspect of the present invention, and a combination thereof; and
(b) a coupling portion coupled to the antibody portion, the coupling portion is selected from the group consisting of a detectable marker, drug, toxin, cytokine, radionuclide, enzyme, and a combination thereof.

In another preferred embodiment, the antibody portion is coupled to the coupling portion by a chemical bond or linker.

In an eleventh aspect of the present invention, it provides an immune cell that expresses or is exposed outside the cell membrane with the antibody according to the fifth aspect of the present invention.

In another preferred embodiment, the immune cell comprises a NK cell, T cell.

In another preferred embodiment, the immune cell is derived from human or non-human mammals (such as mice).

In a twelfth aspect of the present invention, it provides a pharmaceutical composition comprising:
(i) an active ingredient, the active ingredient is selected from the group consisting of: the heavy chain variable region according to the first aspect of the present invention, the heavy chain according to the second aspect of the present invention, the light chain variable region according to the third aspect of the present invention, the light chain according to the fourth aspect of the present invention, or the antibody according to the fifth aspect of the present invention, the recombinant protein according to the sixth aspect of the present invention, the antibody conjugate according to the tenth aspect of the present invention, the immune cell according to the eleventh aspect of the present invention, and a combination thereof; and
(ii) a pharmaceutically acceptable carrier.

In another preferred embodiment, the pharmaceutical composition is a liquid formulation.

In another preferred embodiment, the pharmaceutical composition is an injection.

In another preferred embodiment, the pharmaceutical composition comprises 0.01-99.99% of the antibody according to the fifth aspect of the present invention, the recombinant protein according to the sixth aspect of the present invention, and the antibody conjugate according to the tenth aspect of the present invention, the immune cell according to the eleventh aspect of the present invention, and a combination thereof, and 0.01-99.99% of the pharmaceutical carrier, and the percentage is the mass percentage of the pharmaceutical composition.

In a thirteenth aspect of the present invention, it provides a use of an active ingredient selected from the group consisting of the heavy chain variable region according to the first aspect of the present invention, and the heavy chain according to the second aspect of the present invention, the light chain variable region according to the third aspect of the present invention, the light chain according to the fourth aspect of the present invention, or the antibody according to the fifth aspect of the present invention, the recombinant protein according to the sixth aspect of the present invention, the antibody conjugate according to the tenth aspect of the present invention, the immune cell according to the eleventh aspect of the present invention, and a combination thereof, wherein the active ingredient is used for (a) preparing a diagnostic reagent or kit; and/or (b) preparing a drug for preventing and/or treating diseases related to abnormal expression or function of OX40.

In another preferred embodiment, the diagnostic reagent is a detective slip or a detection plate.

In another preferred embodiment, the diseases related to the abnormal expression or function of OX40 are selected from the group consisting of: cancer, autoimmune diseases.

In another preferred embodiment, the cancer is selected from the group consisting of: breast cancer, melanoma, B-cell lymphoma, head and neck cancer, colon cancer, prostate cancer, lung cancer.

In another preferred embodiment, the diagnostic reagent or kit is used for:
(1) detecting the OX40 protein in a sample; and/or
(2) detecting endogenous OX40 protein in a tumor cell; and/or
(3) detecting a tumor cell expressing OX40 protein;
the drug is used to prevent and/or treat diseases related to abnormal expression or function of OX40, and the diseases related to abnormal expression or function of OX40 are selected from the group consisting of: cancer, autoimmune diseases.

In another preferred embodiment, the cancer is selected from the group consisting of: breast cancer, melanoma, B-cell lymphoma, head and neck cancer, colon cancer, prostate cancer, lung cancer.

In another preferred embodiment, the antibody is in the form of a drug conjugate (ADC) .

In another preferred embodiment, the diagnostic reagent or kit is used for diagnosing OX40 related diseases.

In another preferred embodiment, the diagnostic reagent or kit is used for detecting OX40 protein in a sample.

In a fourteenth aspect of the present invention, it provides a method for detecting (including diagnostic or non-diagnostic) OX40 protein in a sample in vitro, the method comprising the steps:
(1) in vitro, contacting the sample with the antibody according to the fifth aspect of the present invention;
(2) detecting the formation of an antigen-antibody complex, wherein the formation of a complex indicates the presence of OX40 protein in the sample.

In a fifteenth aspect of the present invention, it provides a composition for detecting OX40 protein in a sample in vitro, which comprises the antibody according to the fifth aspect of the present invention, the recombinant protein according to the sixth aspect of the present invention, the antibody conjugate according to the tenth aspect of the present invention, the immune cell according to the eleventh aspect of the present invention, and a combination thereof as an active ingredient.

In a sixteenth aspect of the present invention, it provides a detection plate comprising a substrate (support plate) and a test strip, and the test strip contains the antibody according to the fifth aspect of the present invention, the recombinant protein according to the sixth aspect of the present invention, and the antibody conjugate according to the tenth aspect of the present invention, the immune cell according to the eleventh aspect of the present invention, and a combination thereof.

In a seventeenth aspect of the present invention, it provides a kit comprising:
(1) a first container containing the antibody of the present invention; and/or
(2) a second container containing a secondary antibody against the antibody of the present invention;
   or,
The kit contains the detection plate according to the sixteenth aspect of the present invention.

In an eighteenth aspect of the present invention, it provides a method for preparing a recombinant polypeptide, the method comprising:
(a) culturing the host cell according to the ninth aspect of the present invention under conditions suitable for expression;
(b) isolating the recombinant polypeptide from the culture, the recombinant polypeptide being an antibody according to the fifth aspect of the present invention or a recombinant protein according to the sixth aspect of the present invention.

In a nineteenth aspect of the present invention, it provides a pharmaceutical combination comprising:
(I) a first active ingredient comprising the antibody 1 according to the fifth aspect of the present invention, or the recombinant protein according to the sixth aspect of the present invention, or the antibody conjugate according to the tenth aspect of the present invention, or the immune cell according to the eleventh aspect of the present invention, or the pharmaceutical composition according to the twelfth aspect of the present invention, and a combination thereof;
(ii) a second active ingredient comprising a second antibody, or a chemotherapeutic agent.

In another preferred embodiment, the second antibody is selected from the group consisting of: CTLA4 antibody, PD-1 antibody, PD-L1 antibody, 4-1BB antibody.

In another preferred embodiment, the chemotherapeutic agent is selected from the group consisting of docetaxel, carboplatin, and a combination thereof.

In a twentieth aspect of the present invention, it provides a use of the combination of the antibody of the fifth aspect of the present invention, or the recombinant protein according to the sixth aspect of the present invention, or the antibody conjugate according to the tenth aspect of the present invention, or the immune cell according to the eleventh aspect of the present invention, and/or the pharmaceutical composition according to the twelfth aspect of the present invention and a second antibody or a chemotherapeutic agent in the preparation of a medicament for treating diseases related to abnormal OX40 expression or function.

In another preferred embodiment, the second antibody is selected from the group consisting of: CTLA4 antibody, PD-1 antibody, PD-L1 antibody, 4-1BB antibody.

In a twenty-first aspect of the present invention, it provides a method for treating diseases related to abnormal expression or function of OX40, administering to a subject in need an effective amount of the antibody according to the fifth aspect of the present invention, or the recombinant protein according to the sixth aspect of the present invention, or the antibody conjugate according to the tenth aspect of the present invention, or the immune cell according to the eleventh aspect of the present invention, or the pharmaceutical composition according to the twelfth aspect of the present invention, and a combination thereof.

In another preferred embodiment, the diseases related to the abnormal expression or function of OX40 are selected from the group consisting of cancer and autoimmune diseases.

In another preferred embodiment, the cancer is selected from the group consisting of: breast cancer, melanoma, B-cell lymphoma, head and neck cancer, colon cancer, prostate cancer, lung cancer.

In another preferred embodiment, the method further comprises before, during, and/or after the administration of the first active ingredient, a safe and effective amount of the second antibody is administered to the subject.

In another preferred embodiment, the second antibody is selected from the group consisting of: PD-1 antibody, CTLA4 antibody, PD-L1 antibody, 4-1BB antibody.

It should be understood that, within the scope of the present invention, the technical features specifically described above and below (such as the Examples) can be combined with each other, thereby constituting a new or preferred technical solution which needs not be described one by one.

### Description of Drawings

Figure 1 shows the binding activity of OX40 ECD-hFc and OX40L protein. Wherein The X-axis represents the concentration of OX40L protein, and the Y-axis represents the OD450nm value.
Figure2 shows the results of FACS analysis of the HEK293-hOX40 cell line.
Figure 3 shows the results of FACS analysis of the CHOK1-hOX40 cell line.
Figure 4 shows the results of FACS analysis of the CHOK1-cOX40 cell line.
Figure 5 shows the result of ELISA detection of the serum antibody titer of mice after immunization with OX40 ECD-hFc protein.
Figure 6 shows the results of FACS detection of the serum antibody titer of mice after immunization with OX40 ECD-hFc protein.
Figure 7 shows the results of FACS detection of antibody titer in mouse serum after HEK293-hOX40 cell immunization.
Figure 8 shows the results of FACS detection of antibody titer in mouse serum after pCP-hOX40 plasmid immunization.
Figures 9a and 9b show the FACS detection of the binding reaction between OX40 antibody and CHOK1-hOX40.
Figures 10a and 10b show the FACS detection of the binding reaction between OX40 antibody and CHOK1-cOX40.
Figure 11 shows the FACS detection of hOX40 expression in Jurkat-NF-kB Luc-hOX40 cells.
Figure 12 shows the luciferase method to detect the expression of NF-kB RE-luciferase in Jurkat-NF-kB Luc-hOX40 cells.
Figures 13a and 13b show the NF-kB reporter gene experiment to detect the activation of the OX40 antibody on the NF-kB signaling pathway, wherein blank is a blank control; Ionomycin is ionomycin (IONO).
Figure 14 shows the results of FACS analysis of the CHOK1-hOX40L cell line.
Figures 15a and 15b show the FACS detection of the inhibitory effect of OX40 antibody on the binding of OX40 and OX40L proteins.
Figure 16 shows the FACS detection of the binding reaction of OX40 human-mouse chimeric antibody and CHOK1-hOX40.
Figure 17 shows the FACS detection of the binding reaction of OX40 human-mouse chimeric antibody and CHOK1-cOX40.
Figure 18 shows the NF-kB reporter gene experiment to detect the activation of the OX40 human-mouse chimeric antibody on the NF-kB signaling pathway.
Figures 19a and 19b show the body weight changes of mice in each group after administration.
Figure 20 shows the changes in tumor volume of mice in each group after administration.
Figure 21 shows the survival curve of mice in each group after administration.
Figure 22 shows the FACS detection of the binding reaction of OX40 humanized antibody with CHOK1-hOX40.
Figure 23 shows the NF-kB reporter gene experiment to detect the activation of the OX40 humanized antibody on the NF-kB signaling pathway.
Figure 24 is a T cell activation test to detect the activation of OX40 humanized antibody on memory CD4 positive T cells.

### DETAILED DESCRIPTION

Through extensive and in-depth research, the inventors of the present invention have used human OX40 protein, cell lines stably expressing OX40 protein, and plasmids containing cDNA encoding OX40 protein as immunogens. Using hybridoma technology, they have unexpectedly obtained a group of mouse-derived or human-mouse chimeric OX40 antibodies with a completely new amino acid sequence (for example, mab035, mab053, mab041, and mab058). The OX40 antibody of the present invention is an OX40 agonistic antibody with excellent biological characteristics. It has high affinity with human OX40 protein and has similar binding ability with monkey OX40 protein; it can activate NF-kB signal pathway; It can significantly increase the expression of IFN gamma in memory CD4+ T cells, activate the immune system, and prolong the life cycle of tumor mice, so that it can be used in the treatment of tumors. The antibody of the present invention can be used in the preparation of drugs for treating tumors, autoimmune diseases and the like. The present invention has been completed on this basis.

### Terms

In the present invention, "VH-CDR1" and "CDR-H1" can be used interchangeably, both refer to the CDR1 of the variable region of the heavy chain; "VH-CDR2" and "CDR-H2" can be used interchangeably, both refer to the CDR2 of the variable region of the heavy chain; "VH-CDR3" and "CDR-H3" can be used interchangeably, both refer to the CDR3 of the variable region of the heavy chain. "VL-CDR1" and "CDR-L1" can be used interchangeably, both refer to the CDR1 of the variable region of the light chain; "VL-CDR2" and "CDR-L2" can be used interchangeably, both refer to the CDR2 of the variable region of the light chain; "VL-CDR3" and "CDR-L3" can be used interchangeably, both refer to the CDR3 of the variable region of the light chain.

### Antibody

As used herein, the term "antibody" or "immunoglobulin" is a heterotetrameric glycoprotein of about 150,000 daltons with the same structural characteristics, which is composed of two identical light chains (L) and two identical heavy chains (H) . Each light chain is connected to the heavy chain by a covalent disulfide bond, and the number of disulfide bonds between the heavy chains of different immunoglobulin isotypes is different. Each heavy and light chain also has regularly spaced intrachain disulfide bonds. Each heavy chain has a variable region (VH) at one end, followed by multiple constant regions. Each light chain has a variable region (VL) at one end and a constant region at the other end; the constant region of the light chain is opposite to the first constant region of the heavy chain, and the variable region of the light chain is opposite to the variable region of the heavy chain. Special amino acid residues form an interface between the variable regions of the light and heavy chains.

As used herein, the term "variable" means that certain parts of the variable region of the antibody are different in sequence, which forms the binding and specificity of various specific antibodies to their specific antigens. However, the variability is not evenly distributed throughout the variable regions of antibodies. It is concentrated in three fragments called complementarity determining regions (CDR) or hypervariable regions in the variable regions of the light and heavy chains. The more conserved part of the variable region is called the framework region (FR). The variable regions of the natural heavy chain and light chain each contain four FR regions, which are roughly in a β-folded configuration, connected by three CDRs forming a connecting loop, and in some cases can form a partial β-folded structure. The CDRs in each chain are closely joined together by the FR region and form the antigen binding site of the antibody together with the CDRs of the other chain (see Kabat et al., NIH Publ. No. 91-3242, Volume I, pages 647-669 (1991)). Constant regions do not directly participate in the binding of antibodies to antigens, but they exhibit different effector functions, such as participating in antibody-dependent cytotoxicity of antibodies.

The "light chains" of vertebrate antibodies (immunoglobulins) can be classified into one of two distinct categories (called κ and λ) based on the amino acid sequence of their constant regions. According to the amino acid sequence of the constant region of their heavy chains, immunoglobulins can be divided into different types. There are mainly five classes of immunoglobulins: IgA, IgD, IgE, IgG and IgM, some of which can be further divided into subclasses (isotypes), such as IgG1, IgG2, IgG3, IgG4, IgA and IgA2. The heavy chain constant regions corresponding to different classes of immunoglobulins are called α, δ, ε, γ, and µ, respectively. The subunit structures and three-dimensional configurations of different classes of immunoglobulins are well known to those skilled in the art.

Generally, the antigen-binding properties of antibodies can be described by 3 specific regions located in the variable regions of the heavy and light chains, called variable regions (CDR), which are divided into 4 framework regions (FR), the amino acid sequences of the 4 FRs are relatively conservative and do not directly participate in the binding reaction. These CDRs form a circular structure, and the β - sheet formed by the FRs in between are close to each other in space structure, and the CDRs on the heavy chain and the corresponding CDRs on the light chain constitute the antigen binding site of the antibody. The amino acid sequences of antibodies of the same type can be compared to determine which amino acids constitute the FR or CDR regions.

The invention includes not only complete antibodies, but also fragments of antibodies with immunological activity or fusion proteins formed by antibodies and other sequences. Therefore, the present invention also includes fragments, derivatives and analogues of the antibodies.

In the present invention, antibodies include murine, chimeric, humanized or fully human antibodies prepared by techniques well known to those skilled in the art. Recombinant antibodies, such as chimeric and humanized monoclonal antibodies, including human and non-human parts, can be obtained by standard DNA recombination techniques, and they are all useful antibodies. A chimeric antibody is a molecule in which different parts are derived from different animal species, for example, a chimeric antibody having a variable region from a mouse monoclonal antibody and a constant region from a human immunoglobulin (see, for example, U.S. Patent Nos. 4,816,567 and U.S. Patent No. 4,816,397, which is hereby incorporated by reference in its entirety). Humanized antibodies refer to antibody molecules derived from non-human species, with one or more complementarity determining regions (CDRs) derived from non-human species and framework regions derived from human immunoglobulin molecules (see U.S. Patent 5,585,089, this article is hereby incorporated by reference in its entirety). These chimeric and humanized monoclonal antibodies can be prepared using DNA recombination techniques well known in the art.

In the present invention, antibodies can be monospecific, bispecific, trispecific, or more multispecific.

In the present invention, the antibody of the present invention also includes a conservative variant thereof, which means that compared with the amino acid sequence of the antibody of the present invention, there are at most 10, preferably at most 8, more preferably at most 5, and most preferably at most 3 amino acids are replaced by amino acids with similar or similar properties to form a polypeptide. These conservative variant polypeptides are best produced according to Table 1 through amino acid substitutions.

**Table 1**

| Initial residue | Representative substitution | Preferred substitution |
|---|---|---|
| Ala (A) | Val; Leu; Ile | Val |
| Arg (R) | Lys; Gln; Asn | Lys |
| Asn (N) | Gln; His; Lys; Arg | Gln |
| Asp (D) | Glu | Glu |
| Cys (C) | Ser | Ser |
| Gln (Q) | Asn | Asn |
| Glu (E) | Asp | Asp |
| Gly (G) | Pro; Ala | Ala |
| His (H) | Asn; Gln; Lys; Arg | Arg |
| Ile (I) | Leu; Val; Met; Ala; Phe | Leu |
| Leu (L) | Ile; Val; Met; Ala; Phe | Ile |
| Lys (K) | Arg; Gln; Asn | Arg |
| Met (M) | Leu; Phe; Ile | Leu |
| Phe (F) | Leu; Val; Ile; Ala; Tyr | Leu |
| Pro (P) | Ala | Ala |
| Ser (S) | Thr | Thr |
| Thr (T) | Ser | Ser |
| Trp (W) | Tyr; Phe | Tyr |
| Tyr (Y) | Trp; Phe; Thr; Ser | Phe |
| Val (V) | Ile; Leu; Met; Phe; Ala | Leu |

### Anti-OX40 antibody

In the present invention, the antibody is an anti-OX40 antibody. The present invention provides a high-specificity and high-affinity antibody against OX40, which comprises a heavy chain and a light chain, the heavy chain contains a heavy chain variable region (VH) amino acid sequence, and the light chain contains a light chain variable region (VL) amino acid sequence.

Preferably,
The variable region of the heavy chain (VH) has a complementarity determining region CDR selected from the group consisting of:
The VH-CDR1 as shown in SEQ ID NO.10n+3,
The VH-CDR2 as shown in SEQ ID NO.10n+4, and
The VH-CDR3 as shown in SEQ ID NO.10n+5;
wherein each n is independently 0, 1, 2, 3, 4, 5, 6, 7, 8 or 9;

The variable region of light chain (VL) has a complementarity determining region CDR selected from the group consisting of:
VL-CDR1 as shown in SEQ ID NO.10n+8,
VL-CDR2 as shown in SEQ ID NO.10n+9, and
VL-CDR3 as shown in SEQ ID NO.10n+10;
wherein, each n is independently 0, 1, 2, 3, 4, 5, 6, 7, 8 or 9;
wherein, any one of the above amino acid sequences further includes a derivative sequence that is optionally added, deleted, modified and/or substituted for at least one amino acid and can retain the binding affinity of OX40.

Preferably, the heavy chain variable region (VH) includes the following three complementarity determining region CDRs:
The VH-CDR1 as shown in SEQ ID NO. 10n+3,
The VH-CDR2 as shown in SEQ ID NO. 10n+4, and
The VH-CDR3 as shown in SEQ ID NO. 10n+5;

The light chain variable region (VL) includes the following three complementarity determining region CDRs:
VL-CDR1 as shown in SEQ ID NO. 10n+8,
VL-CDR2 as shown in SEQ ID NO. 10n+9, and
VL-CDR3 as shown in SEQ ID NO. 10n+10;
Each n is independently 0, 1 or 2; preferably n is 0 or 1;

Wherein, any one of the above amino acid sequences further includes a derivative sequence that is optionally added, deleted, modified and/or substituted for at least one amino acid and can retain the binding affinity of OX40.

In another preferred embodiment, the sequence formed by adding, deleting, modifying and/or substituting at least one amino acid sequence is preferably an amino acid sequence with homology or sequence identity of at least 80%, preferably at least 85%, more preferably, at least 90%, and most preferably at least 95%.

Methods of determining sequence homology or identity well-known to those of ordinary skill in the art include, but are not limited to: Computational Molecular Biology, Lesk, edited by A.M., Oxford University Press, New York, 1988; Biocomputing: Information Biocomputing: Informatics and Genome Projects, Smith, edited by D.W., Academic Press, New York, 1993; Computer Analysis of Sequence Data, Part 1, Griffin, A.M. and Griffin, edited by H.G., Humana Press, New Jersey, 1994; Sequence Analysis in Molecular Biology, von Heinje, G., Academic Press, 1987 and Sequence Analysis Primer, Gribskov, M. and Devereux , J. Ed. M Stockton Press, New York, 1991 and Carillo, H. and Lipman, D., SIAM J. Applied Math., 48:1073 (1988). The preferred method for determining identity is to obtain the largest match between the tested sequences. The method for determining identity is compiled in a publicly available computer program. Preferred computer program methods for determining the identity between two sequences include but are not limited to: GCG package (Devereux, J. et al., 1984), BLASTP, BLASTN and FASTA (Altschul, S, F. et al., 1990). The public can obtain the BLASTX program from NCBI and other sources (BLAST Handbook, Altschul, S. et al., NCBI NLM NIH Bethesda, Md. 20894; Altschul, S. et al., 1990). The well-known Smith Waterman algorithm can also be used to determine identity.

Preferably, the antibody as described herein is one or more of an antibody full-length protein, an antigen-antibody binding domain protein fragment, a bispecific antibody, a multispecific antibody, a single chain antibody (Single chain antibody fragment, ScFv), single domain antibody (SdAb) and Signle-domain antibody, and monoclonal or polyclonal antibodies prepared from the above antibodies. The monoclonal antibody can be developed by a variety of approaches and technologies, including hybridoma technology, phage display technology, single lymphocyte gene cloning technology, etc.. The mainstream is to prepare monoclonal antibodies from wild-type or transgenic mice through hybridoma technology.

The antibody full-length protein is a conventional antibody full-length protein in the art, which comprises a heavy chain variable region, a light chain variable region, a heavy chain constant region and a light chain constant region. The heavy chain variable region and light chain variable region of the protein, the human heavy chain constant region and the human light chain constant region constitute a fully human antibody full-length protein. Preferably, the antibody full-length protein is IgG1, IgG2, IgG3 or IgG4.

The antibody of the present invention may be a double-chain or single-chain antibody, and may be selected from the group consisting of animal-derived antibodies, chimeric antibodies, humanized antibodies, more preferably humanized antibodies, human-animal chimeric antibodies, more preferably a fully humanized antibody.

The antibody derivatives of the present invention may be single-chain antibodies, and/or antibody fragments, such as: Fab, Fab ', (Fab')2 or other known antibody derivatives in the field and the like, and any one or more of IgA, IgD, IgE, IgG and IgM antibodies or antibodies of other subtypes.

The single chain antibody is a conventional single chain antibody in the art, which comprises a heavy chain variable region, a light chain variable region and a short peptide of 15-20 amino acids.

Wherein the animal is preferably a mammal, such as a mouse.

The antibody of the present invention may be a chimeric antibody, a humanized antibody, a CDR grafted and/or modified antibody targeting OX40 (such as human OX40)

In the above content of the present invention, the number of added, deleted, modified and/or substituted amino acids is preferably no more than 40% of the total number of amino acids in the initial amino acid sequence, more preferably no more than 35%, more preferably 1-33%, more preferably 5-30%, more preferably 10-25%, more preferably 15-20%.

In the above content of the present invention, more preferably, the number of added, deleted, modified and/or substituted amino acids can be 1-7, more preferably 1-5, more preferably 1-3, more preferably 1-2.

In another preferred example, the heavy chain variable region of the antibody comprises the amino acid sequence as shown in SEQ ID NO. 101, 1, 11 or 21.

In another preferred example, the light chain variable region of the antibody contains the amino acid sequence as shown in SEQ ID NO. 103, 6, 16 or 26.

In another preferred example, the amino acid sequence of the variable region of the heavy chain (VH) of the antibody targeting OX40, and/or the amino acid sequence of the variable region of the light chain are shown in Table 2 below:

**Table 2**

| Antibody number | VH sequence Number | VL sequence Number |
|---|---|---|
| 1 | 101 | 103 |
| 2 | 1 | 6 |
| 3 | 11 | 16 |
| 4 | 21 | 26 |

In another preferred example, the antibodies targeting OX40 are Humab035-24, mab035, mab053, mab041, mab058, mab034, mab049, mab064, mab033, mab047, and mab055.

In another preferred example, the antibodies targeting OX40 are Humab035-24, Mab035, Mab053, Mab041.

In another preferred example, the antibody targeting OX40 is Humab035-24.

In another preferred example, the antibody targeting OX40 is mab035.

In another preferred example, the antibody targeting OX40 is mab053.

In another preferred example, the antibody targeting OX40 is mab041.

### Recombinant protein

The invention also provides a recombinant protein comprising one or more of the heavy chain CDR1 (VH-CDR1), the heavy chain CDR2 (VH-CDR2) and the heavy chain CDR3 (VH-CDR3) of the OX40 antibody, and/or one or more of the light chain CDR1 (VL-CDR1), light chain CDR2 (VL-CDR2) and light chain CDR3 (VL-CDR3) of the OX40 antibody,
the sequence of the heavy chain CDR1-3 is as follows:
The VH-CDR1 as shown in SEQ ID NO.10n+3,
The VH-CDR2 as shown in SEQ ID NO.10n+4, and
The VH-CDR3 as shown in SEQ ID NO.10n+5;
the sequence of the light chain CDR1-3 is as follows:
   VL-CDR1 as shown in SEQ ID NO.10n+8,
   VL-CDR2 as shown in SEQ ID NO.10n+9, and
   VL-CDR3 as shown in SEQ ID NO.10n+10;
each n is independently 0, 1, 2, 3, 4, 5, 6, 7, 8 or 9; preferably n is 0 or 1;
wherein, any one of the above amino acid sequences further includes a derivative sequence that is optionally added, deleted, modified and/or substituted for at least one amino acid and can retain the binding affinity of OX40.

In another preferred embodiment, the sequence formed by adding, deleting, modifying and/or substituting at least one amino acid sequence is preferably an amino acid sequence with homology or sequence identity of at least 80%, preferably at least 85%, more preferably, at least 90%, and most preferably at least 95%.

In another preferred embodiment, the recombinant protein of the present invention includes the heavy chain variable region of the OX40 antibody and/or the light chain variable region of the OX40 antibody. The heavy chain variable region of the antibody contains the amino acid sequence as shown in SEQ ID NO. 101, 1, 11 or 21; the light chain variable region of the antibody contains the amino acid sequence as shown in SEQ ID NO. 103, 6, 16 or 26.

In another preferred embodiment, the recombinant protein of the present invention includes the heavy chain variable region of the OX40 antibody and the light chain variable region of the OX40 antibody. The heavy chain variable region has the amino acid sequence as shown in SEQ ID NO. 101 or 10n+1, the light chain variable region has the amino acid sequence as shown in SEQ ID NO. 103 or 10n+6, wherein n is 0, 1, 2, 3, 4, 5, 6, 7, 8, or 9.

In another preferred embodiment, the recombinant protein of the present invention includes the heavy chain variable region of the OX40 antibody and the light chain variable region of the OX40 antibody. The heavy chain variable region of the antibody contains the amino acid sequence as shown in SEQ ID NO. 101, 1, 11, or 21, and the light chain variable region of the antibody contains the amino acid sequence as shown in SEQ ID NO. 103, 6, 16 or 26.

In another preferred embodiment, the sequence numberings of the recombinant protein and the amino acid sequences of the heavy chain CDR1-3 and the light chain CDR1-3 included in the recombinant protein are shown in Table 3:

**Table 3 Sequence numbering of the amino acid sequence of heavy chain CDR1-3 and light chain CDR1-3**

| Recombin ant protein number | Heavy chain protein | | | | Light chain protein | | | |
|---|---|---|---|---|---|---|---|---|
| | Variable region | VH-CDR 1 | VH-CDR2 | VH-CDR3 | Variable region | VL-CD R1 | VL-CDR2 | VL-CDR 3 |
| 1 | 101 | 3 | 4 | 5 | 103 | 8 | 9 | 10 |
| 2 | 1 | 3 | 4 | 5 | 6 | 8 | 9 | 10 |
| 3 | 11 | 13 | 14 | 15 | 16 | 18 | 19 | 20 |
| 4 | 21 | 23 | 24 | 25 | 26 | 28 | 29 | 30 |

Wherein, any one of the above amino acid sequences further includes a derivative sequence that is optionally added, deleted, modified and/or substituted for at least one amino acid and can retain the binding affinity of OX40.

Preferably, the recombinant protein further comprises an antibody heavy chain constant region and/or an antibody light chain constant region, the antibody heavy chain constant region is conventional in the art, preferably a rat-derived antibody heavy chain constant region or a human-derived antibody heavy chain constant region, more preferably a human-derived antibody heavy chain constant region. The antibody light chain constant region is conventional in the art, preferably a rat-derived light chain antibody constant region or a human-derived antibody light chain constant region, and more preferably a human-derived antibody light chain constant region.

The recombinant protein is a conventional protein in the art, preferably one or more of an antibody full-length protein, an antigen-antibody binding domain protein fragment, a bispecific antibody, a multispecific antibody, a single chain antibody (single chain antibody fragment, scFv), single domain antibody (SdAb) and signle-domain antibody, and monoclonal or polyclonal antibodies prepared from the above antibodies. The monoclonal antibody can be developed by a variety of approaches and technologies, including hybridoma technology, phage display technology, single lymphocyte gene cloning technology, etc.. The mainstream is to prepare monoclonal antibodies from wild-type or transgenic mice through hybridoma technology.

The antibody full-length protein is a conventional antibody full-length protein in the art, which comprises a heavy chain variable region, a light chain variable region, a heavy chain constant region and a light chain constant region. The heavy chain variable region and light chain variable region of the protein, the human heavy chain constant region and the human light chain constant region constitute the full-length protein of the fully human antibody. Preferably, the antibody full-length protein is IgG1, IgG2, IgG3 or IgG4.

The single chain antibody is a conventional single chain antibody in the art, which comprises a heavy chain variable region, a light chain variable region and a short peptide of 15-20 amino acids.

The antigen-antibody binding domain protein fragment is a conventional antigen-antibody binding domain protein fragment in the art, which includes a light chain variable region, a Fd segment of a light chain constant region and heavy chain constant region. Preferably, the antigen-antibody binding domain protein fragment is Fab and F(Ab ').

The single domain antibody is a conventional single domain antibody in the art, which includes a heavy chain variable region and a heavy chain constant region.

The single-region antibody is a conventional single-region antibody in the art, which only includes the heavy chain variable region.

The preparation method of the recombinant protein is the conventional preparation method in the field. The preparation method is preferably: It is isolated and obtained from the expression transformant that recombinantly expresses the protein or obtained by artificially synthesizing the protein sequence. The method of separating and obtaining from the expression transformant that recombinantly express the protein is preferably as follows: cloning the nucleic acid molecule encoding the protein and carrying the point mutation into a recombinant vector, and transforming the obtained recombinant vector into the transformant to obtain the recombinant expression transformant and the recombinant protein can be obtained by separation and purification by culturing the obtained recombinant expression transformant.

### Nucleic acid

The present invention also provides a nucleic acid encoding the above-mentioned antibody (e.g., anti-OX40 antibody) or recombinant protein or the variable region of the heavy chain or the variable region of the light chain of an anti-OX40 antibody.

Preferably, the nucleotide sequence of the nucleic acid encoding the heavy chain variable region is as shown in the sequence listing of SEQ ID NO. 102, 2, 12, 22, 32, 42, 52, 62, 72, 82 or 92; and/or, the nucleotide sequence of the nucleic acid encoding the light chain variable region is as shown in the sequence listing of SEQ ID NO. 104, 7, 17, 27, 37, 47, 57, 67, 77, 87 or 97.

Preferably, the nucleotide sequence of the nucleic acid encoding the heavy chain variable region is as shown in the sequence listing of SEQ ID NO. 102, 2, 12 or 22; and the nucleotide sequence of the nucleic acid encoding the light chain variable region is as shown in the sequence listing of SEQ ID NO. 104, 7, 17 or 27.

The preparation method of the nucleic acid is a conventional preparation method in the field, and preferably comprises the following steps: obtaining a nucleic acid molecule encoding the above protein by a gene cloning technology, or obtaining a nucleic acid molecule encoding the above protein by an artificial full sequence synthesis method.

Those skilled in the art know that the base sequence encoding the amino acid sequence of the above-mentioned protein can be replaced, deleted, changed, inserted or added as appropriate to provide a polynucleotide homolog. The polynucleotide homologs of the present invention can be prepared by replacing, deleting or adding one or more bases of the gene encoding the protein sequence within the scope of maintaining antibody activity.

### Vector

The invention also provides a recombinant expression vector comprising the nucleic acid.

Wherein the recombinant expression vector can be obtained by conventional methods in the art, that is, the nucleic acid molecule of the present invention is connected to various expression vectors to be constructed. The expression vector is a variety of vectors conventional in the art, as long as it can accommodate the aforementioned nucleic acid molecules. The vectors preferably include various plasmids, cosmids, bacteriophages or viral vectors.

The invention also provides a recombinant expression transformant comprising the above-mentioned recombinant expression vector.

Wherein the preparation method of the recombinant expression transformant is the conventional preparation method in the field, preferably: It is prepared by transforming the above-mentioned recombinant expression vector into a host cell. The host cell is a variety of conventional host cells in the art, as long as the recombinant expression vector can replicate itself stably and the nucleic acid carried can be effectively expressed. Preferably, the host cell is E.coli TG1 or E.coli BL21 cell (expressing single-chain antibody or Fab antibody), or HEK293 or CHO cell (expressing full-length IgG antibody). The aforementioned recombinant expression plasmid is transformed into a host cell to obtain the preferred recombinant expression transformant of the present invention. The transformation method is a conventional transformation method in the field, preferably a chemical transformation method, a heat shock method or an electrotransformation method.

### Antibody preparation

The sequence of the DNA molecule of the antibody or its fragment of the present invention can be obtained by conventional techniques, such as PCR amplification or genomic library screening. In addition, the coding sequences of the light chain and the heavy chain can also be fused together to form a single chain antibody.

Once the relevant sequences are obtained, the relevant sequences can be obtained in large quantities by recombination method. It is usually cloned into a vector, then transferred into a cell, and then the relevant sequence is isolated from the host cell after proliferation by conventional methods.

In addition, the relevant sequences can also be synthesized by artificial synthesis, especially when the fragment length is short. Usually, by first synthesizing multiple small fragments, and then ligating to obtain very long fragments.

At present, the DNA sequence encoding the antibody (or fragment or derivative thereof) of the present invention can be obtained completely through chemical synthesis. The DNA sequence can then be introduced into various existing DNA molecules (or such as vectors) and cells known in the art. In addition, mutations can also be introduced into the protein sequence of the present invention through chemical synthesis.

The present invention also relates to a vector containing the above-mentioned appropriate DNA sequence and an appropriate promoter or control sequence. These vectors can be used to transform appropriate host cells so that they can express proteins.

The host cell may be a prokaryotic cell, such as a bacterial cell; or lower eukaryotic cells, such as yeast cells; or higher eukaryotic cells, such as mammalian cells. Preferred animal cells include (but are not limited to): CHO-S, HEK-293 cells.

Generally, the transformed host cell is cultured under conditions suitable for expression of the antibody of the present invention. Then the antibody of the present invention is purified by conventional immunoglobulin purification steps, such as protein A-Sepharose, hydroxyapatite chromatography, gel electrophoresis, dialysis, ion exchange chromatography, hydrophobic chromatography, molecular sieve chromatography or affinity chromatography and other conventional separation and purification methods well known to those skilled in the art.

The obtained monoclonal antibody can be identified by conventional means. For example, the binding specificity of monoclonal antibodies can be determined by immunoprecipitation or in vitro binding assays (such as radioimmunoassay (RIA) or enzyme-linked immunosorbent assay (ELISA)). The binding affinity of the monoclonal antibody can be determined, for example, by the Scatchard analysis of Munson et al., Anal. Biochem., 107:220 (1980).

The antibody of the present invention can be expressed in the cell, on the cell membrane, or secreted out of the cell. If necessary, the physical, chemical, and other characteristics can be used to separate and purify the recombinant protein through various separation methods. These methods are well known to those skilled in the art. Examples of these methods include, but are not limited to: conventional renaturation treatment, treatment with protein precipitation agent (salting out method), centrifugation, bacteria broken through osmosis, ultrasonic treatment, ultracentrifugation, molecular sieve chromatography (gel filtration), adsorption chromatography, ion exchange chromatography, high performance liquid chromatography (HPLC) and various other liquid chromatography techniques and combinations of these methods.

### Antibody-Drug conjugate (ADC)

The present invention also provides antibody-drug conjugate (ADC) based on the antibody of the present invention.

Typically, the antibody-drug conjugate comprises the antibody and an effector molecule, the antibody being coupled to the effector molecule, and preferably chemically coupled. Wherein the effector molecule is preferably a drug having therapeutic activity. In addition, the effector molecule may be one or more of toxic proteins, chemotherapeutic drugs, small molecule drugs or radionuclides.

The antibody of the present invention can be coupled with the effector molecule by a coupling agent. Examples of the coupling agent may be any one or more of a non-selective coupling agent, a coupling agent using a carboxyl group, a peptide chain, and a coupling agent using a disulfide bond. The non-selective coupling agent refers to a compound that makes the effector molecule and the antibody form a covalent bond, such as glutaraldehyde. The coupling agent using carboxyl groups can be any one or more of cis-aconitic acid anhydride coupling agents (such as cis-aconitic acid anhydride) and acyl hydrazone coupling agents (coupling sites are acyl hydrazones).

Certain residues on the antibody (such as Cys or Lys, etc.) are used to connect to a variety of functional groups, including imaging reagents (such as chromophores and fluorescent groups), diagnostic reagents (such as MRI contrast agents and radioisotopes), stabilizers (such as glycol polymers) and therapeutic agents. The antibody can be conjugated to the functional agent to form an antibody-functional agent conjugate. Functional agents (e.g., drugs, detection reagents, stabilizers) are coupled (covalently linked) to the antibody. The functional agent may be directly or indirectly linked to the antibody through a linker.

Antibodies can be conjugated with drugs to form antibody-drug conjugates (ADCs). Typically, the ADC contains a linker between the drug and the antibody. The linker can be a degradable or a non-degradable linker. Degradable linkers are typically easily degraded in the intracellular environment, for example, the linker is degraded at the target site, so that the drug is released from the antibody. Suitable degradable linkers include, for example, enzymatically degraded linkers, including peptidyl-containing linkers that can be degraded by intracellular proteases (such as lysosomal proteases or endosomal proteases), or sugar linkers, for example, a glucuronide-containing linker that can be degraded by glucuronidase. The peptidyl linker may include, for example, dipeptides such as valine-citrulline, phenylalanine-lysine or valine-alanine. Other suitable degradable linkers include, for example, pH-sensitive linkers (for example, linkers that are hydrolyzed at a pH of less than 5.5, such as hydrazone linkers) and linkers that degrade under reducing conditions (for example, disulfide bond linkers). Non-degradable linkers typically release the drug under conditions where the antibody is hydrolyzed by a protease.

Before being connected to the antibody, the linker has a reactive group capable of reacting with certain amino acid residues, and the connection is achieved through the reactive group. Sulfhydryl-specific reactive groups are preferred and include, for example, maleimide compounds, halogenated amides (such as iodine, bromine, or chloro); halogenated esters (such as iodine, bromine, or chloro); halogenated methyl ketones (such as iodine, bromine or chloro), benzyl halides (such as iodine, bromine or chloro); vinyl sulfone, pyridyl disulfide; mercury derivatives such as 3,6-Di-(mercury methyl) dioxane, and the counter ion is acetate, chloride or nitrate; and polymethylene dimethyl sulfide thiosulfonate. The linker may include, for example, maleimide linked to the antibody via thiosuccinimide.

The drug can be any cytotoxic, inhibiting cell growth or immunosuppressive drug. In embodiments, the linker connects the antibody and the drug, and the drug has a functional group that can be bonded to the linker. For example, the drug may have an amino group, a carboxyl group, a sulfhydryl group, a hydroxyl group, or a ketone group that can form a bond with the linker. In the case where the drug is directly connected to the linker, the drug has a reactive active group before being connected to the antibody.

Useful drug categories include, for example, anti-tubulin drugs, DNA minor groove binding reagents, DNA replication inhibitors, alkylating reagents, antibiotics, folate antagonists, antimetabolites, chemotherapy sensitizers, topoisomerase inhibitors, Vinca Alkaloids, etc.. Examples of particularly useful cytotoxic drugs include, for example, DNA minor groove binding reagents, DNA alkylating reagents, and tubulin inhibitors. Typical cytotoxic drugs include, for example, auristatins, camptothecins, duocarmycins, etoposides, maytansines and maytansinoids (e.g., DM1 and DM4), taxanes, benzodiazepines or benzodiazepine containing drugs (e.g., pyrrolo[1,4] benzodiazepines (PBDs), indolinobenzodiazepines and oxazolidinobenzodiazepines and vinca alkaloids.

In the present invention, the drug-linker can be used to form ADC in one simple step. In other embodiments, bifunctional linker compounds can be used to form ADCs in a two-step or multi-step process. For example, the cysteine residue reacts with the reactive part of the linker in the first step, and in the subsequent step, the functional group on the linker reacts with the drug to form ADC.

Generally, the functional group on the linker is selected to facilitate the specific reaction with the appropriate reactive group on the drug moiety. As a non-limiting example, the azide-based moiety can be used to specifically react with the reactive alkynyl group on the drug moiety. The drug is covalently bound to the linker through the 1,3-dipolar cycloaddition between the azide and alkynyl groups. Other useful functional groups include, for example, ketones and aldehydes (suitable for reacting with hydrazides and alkoxyamines), phosphines (suitable for reacting with azides); isocyanates and isothiocyanates (suitable for reaction with amines and alcohols); and activated esters, such as N-hydroxysuccinimide ester (suitable for reaction with amines and alcohols). These and other ligation strategies, such as those described in "Bioconjugation Technology", Second Edition (Elsevier), are well known to those skilled in the art. Those skilled in the art can understand that for the selective reaction between the drug moiety and the linker, when a complementary pair of reactive functional groups is selected, each member of the complementary pair can be used for both linkers and drugs.

The present invention also provides a method for preparing ADC, which may further include: combining an antibody with a drug-linker compound under conditions sufficient to form an antibody conjugate (ADC).

In certain embodiments, the methods of the invention include combining the antibody with a bifunctional linker compound under conditions sufficient to form an antibody-linker conjugate. In these embodiments, the method of the present invention further includes: binding the antibody linker conjugate to the drug moiety under conditions sufficient to covalently link the drug moiety to the antibody via a linker.

In some embodiment, the antibody-drug conjugate ADC is shown in the following molecular formula: 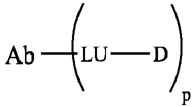 wherein
Ab is an antibody,
LU is a linker;
D is a drug;
and the subscript p is a value selected from 1 to 8.

### Application

The present invention also provides the use of the antibody, antibody conjugate ADC, recombinant protein, and/or immune cell of the present invention, for example, for preparing diagnostic preparations or preparing medicines.

Preferably, the drug is a drug for preventing and/or treating diseases related to abnormal expression or function of OX40.

In the present invention, the diseases related to abnormal expression or function of OX40 are diseases related to abnormal expression or function of OX40 conventionally in the art. Preferably, the disease related to the abnormal expression or function of OX40 is cancer or autoimmune disease.

In the present invention, the cancer is a conventional cancer in the field, preferably breast cancer, melanoma, B-cell lymphoma, head and neck cancer, colon cancer, prostate cancer, and lung cancer. In the present invention, the autoimmune disease is a conventional autoimmune disease in the art, preferably rheumatoid arthritis, inflammatory hyperproliferative skin disease, multiple sclerosis, inflammatory bowel disease, asthma, autoimmune myocardioptis, systemic lupus erythematosus.

The uses of the antibodies, ADCs, recombinant proteins, and/or immune cells of the present invention include (but are not limited to):
(i) diagnosis, prevention and/or treatment of tumor occurrence, growth and/or metastasis, especially tumors with high OX40 expression. The tumors include (but are not limited to): breast cancer, melanoma, B-cell lymphoma, head and neck cancer, colon cancer, prostate cancer, lung cancer.
(ii) diagnosis, prevention and/or treatment of autoimmune diseases, including (but are not limited to): rheumatoid arthritis, inflammatory hyperproliferative skin disease, multiple sclerosis, inflammatory bowel disease, asthma, autoimmune myocardioptis, systemic lupus erythematosus.

### Detection purposes and kits

The antibody or ADC of the present invention can be used in detection applications, for example, to detect samples, so as to provide diagnostic information.

In the present invention, the samples (specimen) used include cells, tissue samples and biopsy specimens. The term "biopsy" used in the present invention shall include all kinds of biopsy known to those skilled in the art. Therefore, the biopsy used in the present invention may include, for example, excision samples of tumors, tissue samples prepared by endoscopic methods or organ puncture or needle biopsy.

The samples used in the present invention include fixed or preserved cell or tissue samples.

The present invention also provides a kit containing the antibody (or fragment thereof) of the present invention. In a preferred embodiment of the present invention, the kit further includes a container, instructions for use, buffers, and the like. In a preferred embodiment, the antibody of the present invention can be immobilized on a detection plate.

### Pharmaceutical composition

The invention also provides a composition. In a preferred example, the composition is a pharmaceutical composition, which contains the above-mentioned antibody or active fragment or fusion protein or ADC thereof or corresponding immune cell, and a pharmaceutically acceptable carrier. Generally, these substances can be formulated in a non-toxic, inert and pharmaceutically acceptable aqueous carrier medium, wherein the pH is usually about 5-8, preferably about 6-8, although the pH value can vary with the nature of the substance being formulated and the condition to be treated.

The formulated pharmaceutical composition can be administered by conventional routes, including (but are not limited to): intratumoral, intraperitoneal, intravenous, or topical administration. Typically, the route of administration of the pharmaceutical composition of the present invention is preferably injection or oral administration. The injection administration preferably includes intravenous injection, intramuscular injection, intraperitoneal injection, intradermal injection, or subcutaneous injection. The pharmaceutical composition is a variety of conventional dosage forms in the art, preferably in the form of solid, semi-solid or liquid, and can be an aqueous solution, non-aqueous solution or suspension, and more preferably a tablet, capsule, or granule, injection or infusion, etc..

The antibody of the present invention can also be used for cell therapy by expressing the nucleotide sequence in a cell, for example, the antibody is used for chimeric antigen receptor T cell immunotherapy (CAR-T) and the like.

The pharmaceutical composition of the present invention is a pharmaceutical composition for preventing and/or treating diseases related to abnormal expression or function of OX40.

The pharmaceutical composition of the present invention can be directly used to bind OX40 protein molecules, and thus can be used to prevent and treat tumors and other diseases.

The pharmaceutical composition of the present invention contains a safe and effective amount (such as 0.001-99wt%, preferably 0.01-90wt%, more preferably 0.1-80wt%) of the above-mentioned monoclonal antibody (or conjugate thereof) of the present invention and a pharmaceutical acceptable carrier or excipient. Such carriers include (but are not limited to): saline, buffer, glucose, water, glycerol, ethanol, and combinations thereof. The pharmaceutical preparation should match the mode of administration. The pharmaceutical composition of the present invention can be prepared in the form of injections, for example, it can be prepared by conventional methods with physiological saline or an aqueous solution containing glucose and other adjuvants. Pharmaceutical compositions such as injections and solutions should be manufactured under aseptic conditions. The dosage of the active ingredient is a therapeutically effective amount, for example, about 1 microgram/kg body weight to about 5 mg/kg body weight per day. In addition, the polypeptides of the present invention can also be used together with other therapeutic agents.

In the present invention, preferably, the pharmaceutical composition of the present invention further includes one or more pharmaceutical carriers. The pharmaceutical carrier is a conventional pharmaceutical carrier in the art, and the pharmaceutical carrier can be any suitable physiologically or pharmaceutically acceptable pharmaceutical excipient. The pharmaceutical excipients are conventional pharmaceutical excipients in the field, and preferably include pharmaceutically acceptable excipients, fillers or diluents. More preferably, the pharmaceutical composition includes 0.01-99.99% of the aforementioned protein and 0.01-99.99% of a pharmaceutical carrier, and the percentage is a mass percentage of the pharmaceutical composition.

In the present invention, preferably, the administration amount of the pharmaceutical composition is an effective amount, and the effective amount is an amount capable of alleviating or delaying the progression of the disease, degenerative or traumatic condition. The effective amount can be determined on an individual basis and will be partly based on consideration of the symptoms to be treated and the results sought. Those skilled in the art can determine the effective amount by using the aforementioned factors such as individual basis and using no more than conventional experiments.

When using the pharmaceutical composition, a safe and effective amount of the immunoconjugate is administered to the mammal, wherein the safe and effective amount is usually at least about 10 micrograms/kg body weight, and in most cases, it does not exceed about 50 mg/kg body weight, preferably the dosage is about 10 micrograms/kg body weight to about 20 mg/kg body weight. Of course, the specific dosage should also consider factors such as the route of administration, the patient's health status, etc., which are within the skill range of a skilled physician.

The present invention provides the application of the above-mentioned pharmaceutical composition in the preparation of drugs for preventing and/or treating diseases related to abnormal expression or function of OX40. Preferably, the disease related to the abnormal expression or function of OX40 is cancer or autoimmune disease.

### Method and composition for detecting OX40 protein in sample

The present invention also provides a method for detecting OX40 protein in a sample (for example, detecting over-expressing OX40 cells), which includes the following steps: the above-mentioned antibody is contacted with the sample to be tested in vitro, and it is sufficient to detect whether the above-mentioned antibody binds to the sample to be tested to form an antigen-antibody complex.

The meaning of overexpression is conventional in the art, and refers to the overexpression of OX40 protein in RNA or protein in the sample to be tested (due to increased transcription, post-transcriptional processing, translation, post-translational processing and changes in protein degradation), as well as local overexpression and increased functional activity due to changes in protein transport mode (increased nuclear localization) (such as in the case of increased enzymatic hydrolysis of the substrate).

In the present invention, the detection method of whether the above-mentioned binding to form an antigen-antibody complex is a conventional detection method in the art, preferably a flow cytometry (FACS) detection.

The present invention provides a composition for detecting OX40 protein in a sample, which includes the above-mentioned antibody, recombinant protein, antibody conjugate, immune cell, or a combination thereof as an active ingredient. Preferably, it also includes a compound composed of the above-mentioned functional fragments of the antibody as an active ingredient.

On the basis of complying with common knowledge in the art, the above preferred conditions can be arbitrarily combined to obtain preferred examples of the present invention.

### The main advantages of the present invention are:

(1) The protein of the present invention is the OX40 antibody, which has high affinity with human-derived OX40 protein, and has similar binding ability with monkey-derived OX40 protein, can activate the NF-kB signaling pathway, and can significantly increase the expression of IFN gamma in memory CD4+ T cells..
(2) Some of the OX40 antibodies of the present invention have different epitopes compared with the antibodies that have been developed so far.
(3) Some of the OX40 antibodies of the present invention can better activate the NF-kB signaling pathway and the function of T cells, and prolong the life cycle of MC38 tumor mice compared with the antibodies that have been developed so far.

The present invention will be further elaborated below in conjunction with specific embodiments. It should be understood that these embodiments are only used to illustrate the present invention and not to limit the scope of the present invention. The following examples do not specify the detailed conditions of the experimental methods, usually according to the conventional conditions such as the conditions described in the "Molecular Cloning Laboratory Guide" (translated by Huang Peitang et al., Beijing: Science Press, 2002) by Sambrook. J et al. , Or in accordance with the conditions recommended by the manufacturer (such as product specifications). Unless otherwise stated, percentages and parts are calculated by weight. The experimental materials and reagents used in the following examples can be obtained from commercial channels unless otherwise specified.

The room temperature described in the embodiment is a conventional room temperature in the art, and is generally 10-30° C.

Unless otherwise specified, the PBS described in the examples is PBS phosphate buffer, pH 7.4.

### EXAMPLE1 Preparation of OX40 antibody

### 1.1 Preparation of immunogen A

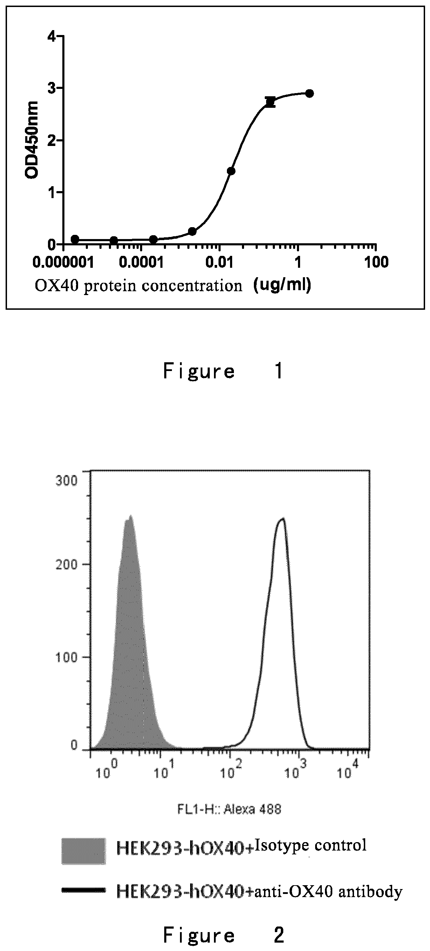

The nucleotide sequence containing the amino acid sequence Leu29-Ala216 (human-derived OX40 protein sequence from Uniprot database, protein sequence number P43489) encoding the extracellular region of human-derived OX40 protein was cloned into pTT5 vector with human IgG Fc fragment (hFc) and prepared plasmids according to established standard molecular biology methods. For specific methods, see Sambrook, J., Fritsch, EF, and Maniatis, T. (1989). Molecular Cloning: A Laboratory Manual, Second Edition (Plainview, New York: Cold Spring Harbor Laboratory Press). Transfecting HEK293 cells with the prepared plasmid transiently (polyetherimide (PEI), purchased from Polysciences) and expanding the culture. After 4 days, the cell culture fluid was collected, and the cell components were removed by centrifugation to obtain the culture supernatant containing the extracellular region of OX40 protein. The culture supernatant was loaded onto a protein A affinity chromatography column (purchased from GE Healthcare), and at the same time, monitoring the change of UV absorption value (A280nm) with UV detector. After loading the sample, washing the protein A affinity chromatography column with PBS until the UV absorption value returns to the baseline, and then eluted with 0.1M glycine hydrochloric acid (pH 2.5) to collect the extracellular domain of OX40 protein with hFc tag (OX40 ECD-hFc) eluted from the protein A affinity chromatography column, and dialyzed overnight in a refrigerator at 4° C with PBS. The dialyzed protein was sterile filtered by 0.22 µm and stored at -80° C to obtain purified immunogen A. Immunogen A undergoes a series of quality control tests before use, such as testing its protein concentration, purity, molecular weight, biological activity, etc.. The results are shown in Figure 1. Figure 1 illustrates that the binding of OX40 and OX40L at the protein level varies with the concentration of OX40. The data in the figure is the OD450nm value.

Among them, the biological activity of immunogen A is detected by ELISA, specifically:
Diluting OX40L recombinant protein (purchased from R&D) with PBS to 1 µ g/mL, adding 100 µ l/well to the ELISA microplate, incubated overnight at 4° C; using ELISA blocking solution (containing 1% BSA, pH7.4 of PBS phosphate buffer , the percentage is the mass percentage.) After sealing at 37° C for two hours, adding OX40 ECD-hFc (i.e., immunogen A) in gradient dilutions, and incubated at 37° C for 1 hour; adding horseradish peroxidase labeled goat anti-human IgG (purchased from Sigma, A8667), after 30 minutes of incubation at 37° C; adding 100µL/well of TMB color developing solution (purchased from InnoReagents, EL0009), after 15 minutes of incubation at room temperature, adding 50µL of IN hydrochloric acid to terminate the chromogenic reaction. Reading the OD450nm value with an ELISA plate reader.

### 1.2 Preparation of immunogen B

The nucleotide sequence encoding the full-length amino acid sequence of human-derived (human) OX40 (the sequence is from the Uniprot database, the protein sequence number is P43489) was cloned into the pLVX-IRES-Puro vector and the plasmid (herein referred to as pLVX-hOX40-IRES-Puro) was prepared. After plasmid transfection of HEK293 cell line, subcloning in 96-well culture plate by limiting dilution method, it was selectively cultured in DMEM medium containing 10% fetal bovine serum containing 0.5 µ g/ml puromycin. After about 2 weeks of culture, some monoclonal wells were selected and expanded into a 24-well plate, and then expanded into a 6-well plate after about 3-4 days. The amplified clones were screened by flow cytometry (FACS) with OX40 antibody (purchased from R&D). Choosing a cell line with better growth, higher fluorescence intensity, and monoclonal cell line to continue to expand culture and freeze in liquid nitrogen to obtain immunogen B (HEK293-hOX40 cells). The FACS result is shown in Figure 2. Figure 2 shows that the HEK293 cell line stably expressing OX40 has been screened.

### 1.3 Preparation of immunogen C

The cDNA encoding the full-length amino acid sequence of human OX40 (sequence is from Uniprot database, protein sequence number is P43489) was cloned into pCP vector (pCP-hOX40 plasmid) and coated into 1.0µm gold colloidal bullet (purchased from Bio-rad), That is, immunogen C was obtained. Immunization with Helios Gene Gun (Helios Gene Gun System, Bio-rad, article number 165-2431). Among them, the method of coating the 1.0µm gold colloidal bullet and immunization can be found in the instruction manual of Helios gene gun.

### 1.4 Construction of a stable cell line expressing OX40 protein

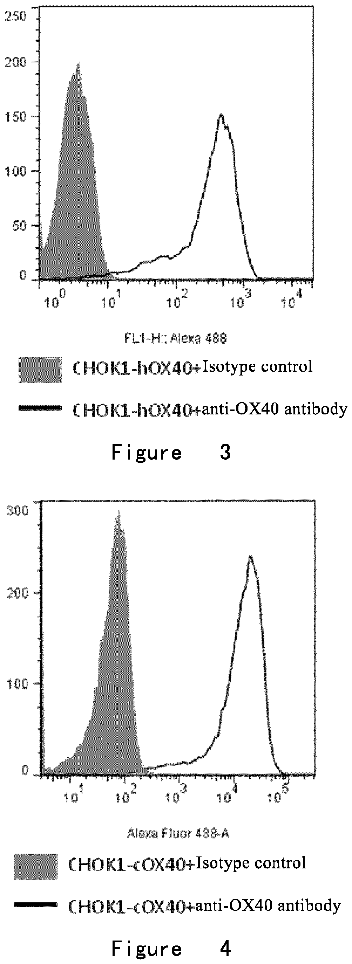

The pLVX-hOX40-IRES-Puro plasmid in Example 1.2 was transfected into the CHOK1 cell line, and subcloned in a 96-well culture plate by the limiting dilution method. Selectively cultivated in F12K medium containing 10% fetal bovine serum containing 6µg/ml puromycin. After about 2 weeks, some monoclonal wells were selected and expanded into 24-well plates, and after about 3-4 days, they were expanded into 6-well plates. The amplified clones were screened by flow cytometry with OX40 antibody (purchased from R&D). The cell line with better growth, higher fluorescence intensity, and monoclonal cell line was selected to continue to expand and culture and cryopreserved in liquid nitrogen to obtain a stable CHOK1 cell line expressing human OX40 protein (herein referred to as CHOK1-hOX40 stable cell line). Figure 3 shows that a stable CHOK1 cell line stably expressing human OX40 has been screened.

The nucleotide sequence encoding the full-length amino acid sequence of monkey-derived OX40 (the monkey-derived OX40 sequence is from NCBI database, the sequence number is XP_005545179.1) was cloned into the pLVX-IRES-Puro vector and the plasmid (herein referred to as pLVX-cOX40-IRES-Puro) was prepared. The pLVX-cOX40-IRES-Puro plasmid was transfected into the CHOK1 cell line, and the CHOK1 stable cell line expressing monkey OX40 (herein referred to as the CHOK1-cOX40 stable cell line) was screened. Figure 4 shows that a stable CHOK1 cell line stably expressing monkey OX40 has been screened.

### 1.5 Preparation of Hybridoma Cells and Screening of Antibodies

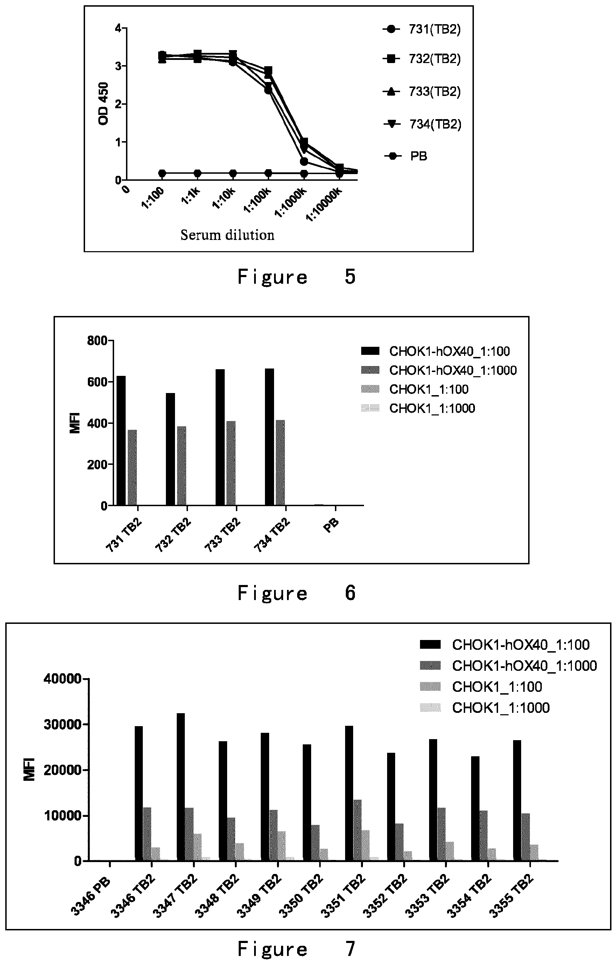

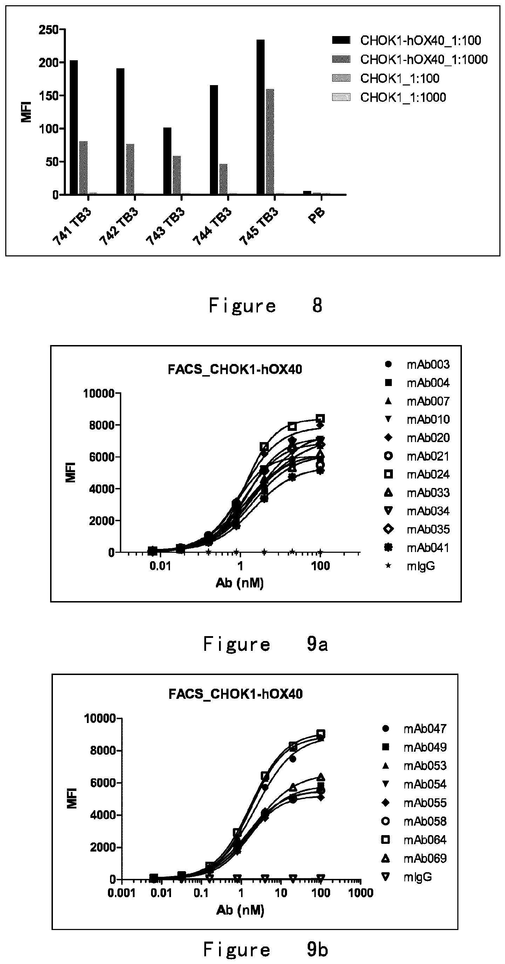

A. Immunogen A was used to immunize Balb/c and SJL mice (purchased from Shanghai SLAC) at the age of 6-8 weeks, and the mice were raised under SPF conditions. During the initial immunization, immunogen A protein was emulsified with Freund's complete adjuvant and injected intraperitoneally with 50 µg. When subjected to booster immunization, immunogen A protein was emulsified with Freund's incomplete adjuvant and injected intraperitoneally with 25 µg. There is an interval of 2 weeks between the initial immunization and the first booster immunization, and an interval of 3 weeks between each subsequent booster immunization. Blood was collected one week after each booster immunization, and the antibody titer and specificity of immunogen A in the serum were detected by ELISA and FACS. The results are shown in Figure 5 and Figure 6. Figures 5 and 6 show that the serum of mice immunized with OX40 ECD-hFc have different levels of binding to the immunogen. The antibody titer of serum detected by ELISA is more than 1:100,000, and the titer of serum antibody detected by FACS is more than 1:1000. Among them, TB2 refers to the mice serum seven days after the second booster immunization, and PB refers to the mice serum before the first immunization. The data in the figure are the OD450nm value and the mean fluorescence intensity (MFI value), respectively.
B. Immunogen B was used to immunize Balb/c and SJL mice aged 6-8 weeks (purchased from Shanghai SLAC). After receiving the mice, they were raised under SPF conditions. The HEK293 cells (HEK293-hOX40) stably expressing human OX40 protein screened in the step (2) of Example 1 were expanded and cultured to 90% confluence in a cell culture dish, the medium was removed, washed with PBS, and adding cell digestion solution TrypLE (purchased from Invitrogen) and treated at 37°C until the cells can be detached from the wall of the culture dish, and then collecting the cells. Washed twice with PBS, counting the cells and resuspended with PBS to 1-2×10⁷ cells per milliliter. Each mouse was intraperitoneally injected with 0.5ml of cell suspension during each immunization. There is an interval of 2 weeks between the first and second immunizations, and an interval of 3 weeks between each subsequent immunization. Except for the first immunization, blood was collected one week after each immunization, and the antibody titer and specificity in the serum were detected by flow cytometry (FACS). The result is shown in Figure 7. Figure 7 shows that after the second booster immunization, the serum antibody titer detected by FACS can reach more than 1:1000. Among them, TB2 refers to the mice serum seven days after the second booster immunization, and PB refers to the mice serum before the first immunization. The data in the figure is the mean fluorescence intensity (MFI).
C. Immunogen C was used to immunize 6-8 weeks old Balb/c and SJL mice (purchased from Shanghai SLAC). After receiving the mice, they were raised under SPF conditions. All mice were immunized with Helios gene gun through the abdomen for 4 times, 4 shots each time, 1.0µg cDNA per shot. The interval between the initial immunization and the first booster immunization is 2 weeks, and the interval between each subsequent booster immunization is 2 weeks. Blood was collected 1 week after each booster immunization, and the antibody titer in the serum was detected by FACS. The result is shown in Figure 8. Figure 8 shows that after the third booster immunization, the serum antibody titer detected by FACS can reach more than 1:1000. Among them, TB3 refers to the mice serum seven days after the third booster immunization, and PB refers to the mice serum before the first immunization. The data in the figure is the mean fluorescence intensity (MFI).

After steps A to C were completed, selecting mice with better serum antibody titer for fusion. For mice that were immune to immunogen A and immunogen C, 25µg of immunogen A (OX40 ECD-hFc recombinant protein) was injected into the enterocoelia for booster immunization before fusion; Mice that have been immune to immunogen B were injected with immunogen B (0.5-1×10⁷ HEK293-hOX40 cells) into the enterocoelia before fusion for booster immunization. After 5 days, the mice were sacrificed, spleen cells were collected, and a spleen cell suspension was obtained. The cells were washed by centrifugation with DMEM basal medium for 3 times, and then mixed with mouse myeloma cells SP2/0 at a ratio of 5:1 in the number of viable cells, and the PEG method (see METHODS IN ENZYMOLOGY, VOL.220) was used for cell fusion. The fused cells were diluted into DMEM medium containing 20% fetal bovine serum and 1×HAT (purchased from Invitrogen). Then adding 1×105/200µL per well to a 96-well cell culture plate, and putting it in a 5% CO₂, 37°C incubator. After 10-14 days, using ELISA or Acumen (microplate cell detection method) to determine the binding activity of the cell fusion plate supernatant to OX40 protein, and selecting positive clones with higher OD450nm value in ELISA or higher mean fluorescence intensity (MFI) in Acumen to expand into 24-well plate, and expanded culture in DMEM medium containing 10% fetal bovine serum and 1×HT (purchased from Invitrogen). After culturing for 3 days, taking the supernatant from the 24-well plate, using ELISA and FACS for secondary screening, determining the binding activity of the antibody in the supernatant to the OX40 protein, and the reporter gene experiment was used to detect the activation of NF-kB signaling pathway by antibodies in the supernatant. (see Example 3.2.2 for the method).

According to the results of the 24-well plate screening, the positive clones that have antigen-antibody binding activity and can activate the NF-kB signal pathway in ELISA and FACS experiments were selected, and subcloned in a 96-well plate by the limiting dilution method. Cultured in DMEM medium containing 10% FBS at 37 °C and 5% CO₂. Seven days after subcloning, preliminary screening was carried out by ELISA or Acumen, and 4 positive monoclonals were selected and amplified to 24-well plates for further culture. After 3 days, the binding activity was determined by ELISA and FACS.

According to the test results of the 24-well plate samples, the optimal clone was selected, and the hybridoma cells of the present invention were obtained by expanding the culture in DMEM medium containing 10% FBS at 37°C and 5% CO₂, and cryopreserving in liquid nitrogen, and can be used for subsequent antibody production and purification.

### EXAMPLE2 Production and Purification of Lead Antibody

The antibody concentration produced by hybridoma cells is low, only about 0.1-10 µg/mL, and the concentration varies greatly. In addition, the various proteins produced by cell culture in the medium and the fetal bovine serum components contained in the medium interfere with many biological activity analysis methods to varying degrees, so that small-scale (1-5mg) antibody production and purification is required.

The hybridoma cells obtained in Example 1 were inoculated into T-75 cell culture flasks, and using DMEM medium containing 10% FBS for expansion culture. When it grows well, inoculating it to the cell culture spinner flask and using a production medium containing 2.5% FBS (Low IgG) (Hybridoma serum free medium, purchased from Invitrogen). Adding 500mL production medium to each 2L culture spinner flask, and the inoculation cell density is 1.0×10⁵/mL. Tightly cap the bottle, and placing the spinner flask on the rotary machine in the 37° C incubator at a speed of 3 revolutions per minute. After 14 days of continuous rotation culture, the cell culture solution was collected and filtered with a 0.22 µm filter membrane until the culture supernatant was clarified. The clarified culture supernatant can be purified immediately or frozen at -30° C.

The monoclonal antibody in the culture supernatant of the clarified hybridoma cells was purified with a protein A column (purchased from GE Healthcare). The protein A column was first equilibrated with PBS phosphate buffer, and then the clarified culture supernatant was loaded onto the protein A column. After loading the sample, washing the protein A column with equilibration buffer (0.1 M Tris-HCl, pH 8.0). Elution solution (0.1 M sodium citrate buffer, pH 4.5 for IgG1, pH 3.5 for other IgG subtypes) was used to elute the OX40 antibody bound to the protein A column. Collecting the eluted antibody, adding 1.0M Tris-HCl buffer to neutralize the pH, and then dialyzed against PBS phosphate buffer overnight. The OX40 antibody after dialysis was collected, sterile filtered with a 0.22 µm filter, subjected to sterile preservation to obtain purified OX40 antibody.

The purified OX40 antibody was tested and analyzed for protein concentration, purity, and endotoxin (Lonza kit). The results are shown in Table 4.

**Table4 Detection and analysis of purified OX40 antibody**

| **Antibody name** | **Subtype** | **Concentration(mg/ml)** | **SDS-PAGE** | **SEC** | **Endotoxins (EU/mg)** |
|---|---|---|---|---|---|
| MAb003 | IgG1, k | 1.584 | >95% | 100% | 0.71 |
| MAb004 | IgG1, k | 0.866 | >95% | 100% | 1.32 |
| MAb007 | IgG2b, k | 0.637 | >95% | 100% | 2.00 |
| MAb010 | IgG1, k | 1.707 | >95% | 100% | 0.61 |
| MAb020 | IgG2c, k | 2.542 | >95% | 100% | 0.36 |
| MAb021 | IgG2b, k | 2.597 | >95% | 100% | 0.40 |
| MAb024 | IgG2c, k | 1.345 | >95% | 100% | 0.84 |
| MAb026 | IgG1, k | 1.906 | >95% | 97.97% | 0.37 |
| MAb033 | IgG1, k | 2.011 | >95% | 97.66% | 0.36 |
| MAb034 | IgG1, k | 1.07 | >95% | 100% | 1.10 |
| MAb035 | IgG1, k | 2.032 | >95% | 97.09% | 0.42 |
| MAb041 | IgG1, k | 1.635 | >95% | 100% | 0.53 |
| MAb047 | IgG2c, k | 0.842 | >95% | 100% | 1.89 |
| MAb049 | IgG1, k | 0.248 | >95% | 100% | 6.43 |
| MAb053 | IgG2c, k | 1.374 | >95% | 100% | 0.96 |
| MAb054 | IgG1, k | 1.243 | >95% | 100% | 1.13 |
| MAb055 | IgG1, k | 0.292 | >95% | 100% | 6.08 |
| MAb058 | IgG1, k | 1.616 | >95% | 100% | 0.08 |
| MAb064 | IgG2c, k | 1.768 | >95% | 100% | 0.07 |
| MAb069 | IgG1, k | 1.043 | >95% | 100% | 0.12 |

### EXAMPLE3 Detection of lead antibody

### 3.1 Flow cytometry (FACS) to detect the binding of antibodies to cells stably expressing OX40 protein

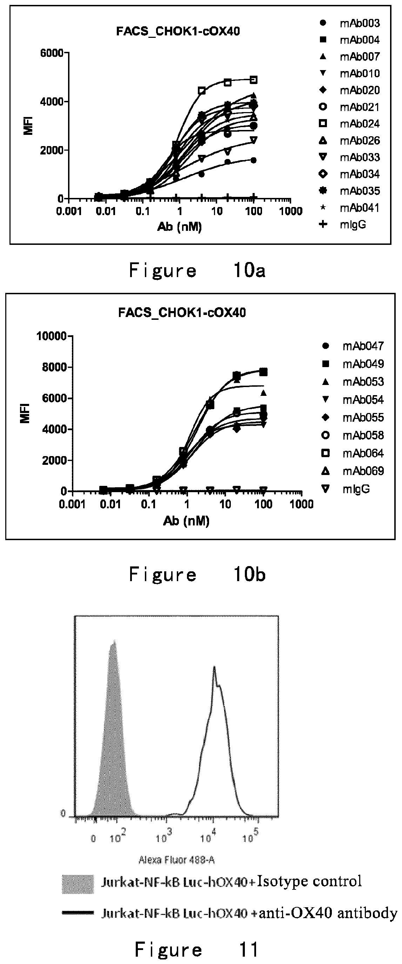

The CHOK1-hOX40 stable cell line and the CHOK1-cOX40 stable cell line constructed in Example 1.4 were expanded to 90% confluence in a cell culture dish, the medium was removed, washed with PBS phosphate buffer, and then the cells were treated and collected with cell digestion solution TrypLE (purchased from Invitrogen). Washing the cells with FACS buffer (PBS+2%FBS). After counting the cells, resuspending the cells in FACS buffer to 3×10⁶ cells/ml, adding 100µL per well to a 96-well FACS reaction plate, and centrifuged at 300g for 5 minutes and removing the supernatant, and a series of concentration gradients of 100 µL of the purified OX40 antibody obtained in Example 2 were added to each well, and incubated at 4°C for 1 hour. Washed twice with FACS buffer by centrifugation, adding 100µL fluorescent (Alexa 488) labeled secondary antibody (purchased from Invitrogen) per well, and incubated for 1 hour at 4°C in the dark. Washing the cells by centrifugation with FACS buffer for 3 times, adding 100µL of PBS buffer per well to suspend the cells, using FACS (FACS Canto II, purchased from BD) to detect and analyze the results. The results are shown in Figure 9a, Figure 9b and Figure 10a, Figure 10b. The antibody to be tested can bind to the OX40 protein on the cell surface. Among them, mIgG is the antibody subtype control mouse IgG, and the data MFI in the figure is the mean fluorescence intensity value of the measured cell population.

### 3.2 The NF-kB reporter gene experiment detects the activation of OX40 antibody on the NF-kB signaling pathway

### 3.2.1 Construction of Jurkat-NF-kB Luc-hOX40 stable cell line

The pGL4.32[luc2P/NF-κB-RE/Hygro] plasmid (purchased from Promega) was transfected into Jurkat cells, cultured in RPMI1640 medium containing hygromycin and 10% fetal bovine serum, and Jurkat cell line stably expressing NF-kB RE-luciferase (herein referred to as Jurkat-NF-kB Luc) was screened and obtained.

The pLVX-hOX40-IRES plasmid in Example 1.2 was transfected into Jurkat-NF-kB Luc cells and cultured with RPMI1640 medium containing both hygromycin, puromycin and 10% fetal bovine serum, the Jurkat cell line stably expressing NF-kB RE-luciferase and OX40 was screened and obtained (herein referred to as Jurkat-NF-kB Luc-hOX40).

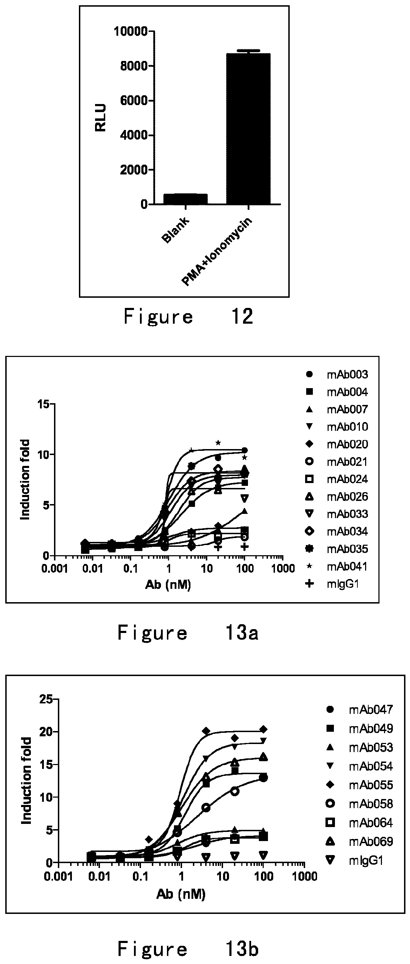

Figure 11 shows the results of FACS detection, indicating that the screened and obtained Jurkat-NF-kB Luc-hOX40 cell line stably expresses hOX40. Figure 12 shows the results of the NF-kB reporter gene experiment, indicating that the screened and obtained Jurkat-NF-kB Luc-hOX40 cell line stably expresses NF-kB RE-luciferase.

### 3.2.2 NF-kB reporter gene experiment

Pre-coated a 96-well plate (purchased from Perkin Elmer) with 1ug/ml anti-CD3 antibody, and incubated overnight at 4°C. Washed three times with PBS before use.

The Jurkat-NF-kB Luc-hOX40 cells screened in 3.2.1 were expanded and cultured. After collection, they were resuspended in RPMI1640 medium containing 1% FBS. Inoculating the cells in a 96-well plate coated with anti-CD3 antibody at a density of 1×10⁵/well. At the same time, 1×10⁵/well Raji cells were added as Fc gamma receptor expression cells. Then adding a series of concentration gradient OX40 antibody, putting it into the cell culture incubator and continuing to culture for 5h. The content of luciferase was determined with One-Glo Luciferase Detection System (purchased from Promega).

The results are shown in Figure 13a and Figure 13b, and the results show that the antibody can significantly activate the NF-kB signaling pathway.

### 3.3 T cell activation test to detect the activation of T cells by OX40 antibody

### 3.3.1 Isolation of peripheral blood mononuclear lymphocytes PBMC from human whole blood

The freshly obtained human whole blood was diluted with phosphate buffer PBS at a volume ratio of 1:1 to obtain the diluted whole blood, and the diluted whole blood was gently spread on the surface of Ficoll (purchased from GE Healthcare) with a sterile pipette, the volume ratio of Ficoll to diluted whole blood is 3:4. Avoiding shaking and mixing. Centrifuged at 400g at room temperature and 20°C gradient for 30 minutes. The centrifuge tube after centrifugation is divided into four layers, the second layer of milky white from top to bottom is mononuclear lymphocytes. Using a sterile pipette to gently absorb the cells in this layer, collecting them in a new centrifuge tube, diluting to three times the volume with PBS phosphate buffer, centrifuged at 200g for 10 minutes at room temperature, and discarding the supernatant. The lymphocytes were resuspended to 10 mL in PBS phosphate buffer, and the previous steps were repeated and washed twice to obtain peripheral blood mononuclear lymphocytes PBMC.

### 3.3.2 Isolation of human memory CD4+ T cells

Purified memory CD4+ T cells were isolated from the human peripheral blood mononuclear lymphocyte PBMC obtained in step 3.3.1 with the human memory CD4+ T cell extraction kit (purchased from Stem Cell, article number 19157). Refer to the kit instructions for the extraction method.

### 3.3.3 T cell activation experiment

Pre-coated a 96-well plate with 1 ug/ml anti-CD3 antibody and 2.2 ug/ml AffiniPure F(ab')₂ Fragment Goat Anti-Mouse IgG (purchased from Jaskson Immunoresearch), and incubated overnight at 4°C. Washed three times with PBS before use.

Diluting the sample solution to be tested with multi-component RPMI1640 medium (purchased from Invitrogen) containing 10% FBS and adding 100uL each well to the pre-coated 96-well plate. Then spreading the memory CD4+ T cells obtained in step 3.3.2 to a pre-coated 96-well plate with 1×10⁵ cells 100uL per well. After the reaction plate was incubated in a 37°C, 5% CO₂ incubator for 72 hours, the supernatant was collected and stored at -20°C for the determination of IFN gamma content.

### 3.3.4 Detection of cytokine IFN gamma content in cell supernatant

The detection of cytokine IFN gamma content in the cell supernatant uses the R&D system detection kit Human IFN-gamma DuoSet ELISA (DY285), and operates in accordance with the instructions.

Detecting the effect of OX40 antibody on the secretion of IFN gamma in the T cell activation experiment described in Example 3.3.2. The results are shown in Tables 5-9. In the T cell activation experiment, the antibody to be tested can enhance the secretion of IFN gamma from memory CD4+ T cells. Among them, mIgG1 is the antibody subtype control, and the data in the table is the IFN gamma value (pg/mL).

**Table5 The effect of OX40 antibody on IFN gamma secretion in T cell activation experiments (PBMC donor-1)**

| IFN gamma (pg/ml) | Antibody concentration(ug/ml) | | | | |
|---|---|---|---|---|---|
| Antibody name | 0.0016 | 0.008 | 0.04 | 0.2 | 1 |
| MAb007 | 2036.4 | 1480.8 | 1673.4 | 2161.2 | 2039.6 |
| MAb020 | 1809.3 | 2755.2 | 2437.3 | 2636.0 | 2373.2 |
| MAb021 | 1596.5 | 1427.1 | 1694.2 | 2613.3 | 2354.3 |
| MAb024 | 1343.8 | 1706.3 | 1945.9 | 1709.6 | 2103.7 |
| MIgG1 | 341.2 | 475.5 | 610.5 | 632.6 | 928.0 |

**Table 6 The effect of OX40 antibody on IFN gamma secretion in T cell activation experiments (PBMC donor-2)**

| IFN gamma (pg/ml) | Antibody concentration(ug/ml) | | | | |
|---|---|---|---|---|---|
| Antibody name | 0.0016 | 0.008 | 0.04 | 0.2 | 1 |
| MAb026 | 1167.1 | 1662.4 | 2258.7 | 3751.4 | 5438.1 |
| MAb033 | 1461.7 | 3008.5 | 5224.1 | 4399.3 | 4150.6 |
| MAb034 | 1299.4 | 2898.1 | 3082.7 | 2686.0 | 3068.5 |
| MAb035 | 1426.5 | 2609.4 | 3569.3 | 4245.3 | 4233.9 |
| MIgG1 | 794.2 | 818.2 | 1079.1 | 967.3 | 896.6 |

**Table 7 The effect of OX40 antibody on IFN gamma secretion in T cell activation experiment (PBMC donor-3)**

| IFN gamma (pg/ml) | Antibody concentration(ug/ml) | | | | |
|---|---|---|---|---|---|
| Antibody name | 0.0016 | 0.008 | 0.04 | 0.2 | 1 |
| MAb041 | 1895.0 | 2909.2 | 5449.0 | 7004.6 | 4930.4 |
| MAb047 | 1927.1 | 2197.8 | 4849.9 | 4775.0 | 5078.2 |
| MAb053 | 1063.7 | 1888.2 | 3226.6 | 3974.0 | 2598.6 |
| MAb064 | 905.8 | 1848.6 | 2631.4 | 2981.4 | 3259.2 |
| MIgG1 | 642.5 | 778.7 | 686.9 | 596.8 | 1515.9 |

**Table 8 The effect of OX40 antibody on IFN gamma secretion in T cell activation experiment (PBMC donor-4)**

| IFN gamma (pg/ml) | Antibody concentration(ug/ml) | | | | |
|---|---|---|---|---|---|
| Antibody name | 0.0016 | 0.008 | 0.04 | 0.2 | 1 |
| MAb049 | 2150.0 | 2940.8 | 5250.2 | 5721.2 | 5438.3 |
| MAb054 | 2044.2 | 4887.3 | 5316.9 | 5220.4 | 6739.3 |
| MAb055 | 1623.4 | 3051.0 | 4368.1 | 3738.8 | 5062.9 |
| MAb058 | 2159.8 | 4575.3 | 5937.3 | 4121.1 | 3870.2 |
| MAb069 | 2116.8 | 3128.5 | 4109.4 | 4480.5 | 4800.2 |
| MIgG1 | 602.7 | 811.1 | 451.8 | 408.4 | 965.5 |

**Table 9 The effect of OX40 antibody on IFN gamma secretion in T cell activation experiment (PBMC donor-5)**

| IFN gamma (pg/ml) | Antibody concentration(ug/ml) | | | | |
|---|---|---|---|---|---|
| Antibody name | 0.0016 | 0.008 | 0.04 | 0.2 | 1 |
| MAb003 | 3377.0 | 5217.5 | 6024.6 | 5636.0 | 6609.7 |
| MAb004 | 2709.0 | 3803.9 | 5892.4 | 4810.3 | 6788.2 |
| MAb010 | 2844.8 | 3993.7 | 4615.4 | 3955.6 | 3448.4 |
| MIgG1 | 1413.2 | 1710.2 | 1716.4 | 1116.1 | 1208.1 |

### 3.4 The inhibitory effect of OX40 antibody on the binding of OX40 and OX40L proteins

### 3.4.1 Construction of a cell line stably expressing OX40L

The nucleotide sequence encoding the full-length amino acid sequence of human-derived OX40L (the protein sequence number of human-derived OX40L in the Uniprot database is P23510) was cloned into the pLVX-IRES-Puro vector and the plasmid (herein referred to as pLVX-hOX40L-IRES-Puro) was prepared. After plasmid transfection of the CHOK1 cell line, it was subcloned in a 96-well culture plate by the limiting dilution method and selectively cultured in F12K medium containing 10% fetal bovine serum containing 10µg/ml puromycin, after about 2 weeks, selecting part of the monoclonal wells to be expanded into a 24-well plate, and about 3-4 days later to be expanded into a 6-well plate. The amplified clones were screened by flow cytometry with OX40L antibody (purchased from R&D). Choosing a cell line with better growth, higher fluorescence intensity, and monoclonal cell lines to continue to expand the culture and cryopreserve in liquid nitrogen to obtain a cell line stably expressing OX40L (herein referred to as CHOK1-hOX40L cells).

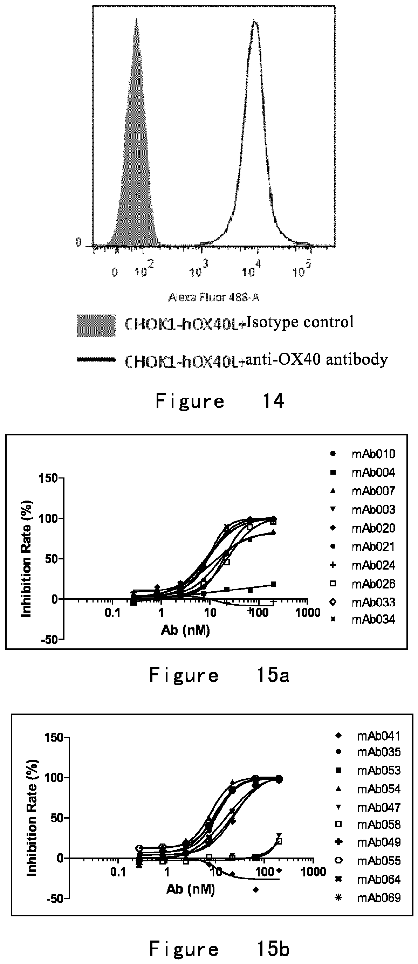

The FACS test results are shown in Figure 14. The results show that the selected cell line can highly express hOX40L.

### 3.4.2 The inhibitory effect of OX40 antibody on the binding of OX40 and OX40L protein

The CHOK1-hOX40L stable cell line constructed in Example 3.4.1 was expanded and cultured in a cell culture dish to 90% confluence, the medium was removed, washed with PBS, the cells were treated and collected with cell digestion solution TrypLE (purchased from Invitrogen). Washing the cells with FACS buffer (PBS+2%FBS). After counting the cells, resuspending the cells to 3×10⁶/mL in FACS buffer, adding 100 µL per well to a 96-well FACS reaction plate, and centrifuged at 300g for 5 minutes and the supernatant was removed, a series of concentration gradients of 50 µL of the purified OX40 antibody obtained in Example 2 were added to each well, and at the same time, adding 50 µL of biotin-labeled OX40 ECD-Fc protein per well, and incubated at 4°C for 1 hour. Washed twice with FACS buffer by centrifugation, adding 100 µL fluorescent (Alexa 488)-labeled streptavidin (purchased from Invitrogen) per well, and incubated for 1 hour at 4°C in the dark. Washing the cells by centrifugation with FACS buffer for 3 times, adding 100 µL PBS buffer per well to suspend the cells, using FACS (FACS Canto II, purchased from BD) to detect and analyze the results.

The results are shown in Figure 15a and Figure 15b. The results show that most of the antibodies of the present invention can inhibit the binding of OX40 and OX40L proteins.

### EXAMPLE4 Determination of the amino acid sequence of the variable region of the light and heavy chains

Isolation of total RNA: After the supernatant obtained from the subclonal culture in Example 1 was tested for antigen binding (that is, after the verification and activity determination in Example 3), 5×10⁷ hybridoma cells were collected by centrifugation, and 1 mL Trizol was added and mixed well and transferred to a 1.5mL centrifuge tube, placed at room temperature for 5 minutes; adding 0.2mL chloroform, shaked for 15 seconds, placed for 2 minutes, centrifuged at 12000g at 4°C for 5 minutes, taking the supernatant and transferred to a new 1.5mL centrifuge tube; adding 0.5mL isopropanol, gently mixing the liquid in the tube, placed at room temperature for 10 minutes, centrifuged at 4°C, 12000g for 15 minutes, discarding the supernatant; adding 1mL of 75% ethanol, gently washing the precipitate, 4 °C , after centrifugation at 12000g for 5 minutes, the supernatant was discarded, the precipitate was air-dried, and DEPC-treated H₂O was added to dissolve it (55 °C water bath to promote solvent for 10 minutes) to obtain total RNA.

Reverse transcription and PCR: Taking 1µg of total RNA, configuring a 20µL system, adding reverse transcriptase and reacted at 42°C for 60 minutes, and at 7°C for 10 minutes to terminate the reaction. Configuring 50µL PCR system, including 1µL cDNA, 25pmol of each primer, 1 µL DNA polymerase and matching buffer system, 250 µmol dNTPs; setting up PCR program, 95°C pre-denaturation for 3 minutes, 95°C denaturation for 30 seconds, annealed at 55°C for 30 seconds, extended at 72° C for 35 seconds, and then extended at 72° C for an additional 5 minutes after 35 cycles to obtain a PCR product. The kit used for reverse transcription is PrimeScript RT Master Mix, purchased from Takara, item number RR036; the kit used for PCR includes Q5 ultra-fidelity enzyme, purchased from NEB, item number M0492.

Cloning and sequencing: Taking 5µl of PCR product for agarose gel electrophoresis detection, and using the column recovery kit to purify the test positive samples. The recovery kit is Gel&PCR Clean-up, purchased from MACHEREY-NAGEL, catalog number 740609. Carrying out ligation reaction: sample 50ng, T vector 50ng, ligase 0.5µL, buffer 1µL, reaction system 10µL, reacted at 16°C for half an hour to obtain the ligation product, the ligation kit is T4 DNA ligase, purchased from NEB, catalog number M0402; Taking 5µl of the ligation product and adding to 100µL of competent cells (Ecos 101 competent cells, purchased from Yeastern, Catalog No. FYE607), ice bath for 5 minutes, then heat shock in a water bath at 42°C for 1 minute, putting it back on ice for 1 minute and adding 650µL antibiotic-free SOC medium, resuscitated on a 37°C shaker at 200 rpm for 30 minutes, taking out 200 µL and spreading it on LB solid medium containing antibiotics and incubated overnight at 37°C incubator; the next day, using primers M13F and M13R on the T vector to configure a 30µL PCR system for colony PCR. Using a pipette tip to dip the colony into the PCR reaction system and pipette, and aspirate 0.5µL and place it on another LB solid petri dish containing 100nM ampicillin to save the strain; after the PCR reaction was over, taking out 5µL for agarose gel electrophoresis detection, and sequencing and analysis of positive samples [see Kabat, "Sequences of Proteins of Immunological Interest", National Institutes of Health, Bethesda, Md. (1991)].

See the sequence information of the present invention in the appendix for the sequencing results.

### EXAMPLE 5 Epitope identification of OX40 antibody

According to the sequence in the published patent, we prepared four control antibodies, namely control antibody 1 (MOXR0916), control antibody 2 (PF-04518600), control antibody 3 (MEDI0562), and control antibody 4 (BMS-986178) . The OX40 antibody obtained in Example 1 and the four control antibodies were respectively subjected to competitive ELISA experiments. The results are shown in Table 10. The percentage in the table is the percentage of competition. The higher the percentage of competition, the closer the epitopes of the two antibodies are. Among them, mAb007, mAb021, mAb024, mAb041, and mAb058 have different epitopes from the four control antibodies.

**Table 10 Competitive ELISA experiment**

| Antibody name | Compete with MOXR0916 | Compete with PF-04518600 | Competing with MEDI0562 | Compete with BMS-986178 |
|---|---|---|---|---|
| MAb010 | 56% | -19% | 96% | -6% |
| MAb049 | 90% | -2% | 97% | 0% |
| MAb064 | 90% | 4% | 96% | 1% |
| MAb069 | 73% | -1% | 96% | 1% |
| MAb004 | -2% | -12% | 92% | -8% |
| MAb026 | 3% | -13% | 63% | 11% |
| MAb035 | 3% | -5% | 59% | 90% |
| MAb047 | -3% | -2% | 97% | 0% |
| MAb003 | -2% | 94% | -3% | 5% |
| MAb053 | -2% | 93% | 11% | 2% |
| MAb054 | -2% | 95% | 11% | 18% |
| MAb055 | -3% | 95% | 16% | 3% |
| MAb020 | -4% | -15% | -3% | 50% |
| MAb033 | -1% | -7% | 7% | 81% |
| MAb034 | 0% | 5% | 13% | 93% |
| MAb007 | -2% | 16% | -3% | -6% |
| MAb021 | -2% | -10% | 3% | 7% |
| MAb024 | -1% | -7% | 9% | -2% |
| MAb041 | 1% | -8% | 2% | -6% |
| MAb058 | -3% | 11% | 26% | -4% |

### EXAMPLE 6 Preparation and identification of OX40 human-mouse chimeric antibody

### 6.1 Plasmid construction and preparation

Purified OX40 antibody has been obtained from the culture supernatant of hybridoma cells in Example 2, and according to the sequencing results of Example 4, the heavy chain variable region and light chain variable region sequences of the OX40 antibody have been identified. The heavy chain variable region sequence of the OX40 antibody was recombined into an expression vector containing the signal peptide and the human-derived heavy chain antibody IgG1 constant region, and the light chain variable region sequence of the OX40 antibody was recombined to an expression vector containing signal peptide and human-derived antibody light chain kappa constant region, a recombinant plasmid was obtained and verified by sequencing (the sequencing method is the same as that in Example 4). Using an alkaline lysis kit (purchased from MACHEREY-NAGEL) to extract the recombinant plasmids with improved purity with a mass of 500µg or more, and filtering it through a 0.22µm filter membrane (purchased from Millipore) for transfection.

### 6.2 Cell transfection

Culturing 293E cells (purchased from Invitrogen) in Freestyle 293 expression medium (purchased from Invitrogen). The shaker is set to 37°C, 130RPM, and 8% CO₂ (v/v) concentration.

In Freestyle 293 expression medium, 10% (v/v) F68 (purchased from Invitrogen) was added during transfection to a final concentration of F68 at 0.1% (v/v) to obtain Freestyle 293 expression culture containing 0.1% (v/v) F68, that is, medium A.

Taking 5mL medium A and 200µg/mL PEI (purchased from Sigma) and mixed well to obtain medium B. Taking 5 mL of medium A and 100 µg/mL of the recombinant plasmid obtained in step 6.1 and mixed well to obtain medium C. After 5 minutes, the medium B and the medium C were combined and mixed, and the mixture was allowed to stand for 15 minutes to obtain a mixture D. Adding 10mL mixture D slowly to 100mL Freestyle 293 expression medium containing 293E cells until the cell density of 293E was 1.5×10⁶/mL, and shaking while adding to avoid excessive concentration of PEI. Placing it in a shaker for culture. Peptone was added the next day to a final concentration of 0.5% (w/v). On the 5th to 7th day, the antibody titer of the culture medium was measured. On the 6th to 7th day, the supernatant was collected by centrifugation (3500 rpm, 30 minutes), and filtered through a 0.22µm filter membrane to obtain the filtered cell supernatant for purification.

### 6.3 Antibody purification and identification

For continuous production of endotoxin-free chromatography columns and Protein A packing (purchased from GE), using 0.1M NaOH for 30 minutes or 5 times the column volume of 0.5M NaOH for washing; for column materials and chromatography columns that have not been used for a long time, using 1M NaOH to soak for at least 1 hour, rinsed with non-endotoxic water to neutrality, and washing the column material with 10 times the column volume of 1% Triton × 100. Using 5 column volumes of PBS (PBS phosphate buffer, pH 7.2) for equilibration, putting the filtered cell supernatant obtained in step 6.2 on the column, and collecting the flow-through if necessary. After loading the column, washed with 5 times the column volume of PBS. Eluted with 5 column volumes of 0.1M Glycine-HCl pH3.0, collecting the eluate, and neutralized with 0.5 times column volume eluent of 1M Tris-HCl (1.5M NaCl) with pH 8.5, and harvesting OX40 human mouse chimeric antibody. Dialysis in 1×PBS for 4 hours to avoid endotoxin contamination. After dialysis, using spectrophotometry or a kit to determine the concentration, using HPLC-SEC to determine the purity of the antibody, and using an endotoxin detection kit (purchased from Lonza) to detect the content of antibody endotoxin. The characteristics of the obtained OX40 human-mouse chimeric antibody were identified (same as Example 3).

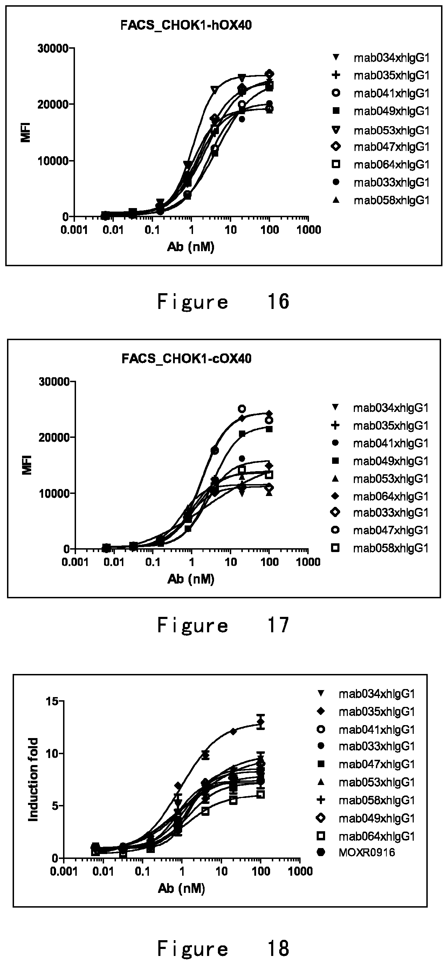

The test results are shown in Figure 16, Figure 17, Figure 18 and Table 11. Wherein MOXR0916 is the positive control antibody, and hIgG1 is the antibody subtype control.

**Table 11 Effect of OX40 human-mouse chimeric antibody on IFN gamma secretion in T cell activation experiment**

| IFN gamma (pg/ml) | Antibody concentration (ug/ml) | | | | | |
|---|---|---|---|---|---|---|
| Antibodv name | 1 | 0.2 | 0.04 | 0.008 | 0.0016 | 0 |
| MAb041 × hIgG1 | 4985.0 | 5071.4 | 6794.6 | 5352.7 | 2438.9 | 731.9 |
| MAb058 × hIgG1 | 3918.4 | 5054.8 | 5102.0 | 3992.2 | 3137.7 | 709.5 |
| MAb004 × hIgG1 | 4088.8 | 5074.6 | 4343.5 | 5612.0 | 3477.5 | 667.7 |
| MAb033 × hIgG1 | 4646.7 | 3502.5 | 5833.2 | 5032.4 | 2273.8 | 667.7 |
| MAb034 × hIgG1 | 4636.9 | 4673.1 | 4847.4 | 5191.5 | 3805.2 | 667.7 |
| MAb035 × hIgG1 | 5168.6 | 4993.0 | 4439.2 | 4737.1 | 4168.9 | 609.5 |
| MAb047 × hIgG1 | 5173.7 | 5704.0 | 6132.3 | 3252.5 | 1129.7 | 609.5 |
| MAb049 × hIgG1 | 3855.0 | 4722.6 | 4258.1 | 3943.8 | 1781.2 | 609.5 |
| MOXR0916 | 4469.0 | 3937.6 | 5007.3 | 2454.2 | 1418.5 | 709.5 |
| HIgG1 | 569.4 | 695.8 | 790.8 | 742.4 | 717.8 | 709.5 |

The results show that the obtained OX40 human-mouse chimeric antibody can bind well to both human OX40 and monkey OX40, can activate the NF-kB signaling pathway and can significantly increase the expression of IFN gamma in memory CD4+ T cells. Compared with the positive control antibody MOXR0916, mab035xhIgG1 has a better effect on activating T cells.

### EXAMPLE 7 In vivo efficacy experiment of OX40 antibody

In order to study the anti-tumor effect of OX40 antibody in vivo, OX40 humanized mouse colon cancer MC-38 subcutaneous tumor model was established. OX40 humanized mice were purchased from Beijing Biocytogen Co.,Ltd, female, 5-6 weeks old. 100 µL of cell suspension (cells suspended in base DMEM medium) containing 1x10⁶ MC-38 tumor cells was inoculated subcutaneously on the right side of the mouse. Animals with a tumor volume of 30 mm³ ∼ 80 mm³ will be introduced into the experiment and randomly divided into different test groups according to the tumor volume. The animal experiment grouping and dosing schedule are shown in Table 12 below.

**Table 12 Animal experiment grouping and dosing schedule**

| **Group** | **Name of administration** | **Dose of administration (mg/kg)** | **Number of mice** | **Administration route** | **Administration cycle** |
|---|---|---|---|---|---|
| 1 | Control (Vehicle) | / | 10 | Intraperitoneal injection | Twice a week for three weeks |
| 2 | MOXR0916 | 3 | 10 | | |
| 4 | BMS-986178 | 3 | 10 | | |
| 5 | Mab035xhIgG1 | 3 | 10 | | |
| 6 | Mab041xhIgGl | 3 | 10 | | |

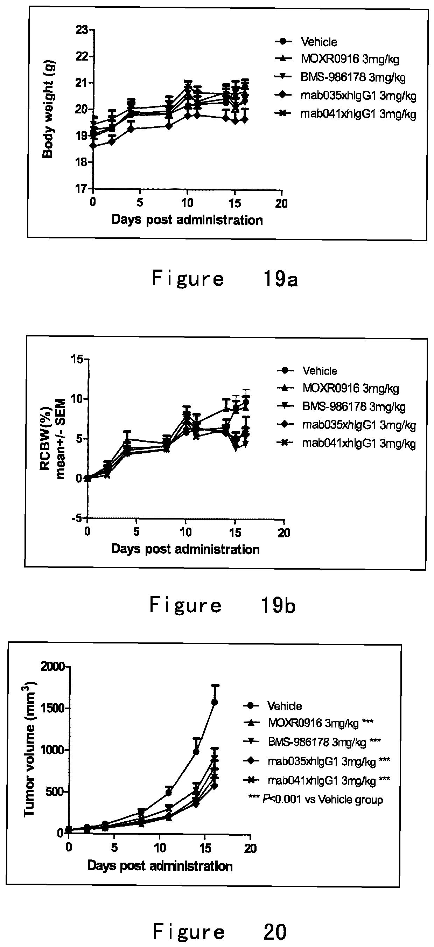

After the administration, the weight of the mice was recorded 2-3 times a week, and the tumor diameter was measured with a vernier caliper. The calculation formula of tumor volume is: V=a×b²/2, wherein a and b represent the long diameter and short diameter of the tumor, respectively. Each mouse reached the experimental endpoint (weight loss greater than 20%; or tumor volume greater than 2,000 mm³) and was euthanized and the survival time was recorded, Kaplan-Meier survival curve. Changes in body weight and tumor volume were analyzed by Two-way ANOVA, Kaplan-Meier survival curve was analyzed by Log-Rank, and P<0.05 was considered as a significant difference. The experimental results are shown in Figure 19a, Figure 19b, Figure 20, and Figure 21. Compared with the solvent control (vehicle) group, OX40 antibody can significantly inhibit tumor growth, and mab035xhIgG1 has the best effect on prolonging the life cycle of mice.

### EXAMPLE 8 Preparation of humanized antibodies

After sequence analysis, the candidate antibody mAb035 has no important hotspots in the heavy chain variable region and the light chain variable region. Through sequence alignment (NCBI-Igblast), the germline gene sequence with the highest homology of the heavy chain variable region and the variable region of the light chain of the candidate antibody mAb035 is selected as the variable region transplantation framework: IGHV1-46*03 (61.2%) and IGKV1-12*01 (67.4%). After selecting the human antibody framework, through homology modeling, predicting the key amino acids that may determine the structure in the mouse anti-constant region, and the grafted framework region is designed for back mutation.

According to the above principles, designing 10 heavy chain variable region sequences (mab035VH.g0, mab035VH.gl, mab035VH.g2, mab035VH.g3, mab035VH.g4, mab035VH.g5, mab035VH.g6, mab035VH.g7, mab035VH.g8, mab035VH.g9) and 5 light chain variable region sequences (mab035VL.g0, mab035VL.gl, mab035VL.g2, mab035VL.g3, mab035VL.g4). Subsequently, cross-combination was performed for expression, and a total of the following 28 humanized antibodies were obtained, as shown in Table 13.

**Table 13 humanize antibody expression combinations**

| | **Mab035VL.g 0** | **Mab035VL.g 1** | **Mab035VL.g 2** | **Mab035VL.g 3** | **Mab035VL.g 4** |
|---|---|---|---|---|---|
| **Mab035VH. g0** | Humab035-1 | Humab035-2 | Humab035-3 | Humab035-4 | |
| **Mab035VH. g1** | Humab035-5 | Humab035-6 | Humab035-7 | Humab035-8 | |
| **Mab035VH. g2** | Humab035-9 | Humab035-10 | Humab035-11 | Humab035-12 | |
| **Mab035VH. g3** | Humab035-13 | Humab035-14 | Humab035-15 | Hu Mab035-16 | |
| **Mab035VH. g4** | | Hu Mab035-17 | | | Hu Mab035-23 |
| **Mab035VH. g5** | | Hu Mab035-18 | | | Hu Mab035-24 |
| **Mab035VH. g6** | | Hu Mab035-19 | | | Hu Mab035-25 |
| **Mab035VH. g7** | | Hu Mab035-20 | | | Hu Mab035-26 |
| **Mab035VH. g8** | | Hu Mab035-21 | | | Hu Mab035-27 |
| **Mab035VH. g9** | | Hu Mab035-22 | | | Hu Mab035-28 |

Vector construction: The amplification primers were synthesized by Genewiz, and then the variable regions of the light chain and the heavy chain were amplified separately by PCR method. Configuring 50 µL reaction system, including 50-100ng heavy chain variable region, light chain variable region, 1ul forward and reverse primers, 1ul pfxD enzyme (purchased from invitrogen, 12344-012), 10*pfx buffer 5ul (Same supplier as pfx enzyme) and adding water to make up to 50 µL. Setting up the PCR program, pre-denaturation at 95°C for 5 minutes, denaturation at 95°C for 30 seconds, annealing at 56°C for 30 seconds, extension at 68°C for 30 seconds, after 25 cycles, an additional extension at 68°C for 10 minutes to obtain a PCR product. Taking 5 µL of the PCR product for agarose gel electrophoresis detection, and using the recovery kit to purify the test positive samples. The recovery kit is PureLink Quick Gel extraction kit, purchased from Qiagen, catalog number 28706.

Preparation of humanized antibody: Carrying out ligation reaction: inserting fragment 20-40ng, digested expression vector 60-100ng, recombinase Exnase (purchased from Vazyme, catalog number C112-01/02) 1 µL, buffer 2 µL, the reaction system is 10 µL and reacted at 37°C for half an hour to obtain the ligation product, which is the constructed recombinant vector. The buffer is the buffer used for the purchase of the recombinase; the heavy chain variable region is directional cloned into the expression vector containing the signal peptide and the human antibody heavy chain IgG4 (S228P) constant region (wherein the expression vector is purchased from Invitrogen, the recombination step is a normal step), the light chain variable region is directional cloned into an expression vector containing a signal peptide and a human antibody light chain kappa constant region (wherein the expression vector is purchased from Invitrogen, and the recombination step is a conventional step). Adding 10 µL of the ligation product to 100 µL of competent cells (Ecos 101 competent cells, purchased from Yeastern, Catalog No. FYE607), heat shock in a 42 °C water bath for 60 seconds, putting it back on ice for 3 minutes, taking out 80 µL and spreading it on the LB solid medium containing ampicillin, incubated overnight at 37° C in an incubator. The next day, using the primers pEF1A and pSV40 on the expression vector to configure a 30 µL PCR system for colony PCR. The colony PCR system is: 1 µL for each primer, 15 µL of PCR master mix (purchased from Novoprotein), making up to 30 µL. Using a pipette tip to dip the colony into the PCR reaction system and pipette, and aspirating 0.5 µL onto another LB solid petri dish containing 100 µg/mL ampicillin to preserve the strain. After the PCR reaction, 4.5 µL was taken out for agarose gel electrophoresis detection, and the positive samples were sequenced.

The recombinant antibody heavy and light chain expression vectors with the correct sequence were amplified, and then transiently transfected into FreeStyle™ 293-F cells (purchased from Invitrogen) to produce antibodies. During transfection, the density of 293-F cells should be 1-1.2×10⁶ cells/mL, and for 100 mL of cells, 100 µg of the constructed recombinant vector and 200 µg of the transfection reagent polyethyleneimine (PEI) were required. The recombinant vector and PEI were added to 5 mL of culture medium, and the mixture was allowed to stand at room temperature for 5 minutes. After filtration with 0.22 µm filter membrane, the mixture of recombinant vector and PEI was allowed to stand at room temperature for 15 minutes. Then the above mixture was slowly added to the cells, and cultured in a 37°C, 8% (v/v) CO₂ incubator at 130 rpm. The culture supernatant and cell pellet are taken every day to detect the expression of antibodies. After 5 days, the cell culture solution was centrifuged at 3000 g for 30 minutes, the supernatant was collected, and filtered with a 0.22 µm filter. Purifying the monoclonal antibody in 200 mL of clear supernatant with 1 mL MabSelect™SuRe™column (purchased from GE Healthcare). MabSelect™SuRe™column was first equilibrated with equilibration buffer (PBS phosphate buffer, pH7.2). After loading the sample, washing the MabSelect™SuRe™column with equilibration buffer. The volume of the equilibration buffer is 5 times the volume of the protein A column bed. The monoclonal antibody bound to the MabSelect™ SuRe™ column was eluted with an eluent (0.1 M glycine hydrochloride buffer, pH 3.0). Collecting the eluted antibodies and adding 10% (v/v) 1.0 M Tris-HCl buffer to neutralize the pH. Then immediately dialyzed with PBS phosphate buffer overnight. Collecting the dialyzed monoclonal antibodies, filtering them aseptically with a 0.22 µm filter, and storing them aseptically to obtain purified humanized OX40 antibodies. The obtained antibody was tested and analyzed for protein concentration and purity.

The results are shown in Table 14 below. The results show that the yield and purity analysis of humanized antibody are normal.

**Table 14 Humanized antibody expression and purification results**

| **Antibody name** | **Volume (mL)** | **batch number** | **Concentrat ion (mg/ml)** | **Yield (mg)** | **Purity (% SEC)** |
|---|---|---|---|---|---|
| Humab035-1 | 1.8 | 2018071901 | 1.18 | 2.124 | 99.018 |
| Humab035-2 | 1.8 | 2018071902 | 1.261 | 2.270 | 99.367 |
| Humab035-3 | 1.9 | 2018072503 | 0.504 | 0.958 | 100 |
| Humab035-4 | 1.8 | 2018071904 | 1.51 | 2.718 | 99.592 |
| Humab035-5 | 1.7 | 2018071905 | 1.288 | 2.190 | 99.557 |
| Humab035-6 | 1.7 | 2018071906 | 1.012 | 1.720 | 99.696 |
| Humab035-7 | 1.8 | 2018071907 | 1.169 | 2.104 | 99.609 |
| Humab035-8 | 1.8 | 2018071908 | 1.186 | 2.135 | 99.872 |
| Humab035-9 | 1.8 | 2018071909 | 1.078 | 1.940 | 99.400 |
| Humab035-10 | 1.8 | 2018071910 | 1.171 | 2.108 | 99.358 |
| Humab035-11 | 1.8 | 2018071911 | 0.989 | 1.780 | 99.421 |
| Humab035-12 | 1.8 | 2018071912 | 1.206 | 2.171 | 99.718 |
| Humab035-13 | 1.8 | 2018071913 | 1.176 | 2.117 | 97.173 |
| Humab035-14 | 1.8 | 2018071914 | 0.813 | 1.463 | 95.878 |
| Humab035-15 | 1.8 | 2018072515 | 0.902 | 1.6236 | 96.390 |
| Humab035-16 | 1.8 | 2018071916 | 1.241 | 2.234 | 98.147 |
| Humab035-17 | 1.7 | 2018100901 | 2.569 | 4.367 | 97.88 |
| Humab035-18 | 1.7 | 2018100902 | 2.425 | 4.123 | 97.87 |
| Humab035-19 | 1.7 | 2018100903 | 2.868 | 4.876 | 97.36 |
| Humab035-20 | 1.7 | 2018100904 | 2.074 | 3.526 | 97.84 |
| Humab035-21 | 1.7 | 2018100905 | 2.456 | 4.175 | 97.68 |
| Humab035-22 | 1.7 | 2018100906 | 2.407 | 4.092 | 98.10 |
| Humab035-23 | 1.7 | 2018100907 | 2.279 | 3.874 | 98.56 |
| Humab035-24 | 1.7 | 2018100908 | 1.731 | 2.943 | 98.90 |
| Humab035-25 | 1.7 | 2018100909 | 2.232 | 3.794 | 98.66 |
| Humab035-26 | 1.7 | 2018100910 | 2.151 | 3.657 | 98.61 |
| Humab035-27 | 1.7 | 2018100911 | 2.306 | 3.920 | 98.58 |
| Humab035-28 | 1.7 | 2018100912 | 1.952 | 3.318 | 100.00 |

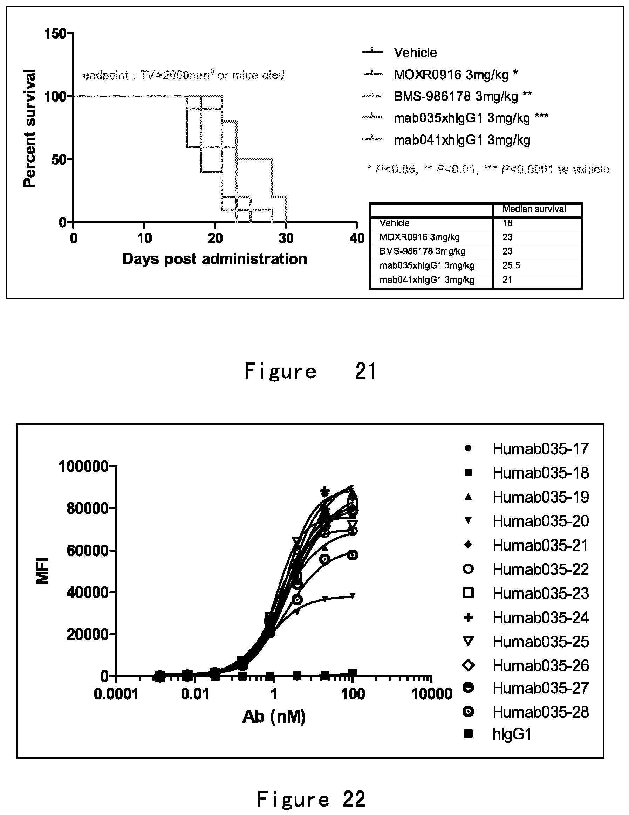

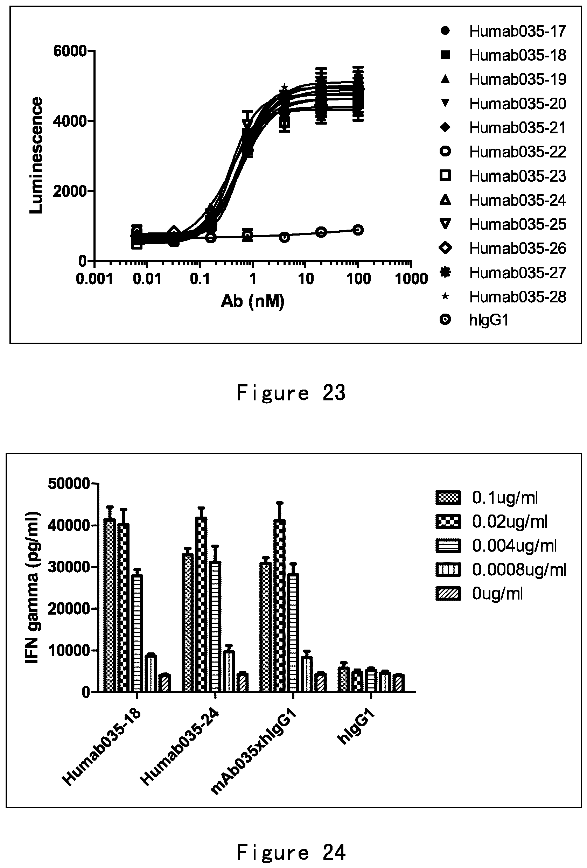

Activity identification of humanized antibody (the method is the same as in Example 3)
A. Flow cytometry (FACS) detects the binding of antibodies to cells expressing human OX40. The results are shown in Figure 22. The antibodies obtained can all bind to the human-derived OX40 on the cell surface. The isotype control is human IgG1, and the data in the figure is the mean fluorescence intensity (MFI) of the measured cell population.
B. The NF-kB reporter gene experiment detects the activation of the antibody on the NF-kB signaling pathway. The results are shown in Figure 23. The detection antibody can activate the NF-kB signaling pathway. The isotype control is human IgG1, and the data in the figure is luminous intensity (Luminescence).
C. The T cell activation test detects the activation effect of antibodies on T cells. The results are shown in Figure 24. The detection antibody can significantly increase the expression of IFN gamma in memory CD4+ T cells and activate T cells. The isotype control is human IgG1, and the data in the figure is the content of IFN gamma in the supernatant.

All documents mentioned in the present invention are incorporated by reference herein as if each document were incorporated separately by reference. Furthermore, it should be understood that after reading the foregoing teachings of the invention, various changes or modifications may be made to the invention by those skilled in the art and that these equivalents are equally within the scope of the claims appended to this application.

### Appendix Sequence information of the present invention

### MAb035-H

SEQ ID NO. 1 Amino Acid Sequence (116aa) 
QVQLQQSGAELVRPGASVTLSCKASDYTFTDYEMHWVYQTPVHGLEWIGAIDPETGGTAY
NQKFKDKAILTADKSSNTAYMELRSLTSEDSAVYFCTKTGYQFDYWGQGTTLTVSS
SEQ ID NO. 2 > 3626-mAb035-13-68A8G10-H (348 bp)

CAGGTTCAACTGCAGCAGTCTGGGCTGAGCTGGGGAGGCCTGGGCTTCAGTGACGCTG
CCTGCAAGGCTTCGGGCTTCCTGCAAGGCTTCGGACTGACTGACTATGAAATGCACTGGAA
ATGCACTGGCCTGGGAATGGAATGGCCTGGGAATGGGAATTGCCTGGGAATGGCCTGGGA
ATTGGCCTGGGAATTGCCTGGGAATTGCCTGGGAATTGCCTGGGGGACATTGCCTGGGGG
ACATTGCCTGGGCCGCAGCCTGGGACATGATTGCCTGGGGACATTGCCTGGGACATTGCCT
GGGGACATTGCCGCAGCCTGACTGACTGAGGACTCTGCCGTCTATTTCTGTACAAAAACTG
GGTACCAGTTTGGACTACTGGGGCCAAGGCACCACTCTCTCCTCA

| region | Sequence fragment | Position in SEQ ID NO.1 | Sequence Number SEQ ID NO. | Length |
|---|---|---|---|---|
| HFR1 | Amino acids 1-30 in SEQ ID NO.1 | 1 - 30 | | 30 |
| CDR-H1 | DYEMH | 31 - 35 | 3 | 5 |
| HFR2 | Amino acids 36-39 in SEQ ID NO.1 | 36 - 49 | | 14 |
| CDR-H2 | AIDPETGGTAYNQKFKD | 50 - 66 | 4 | 17 |
| HFR3 | Amino acids 67-98 in SEQ ID NO.1 | 67 - 98 | | 32 |
| CDR-H3 | TGYQFDY | 99 - 105 | 5 | 7 |
| HFR4 | Amino acids 67-98 in SEQ ID NO.1 | 106 - 116 | | 11 |

### MAb035-L

SEQ ID NO. 6 Amino Acid Sequence: (107aa) 
DIVMTQSQKFMSTSVGDRVSVTCKASQNVGSNVAWYQQKPGQSPKALIYSASYRYSGVPD
RFAGSGSGTDFTLTISNVQSEDLADYFCQQYNSHPYTFGGGTKLEIK
SEQ ID NO. 7 > 3626-mAb035-13-68A8G10-L (321 bp)

GACATTGTGATGACCCAGTCTCAAAATTCATGTCCACATCAGTAGGAGACAGGGTCAA
GGCAAGGGTCAAGGCAAGGCCAGTAGTAGTAGTAGTAGTAGTAGTAGTAGTAGATCTCCT
AAGATTGATTTCGGATCTGGATCTGGATCTGGACAGATTGATTGTCAGTGATTGTCAGATT
GTCAGATTGTCAGATTGTCAGATTGTCAGATTGTCAGATTGTCAGATTGTCAGATTGTCAG
ATTGTCAGATGTCAGATGTCAGATGTCAGATGTCAGATGTCAGATGTCAGATGTCAGATGT
CAGATGTCAGATGTCAGATGTCAGATGTCAGATGTCAGATGTCAGATGTCAGATGTCAGCAATACAAC
AGCCATCCGTACACGTTCGGAGGGGGACCAACTGGAAATAAAA

| region | Sequence fragment | Position in SEQ ID NO. 6 | Sequence Number SEQ ID NO. | Length |
|---|---|---|---|---|
| LFR1 | Amino acids 1-23 in SEQ ID NO.6 | 1 - 23 | | 23 |
| CDR-L1 | KASQNVGSNVA | 24 - 34 | 8 | 11 |
| LFR2 | Amino acids 35-49 in SEQ ID NO. 6 | 35 - 49 | | 15 |
| CDR-L2 | SASYRYS | 50 - 56 | 9 | 7 |
| LFR3 | Amino acids 57-88 in SEQ ID NO. 6 | 57 - 88 | | 32 |
| CDR-L3 | QQYNSHPYT | 89 - 97 | 10 | 9 |
| LFR4 | Amino acids 98-107 in SEQ ID NO.6 | 98 - 107 | | 10 |

### MAb053-H

SEQ ID NO. 11 Amino Acid Sequence: (112aa) 
QVQLKQSGPGLVQPSQSLSIICTVSGFSLSDYGVMWVRQSPGKGLEWLGVIWSGGSTDYNA
AFTSRLSISRDNSKRQVIFKMNSLQAEDTAIHYCVRETMDYWGQGTSVTVS
SEQ ID NO. 12 > 3626-mAb053-22-82G5B7-H (339 bp)

CAGGTGCAGCTGAAGCAGTCAGGACCTGCCTAGTGCAGCCTAGTGCAGCACAGACCTG
TCCTCCCTCACAGACCTGTTCTCTCATTAAGTGACTATGGGGTTCTGTAATGTGTGTGGA
TTGGGGTGGGTGGGTGGGTGGGGTGGGGTGGGGTGGGGTGGGGTGGGGTGGGGTGGGGTG
GGGTGGGGTGGGGGGGGGGGGGGGGGGGGGGGGGGGGGGGGGGGGGGGGGGGGGGGGGGG
GGGGGGGGGGGGGGGGGGGGGGGGGGGGGGGGGGGGGGGGGGGGGGGGGCAGCCATACAT
TACTGTGTCAGAGAAACGATGGGACTACTGGGGTCAAGGAACCTCAGTCGTCTCCTCA

| region | Sequence fragment | Position in SEQ ID NO. 11 | Sequence Number SEQ ID NO. | Length |
|---|---|---|---|---|
| HFR1 | Amino acids 1-30 in SEQ ID NO.11 | 1 - 30 | | 30 |
| CDR-H1 | DYGVM | 31 - 35 | 13 | 5 |
| HFR2 | Amino acids 36-49 in SEQ ID NO.11 | 36 - 49 | | 14 |
| CDR-H2 | VIWSGGSTDYNAAFTS | 50 - 65 | 14 | 16 |
| HFR3 | Amino acids 66-97 in SEQ ID NO.11 | 66 - 97 | | 32 |
| CDR-H3 | ETMDY | 98 - 102 | 15 | 5 |
| HFR4 | Amino acids 103-112 in SEQ ID NO.11 | 103 - 112 | | 10 |

### MAb053-L

SEQ ID NO. 16 Amino Acid Sequence: (107aa) 
DIQMTQTTSSLSASLGDRVTISCRASQDISNYLNWYQQRPDGTVKRLIYYTSRLHSGVPSRF
SGSGSGTDYSLTISSLEQEDIATYFCQQAHTLPWTFGGGTKLEIK
SEQ ID NO. 17 > 3626-mAb053-22-82G5B7-L (321 bp)

GATATCCAGATGACACAGACTACATCCCCTGTCTGCCTCTCTGGGAGACAGAGTCACCA
TCAGTTGCAGGCAAGTGCAAGTGCAAGTGCAAGATTGTAAACTGGTAACTGGTAACTGGT
AACTGGTAACTGGTAACTGGTAGGGGTGTGGCAGTGGGTCTGGAACATTAGGGGGTGTGG
CACACTGGGGTGTGGCACACTGGGGGGGGGGGGGGGGGGGGGGGGGGGGGGGGGGGGGG
GGGGGGGGGGGGGGGGGGGGGGGGGGGGGGGGGGGGGGGGGGGGGGGGGGGGGGGGGGG
GGGGGGGGGCTCATACGCTTCCGTGGACGTTCGGGAGGCACCAAGTTGGAGATCAAA

| region | Sequence fragment | Position in SEQ ID NO. 16 | Sequence Number SEQ ID NO. | Length |
|---|---|---|---|---|
| LFR1 | Amino acids 1-23 in SEQ ID NO.16 | 1 - 23 | | 23 |
| CDR-L1 | RASQDISNYLN | 24 - 34 | 18 | 11 |
| LFR2 | Amino acids 35-49 in SEQ ID NO.16 | 35 - 49 | | 15 |
| CDR-L2 | YTSRLHS | 50 - 56 | 19 | 7 |
| LFR3 | Amino acids 57-88 in SEQ ID NO.16 | 57 - 88 | | 32 |
| CDR-L3 | QQAHTLPWT | 89 - 97 | 20 | 9 |
| LFR4 | Amino acids 98-107 in SEQ ID NO.16 | 98 - 107 | | 10 |

### MAb041-H

SEQ ID NO. 21 Amino Acid Sequence: (112aa) 
EVQLVESGGGLVKPGGSLKLSCAVSGFTFSNSAMSWVRQTPEKRLEWVATITDGGDYTYY
SDNVKGRFTISRDNAKNNLYLQMSHLKSEDTAMYYCVRGISYWGQGTLVTVS
SEQ ID NO. 22 > 3626-mAb041-17-45E5G11-H (339 bp)

GAAGTGCAGCTGGTGGAGTCTGGGAGGTAGTGAAGCCTGGAGGGCCTGGAGGGCCTGG
AGGGCCTGCAGTCTCTGGATTCTCTGGATTCTGGATTCTGCCATGGACTCTGCCATGGGGG
TTCTGCCATGGAAGAGGCTGCCAGACTCTGCCATGGGGGGTTCGCCAGACTCTGCCATGGG
GGGTTCGCCAGACTCTGCCGGGGGGTTCGCCAGACTCTGCCGGGGGTGGGGTGGGGTCTG
CCAGACTCTGCCGGGGGTGGGGGGGGGGGGGGGGGGGGGGGGGGGGGGGGGGGGGG
GGGGGGGGGGGAGGACACAGCCATGTATTACTGTGTAAGAGGGATTTCTTACTGGGGCC

AAGGACTCTGGTCTGGCCAAGGACTCTGGTCTCTGCA

| region | Sequence fragment | Position in SEQ ID NO. 21 | Sequence Number SEQ ID NO. | Length |
|---|---|---|---|---|
| HFR1 | Amino acids 1-30 in SEQ ID NO. 21 | 1 - 30 | | 30 |
| CDR-H1 | NSAMS | 31 - 35 | 23 | 5 |
| HFR2 | Amino acids 36-49 in SEQ ID NO. 21 | 36 - 49 | | 14 |
| CDR-H2 | TITDGGDYTYYSDNVKG | 50 - 66 | 24 | 17 |
| HFR3 | Amino acids 67-98 in SEQ ID NO. 21 | 67 - 98 | | 32 |
| CDR-H3 | GISY | 99 - 102 | 25 | 4 |
| HFR4 | Amino acids 103-112 in SEQ ID NO. 21 | 103 - 112 | | 10 |

### MAb041-L

SEQ ID NO. 26 Amino Acid Sequence: (111aa) 
DIVLTQSPASLAVSLGQRATISCRASKSVSTSSDSYMHWYQQKPGQPPKLLIKYASNLESGV
PVRFSGSGSGTDFTLNIHPVEEEDAATYYCQHSREFPWTFGGGTKLEIK
SEQ ID NO. 27 > 3626-mAb041-17-45E5G11-L (333 bp)

GACATTGTGCTGACACAGTCTCCTGCTTCCTTAGCTGTATCTCTGGGCAGAGGGCCACA
GAGGGCCAGCAGAAAGTGTCAGTACATCTAGCACTGCACTGCCCAAACAGGACAGGACAG
GACAGGACAGGACAGCCGCCCAAACTGCCCAAACTGGGGGGGGGGGGGGGGGGGGGGGG
GGGGGGGGGGGGGGGGGGGGGGGGGGGGGGGGGGGGGGGGGGGGGGGGGGGGGGGGG
GGGGGGGGGGGGGGGGGGGGGGGGGGGGGGGGGGGGGGGGGGGGGGGGGGGGGGGGG
GGTATTGTCAGCACAGTAGGGAGTTTCCGTGGACGTTCGGGAGGCACCAAGCTGGAAAT
CAAA

| region | Sequence fragment | Position in SEQ ID NO. 26 | Sequence Number SEQ ID NO. | Length |
|---|---|---|---|---|
| LFR1 | Amino acids 1-23 in SEQ ID NO. 26 | 1 - 23 | | 23 |
| CDR-L 1 | RASKSVSTSSDSYMH | 24 - 38 | 28 | 15 |
| LFR2 | Amino acids 39-53 in SEQ ID NO. 26 | 39 - 53 | | 15 |
| CDR-L 2 | YASNLES | 54 - 60 | 29 | 7 |
| LFR3 | Amino acids 61-92 in SEQ ID NO. 26 | 61 - 92 | | 32 |
| CDR-L 3 | QHSREFPWT | 93 - 101 | 30 | 9 |
| LFR4 | Amino acids 102-111 in SEQ ID NO. 26 | 102 - 111 | | 10 |

### MAb058-H

SEQ ID NO. 31 Amino Acid Sequence: (116aa) 
EVQLQQSGPVLVKPGAAVKMSCKASGYIFTDYYIHWVKLSRGESLEWIGLINPYNGGTNY
NQQFTGKATLTVDKSSSTAYVELNSLTSGSSAVYYCVRRTGYFDYWGQGTTLTVSS
SEQ ID NO. 32 > 3626-mAb058-25-71F5C6-H (348 bp)

GAGGTCCAGCTGCAACAGTCTGGACCTGTAGGGAAGCCTGGGGCTGCAGTGAAGATGT
CCTGTAAGATTATTCACTGGGGTGATACATATACACACTGGGGGGTGGAAGAGCCTTGGG
GGGGGGGGGGGGGGGGAGAGGCCTTGGGGGGGGGGGGGGGGGGGGGGGGGGGGG
GGGGGGGGGGGGGGGGGGGGGGGGGGGGGGGGGGGGGGGGGGGGGGGGGGGGGGG
GGGGGGGGGGGGGGGGGGGGGGGGGGGGGGGGGGGGGGGGGGGGGGGGACTCTGCA
GTCTATTATTGTGTACGGAGGACTGGATACTTTGGACTGGACTACTGGGGCCAAGGCACCA
CTCTCTCTCCTCCA

| region | Sequence fragment | Position in SEQ ID NO. 31 | Sequence Number SEQ ID NO. | Length |
|---|---|---|---|---|
| HFR1 | Amino acids 1-30 in SEQ ID NO.31 | 1 - 30 | | 30 |
| CDR-H 1 | DYYIH | 31 - 35 | 33 | 5 |
| HFR2 | Amino acids 36-49 in SEQ ID NO.31 | 36 - 49 | | 14 |
| CDR-H 2 | LINPYNGGTNYNQQFTG | 50 - 66 | 34 | 17 |
| HFR3 | Amino acids 67-98 in SEQ ID NO.31 | 67 - 98 | | 32 |
| CDR-H 3 | RTGYFDY | 99 - 105 | 35 | 7 |
| HFR4 | Amino acids 106-116 in SEQ ID NO.31 | 106 - 116 | | 11 |

### MAb058-L

SEQ ID NO. 36 Amino Acid Sequence: (112aa) 
DVVMTQTPLSLPVSLGDQASISCRSSQSLVHSNGNTYLHWYLQKPGQSPKLLIYKVSNRFSG
VPDRFSGSGSGTDFTLKISRVEAEDLGVYFCSQTTHVPYTFGGGTKLEIK
SEQ ID NO. 37 > 3626-mAb058-25-71F5C6-L (336 bp)

GATGTTGGATGACCCAAACTCCACACACTCTCTCCTGCCTGTCAGTCTTGGAGATCAAG
CCTTGCAGATCTAGTCAGAGTCCTTGTAGACACAGTAGATGGTAGTGTAGATTGTAGTGTA
GTGTAGTGTAGTGTAGTGCCGACAGTGTGGCAGTGATGGACAGATTTCGGGGGGGGGG
GGGGGGGGGGGGGGGGGGGGGGGGGGGGGGGGGGGGGGGGGGGGGGGGGG
GGGGGGGGGGGGGGGGGGGGGGGGGGGGGGGGGGGGGGGGGGGGGGGGGG
GGGGTTCTGCTCTCAAACTACACATGTTCCGTACACGTTCGGAGGGACCAAGCTGGAAAT
AAAA

| region | Sequence fragment | Position in SEQ ID NO. 36 | Sequence Number SEQ ID NO. | Length |
|---|---|---|---|---|
| LFR1 | Amino acids 1-23 in SEQ ID NO. 36 | 1 - 23 | | 23 |
| CDR-L 1 | RSSQSLVHSNGNTYLH | 24 - 39 | 38 | 16 |
| LFR2 | Amino acids 40-54 in SEQ ID NO. 36 | 40 - 54 | | 15 |
| CDR-L 2 | KVSNRFS | 55 - 61 | 39 | 7 |
| LFR3 | Amino acids 62-93 in SEQ ID NO. 36 | 62 - 93 | | 32 |
| CDR-L 3 | SQTTHVPYT | 94 - 102 | 40 | 9 |
| LFR4 | Amino acids 103-112 in SEQ ID NO. 36 | 103 - 112 | | 10 |

### MAb034-H

SEQ ID NO. 41 Amino Acid Sequence (119aa) 
EVQLQQSGPVLVKPGASVKMSCKASGYTFTDFYMNWMRQSHGKSLEWIGVIDPYNGNTS
YNQKFKGKATLTVDKSSSTAYMELNSLTSEDSAVYYCARGNYYYYAMDYWGQGTSVTVSS
SEQ ID NO. 42 > 3626-mAb034-12-68E3A9-H (357 bp)

GAGGTCCAGCTGCAACAGTCTGGACCTGGCTTGGAAGCCTGGGCTTCAGTGAAGATG
TCCTGTAAGGCTTCTGGAAGATGCCTGTAAGTCTGGATGATGGAACTGGATGGAGTGGATG
GAGTGGAGTGATGGAGTGCCTTACAACGGAGTGATTGGGAGTGATTGGAGTGATTGCCTT
ACAACGGAGTGATTGCCTTAGAGTGATTGCCTTAGAGTGATTGCCTTAGAGTGATTGCCTT
AGAGTGATTGCCTTAGAGTGATTGCCTTAGAGGGGGGGGGGGGGGGGGGGGGGGGGGG

GGGGGGGGGGTCTGCAGTCTATTACTGTGCAAGAGGTAACTATTATTACTGGTAACTATTA
TTACTATGCTATGGACTACTGGGGGTCAAGGAACTTCAGTCACGGTCTCTCCTCA

| region | Sequence fragment | Position in SEQ ID NO. 41 | Sequence Number SEQ ID NO. | Length |
|---|---|---|---|---|
| HFR1 | Amino acids 1-30 in SEQ ID NO. 41 | 1 - 30 | | 30 |
| CDR-H 1 | DFYMN | 31 - 35 | 43 | 5 |
| HFR2 | Amino acids 36-49 in SEQ ID NO. 41 | 36 - 49 | | 14 |
| CDR-H 2 | VIDPYNGNTSYNQKFKG | 50 - 66 | 44 | 17 |
| HFR3 | Amino acids 67-98 in SEQ ID NO. 41 | 67 - 98 | | 32 |
| CDR-H 3 | GNYYYYAMDY | 99 - 108 | 45 | 10 |
| HFR4 | Amino acids 109-119 in SEQ ID NO. 41 | 109 - 119 | | 11 |

### MAb034-L

SEQ ID NO. 46 Amino Acid Sequence (111aa) 
DIVLTQSPASLAVSLGQRATISCRASESVSIHGTHLMYWYQQKPGQPPKLLIYAASNLESGV
PARFSGSGSETDFTLNIHPVEEEDAATFFCQQSIEDPPTFGGGTKLEIK
SEQ ID NO. 47 > 3626-mAb034-12-68E3A9-L (333 bp)

GACATTGGCTGACCCAATCTCCAGCTTCTTGGCTGTCTCTAGGGCAGAGGGCCAGAGG
GCCACTCATTCCTGCAGGAAAGTGTCAGTATTCATGGTAGTGTAGTATGTAGTGTAGTGTA
GTAGATGTAGTAGTAGTAGTAGTAGTAGTAGTAGATGATGGGAGTGGCATTGGCATTGGC
ATTGGCATTGGCATTGGCATTGGCATTGGGTGTGGTGTGGTGGTGGTGGGGGGGGGGGG
GGGGGGGGGGGGGGGGGGGGGGGGGGGGGGGGGGGGGGGGGGGGGGGGGGGGGGGG
GGGGGGGGGTTCTGTCAGCAAAGTATTGAGGATCCTCCCACGTTCGGAGGGGGACCAAG
CTGGAAATAAA

| region | Sequence fragment | Position in SEQ ID NO. 46 | Sequence Number SEQ ID NO. | Length |
|---|---|---|---|---|
| LFR1 | Amino acids 1-23 in SEQ ID NO. 46 | 1 - 23 | | 23 |
| CDR-L 1 | RASESVSIHGTHLMY | 24 - 38 | 48 | 15 |
| LFR2 | Amino acids 39-53 in SEQ ID NO. 46 | 39 - 53 | | 15 |
| CDR-L 2 | AASNLES | 54 - 60 | 49 | 7 |
| LFR3 | Amino acids 61-92 in SEQ ID NO. 46 | 61 - 92 | | 32 |
| CDR-L 3 | QQSIEDPPT | 93 - 101 | 50 | 9 |
| LFR4 | Amino acids 102-111 in SEQ ID NO. 46 | 102 - 111 | | 10 |

### MAb049-H

SEQ ID NO. 51 Amino Acid Sequence (117aa) 
EVQLQQSGPELVKPGASVKISCKASGHTLTDYYMNWVKQSHGKSPEWIGDINPNNDGIDY
NQKFRGKAILTVDKSSSSFYMELRSLTSEDSAVYYCVGFVRGYFVSWGQGTTLTVSS
SEQ ID NO. 52 > 3626-mAb049-20-80C8B1-H (351 bp)

GAGGTCCAGCTGCAACAATCTGGACCTGAGCTGGTGAAGCCTGGGCTTCAGTGAAGAT
TTCCTGTAAGGCTTCTGTAAGATTGGGGAAGAGCCATGGGGATTGGATTGGATTGGATTG
GAAGATTAGCCTAATAGATTGGATTGGATTGGATTGGATTGGATTGGATTGATTGCCATAT

TGACATTGACATTGACATTGGAACTGAGGGGGGGACATTGACATTGACATTGACATGGGG
GGGGGGGGGGGGGGGGGGGGGGGGGGGGGGGGGGGGGGGGGGGGGGGGGGGGGGGGGGGG
GGGGGGGGGGGGTCTGCAGTCTATTATTGTGGGGGTTCGTACGAGGCTACTTTTGTCTCC
TGGGCCAAGCACCACTCTCTCCTCA

| region | Sequence fragment | Position in SEQ ID NO.51 | Sequence Number SEQ ID NO. | Length |
|---|---|---|---|---|
| HFR1 | Amino acids 1-30 in SEQ ID NO.51 | 1 - 30 | | 30 |
| CDR-H1 | DYYMN | 31 - 35 | 53 | 5 |
| HFR2 | Amino acids 36-49 in SEQ ID NO.51 | 36 - 49 | | 14 |
| CDR-H2 | DINPNNDGIDYNQKFRG | 50 - 66 | 54 | 17 |
| HFR3 | Amino acids 67-98 in SEQ ID NO.51 | 67 - 98 | | 32 |
| CDR-H3 | FVRGYFVS | 99 - 106 | 55 | 8 |
| HFR4 | Amino acids 107-117 in SEQ ID NO.51 | 107 - 117 | | 11 |

### MAb049-L

SEQ ID NO. 56 Amino Acid Sequence (106aa) 
DIQMTQTTSSLSASLGDRVTINCRASQDISNYLNWYQQKPDGTVKLLIYYTSRLRSGVPSRF
SGSGSGTDYSLTINNLDQEDFATYFCQQGNTLPWTFGGGTKLEI
SEQ ID NO. 57 > 3626-mAb049-20-80C8B1-L (321 bp)

GATATCCAGATGACACAGACTACATCCCCCTGTCTGCCTCTCTGGGAGACAGAGTCACA
TTGCAGGCAAGTGCAAGTAATTGGTATTGTATCAGCAGAAACCAGATGGAACTGGTATT
GTAAACTGGGGGGGGGGGGGGGGGGGGGGGGGGGGGGGGGGGGGGGGGGGGGGGGG
GGGGGGGGGGGGGGGGGGGGGGGGGGGGGGGGGGGGGGGGGGGGGGGGGGGGGGGG
GGGGGGGGGGGGGGGGGGGGGGGGGGGGGGGGGGGGGGGGGGGGGGGGGGGGGGGT
AATACGCTTCCGTGGACGTTCGGTGGAGGCACCAAGCTGGAAATCAAA

| region | Sequence fragment | Position in SEQ ID NO. 56 | Sequence Number SEQ ID NO. | Length |
|---|---|---|---|---|
| LFR1 | Amino acids 1-23 in SEQ ID NO. 56 | 1 - 23 | | 23 |
| CDR-L1 | RASQDISNYLN | 24 - 34 | 58 | 11 |
| LFR2 | Amino acids 35-49 in SEQ ID NO. 56 | 35 - 49 | | 15 |
| CDR-L2 | YTSRLRS | 50 - 56 | 59 | 7 |
| LFR3 | Amino acids 57-88 in SEQ ID NO. 56 | 57 - 88 | | 32 |
| CDR-L3 | QQGNTLPWT | 89 - 97 | 60 | 9 |
| LFR4 | Amino acids 98-106 in SEQ ID NO. 56 | 98 - 106 | | 9 |

### MAb064-H

SEQ ID NO. 61 Amino Acid Sequence (116aa) 
QVQLKESGPGLVAPSQTLSITCSVSGFSLTNYAVSWVRQPPGKGLEWLGVMWGDGNTNYH
STLISRLSISKDNSKSQVFLKLNSLQTDDTATYYCARSSGWYFDVWGTGTTVTVSS
SEQ ID NO. 62 > 3626-mAb064-27-80E5G2-H (348 bp)

CAGGTGCAGCTGAAGGAGTCAGGACCTGCCTGGCCTGGCCCTCACAGACCCCTGTT
CTCAGGTTCTCTCTAACCAACTATGCTGTAAAGGGGGGTTCGCCAGGTGTTCGCCAGGAAG
GTGTGGGGGGGGGGGGGGGGGGGGGGGGGGGGGGGGGGGGGGGGGGGGGGGGGGGGGG
GGGGGGGGGGGGGGGGGGGGGGGGGGGGGGGGGGGGGGGGGGGGGGGGGGGGGGGGG
GGGGGGGGGGGGGGGGGGGGGGGGGGGGGGGGGGGGGGGGGGGGGGGGCACAGCCACG
TACCTGTGCCCGTAGTAGCGCTGGTAGTAGCGCTGGTACTTCGATGTCTGGGGCACAGGAC
CACGGGCACCCGTCTCCTCTCCTCA

| region | Sequence fragment | Position in SEQ ID NO. 61 | Sequence Number SEQ ID NO. | Length |
|---|---|---|---|---|
| HFR1 | Amino acids 1-30 in SEQ ID NO. 61 | 1 - 30 | | 30 |
| CDR-H1 | NYAVS | 31 - 35 | 63 | 5 |
| HFR2 | Amino acids 36-49 in SEQ ID NO. 61 | 36 - 49 | | 14 |
| CDR-H2 | VMWGDGNTNYHSTLIS | 50 - 65 | 64 | 16 |
| HFR3 | Amino acids 66-97 in SEQ ID NO. 61 | 66 - 97 | | 32 |
| CDR-H3 | SSGWYFDV | 98 - 105 | 65 | 8 |
| HFR4 | Amino acids 106-116 in SEQ ID NO. 61 | 106 - 116 | | 11 |

### MAb064-L

SEQ ID NO. 66 Amino Acid Sequence (107aa) 
DIQMTQTTPSLSVTLGDRVTIRCRASQDINNYLNWYQQKPDGTVKLLIYYTSESRSGVPSRF
SGSGSGTDYSLTISNLEQEDIATYFCQQGYALPPAFGGGTKLEIK
SEQ ID NO. 67 > 3626-mAb064-27-80E5G2-L (321 bp)

GATATCCAGATGACACACAGACTACACCTCCCTTGACTCTGGACTCTGGAGACAGAGT
CACTCTGGGCAAGTGCAAGTGCAAGTGGACATTAACAATTATTTAAATTGGTATC
AGAGAAGATTGGTATCAGAAGATGGAACTGGAACTGGAACTGGGGGGGGGGGGGG
GGGGGGGGGGGGGGGGGGGGGGGGGGGGGGGGGGGGGGGGGGGGGGGGGGGG
GGGGGGGGGGGGGGGGGGGGGGGGGGGGGGGGGGGGGGGGGGGGGGGGGGGG
GGGGGGGTTATGCGCTTCCTCCGGCGTTCGGTGGAGGCACCAAGCTGGAAATAAAA

| Region | Sequence fragment | Position in SEQ ID NO.66 | Sequence Number SEQ ID NO. | Length |
|---|---|---|---|---|
| LFR1 | Amino acids 1-23 in SEQ ID NO. 66 | 1 - 23 | | 23 |
| CDR-L1 | RASQDINNYLN | 24 - 34 | 68 | 11 |
| LFR2 | Amino acids 35-49 in SEQ ID NO. 66 | 35 - 49 | | 15 |
| CDR-L2 | YTSESRS | 50 - 56 | 69 | 7 |
| LFR3 | Amino acids 57-88 in SEQ ID NO. 66 | 57 - 88 | | 32 |
| CDR-L3 | QQGYALPPA | 89 - 97 | 70 | 9 |
| LFR4 | Amino acids 98-107 in SEQ ID NO. 66 | 98 - 107 | | 10 |

### MAb033-H

SEQ ID NO. 71 Amino Acid Sequence: (118aa) 
QVQLQQSGAELVRPGTAVRVSCKASGYAFTNYFIEWVKQRPGQGLEWIGVINPGSGDTYY
SEKFQGKATLTADKSSSTAYMQLSSLTSEDSAVYFCAREDDYSWFTYWGQGTLVTVSA
SEQ ID NO. 72 > 3626-mAb033-11-67G9A3-H (354 bp)

CAGGTCCAGCTGCAGCAGTCTGGAGCTGAGTTGTAAGGCCTGGACTGCAGTGGATGGA
TACGCCTTCACTAATTATATATATATATATATATATATAGTGGATTGGATTGGATTAGT
GATTAGTGATTAGATTAGTCCTGGATTGATTAGATTAGTCCTGCAAGGACTGACTGACTGA
CTGCCTAGGACAGTCCTAGGACTGACTGCCTAGCACTGACTGCCTAGCACTGCCTAGCACT
GCCTAGCACTGCCTAGCACTGCCTAGCACTGCCTAGCACTGCCTAGCACTGCCTAGCACTG
CCTACATGCCTAGTGACTGACTGACTGACTGACTGACTGACTGACTGACTGACTGAGGACT
CTGCGGTCTATTTCTGTGCAAGAGAGGATGATTACTCCTGGTTTACTTACTGGGCCAAGGA
CTCTGGTCTCTGTCTCTGCA

| Region | Sequence fragment | Position in SEQ ID NO.111 | Sequence Number SEQ ID NO. | Length |
|---|---|---|---|---|
| HFR1 | Amino acids 1-30 in SEQ ID NO.111 | 1 - 30 | | 30 |
| CDR-H 1 | NYFIE | 31 - 35 | 73 | 5 |
| HFR2 | Amino acids 36 - 49 in SEQ ID NO.111 | 36 - 49 | | 14 |
| CDR-H 2 | VINPGSGDTYYSEKFQG | 50 - 66 | 74 | 17 |
| HFR3 | Amino acids 67 - 98 in SEQ ID NO.111 | 67 - 98 | | 32 |
| CDR-H 3 | EDDYSWFTY | 99 - 107 | 75 | 9 |
| HFR4 | Amino acids 108 - 118 in SEQ ID NO.111 | 108 - 118 | | 11 |

### MAb033-L

SEQ ID NO. 76 Amino Acid Sequence: (111aa) 
DIVLTQSPASLAVSLGQRATISCRASENVDNYGISFMHWYRQKPGQPPKLLIYRASNLESGIP
ARFSGSGSRTDFTLTINPVETDDVATYYCQQSNKDPYTFGGGTKLEIK
SEQ ID NO. 77 > 3626-mAb033-11-67G9A3-L (333 bp)

GACATTGGCTGACCCAATCTCCAGCTTCTTTGGCTGTGTCTCTAGGGCAGAGGCCAGAG
GGCAGAGATTGGCATTAGTTTATGGCATTAGTTTATGGCATTAGTTTATGGCATTAGTTTAT
GGCACTGGTAGGCAGAAGTTATGCACTGGGATCCGGGGGGGGGGGGGGGGGGGGGGGG
GGGGGGGGGGGGGGGGGGGGGGGGGGGGGGGGGGGGGGGGGGGGGGGGGGGGGGGG
GGGGGGGGGGGGGGGGGGGGGGGGGGGGGGGGGGGGGGGGGGGGGGGGGGGGGGCTA
TTACTGTCAGCAAAGTAATAAGGATCCGTACACGTTCGGAGGGGACCAAACTGGAAATAA
AA

| Region | Sequence fragment | Position in SEQ ID NO. 116 | Sequence Number SEQ ID NO. | Length |
|---|---|---|---|---|
| LFR1 | Amino acids 1-23 in SEQ ID NO. 116 | 1 - 23 | | 23 |
| CDR-L 1 | RASENVDNYGISFMH | 24 - 38 | 78 | 15 |
| LFR2 | Amino acids 39 - 53 in SEQ ID NO. 116 | 39 - 53 | | 15 |
| CDR-L 2 | RASNLES | 54 - 60 | 79 | 7 |
| LFR3 | Amino acids 61 - 92 in SEQ ID NO. 116 | 61 - 92 | | 32 |
| CDR-L 3 | QQSNKDPYT | 93 - 101 | 80 | 9 |
| LFR4 | Amino acids 102-111 in SEQ ID NO. 116 | 102 - 111 | | 10 |

### MAb047-H

SEQ ID NO. 81 Amino Acid Sequence: (117aa) 
EVQLVESGGGLVKPGGSLKLSCAASGFTFSDYGMYWIRQAPEKGLEWVAYISNGGSSIYYA
DTVKGRFTVSRDNAKNTLFLQMTSLRSEDTAIYYCARGGELGFAYWGQGTLVTVSA
SEQ ID NO. 82 > 3626-mAb047-19-76C9F6-H (351 bp)

GAGGTGCAGCTGGTGGAGTCTGGGAGGTAGTGAAGCCTGGAGGCCTGGAGGGCCTGGA
GGGCCTGGAGACTCTCCTGGATTCTTGATTCTGATTCTGATTCTGATTGACTGGATTGGATG
GACTGGATTGGATTGGATGGATGGGGGGATGGATGGGGGGGGGGGGGGGGGGGGGGGG
GGGGGGGGGGGGGGGGGGGGGGGGGGGGGGGGGGGGGGGGGGGGGGGGGGGGGGGG
GGGGGGGGGGGGGGGGGGGGGGGGGGGGGGGGGGGGGGGGGGGGGGGGGCTGAGGACA
CGGCCATATTACTGTGCAAGGGGAGAGAACTCGGGTTTGCTTACTGGGCCAAGGACTCTGG
TCTCTGGTCTCTGCA

| Region | Sequence fragment | Position in SEQ ID NO. 121 | Sequence Number SEQ ID NO. | Length |
|---|---|---|---|---|
| HFR1 | Amino acids 1-30 in SEQ ID NO. 121 | 1 - 30 | | 30 |
| CDR-H | DYGMY | 31 - 35 | 83 | 5 |
| 1 | | | | |
| HFR2 | Amino acids 36-49 in SEQ ID NO. 121 | 36 - 49 | | 14 |
| CDR-H 2 | YISNGGSSIYYADTVKG | 50 - 66 | 84 | 17 |
| HFR3 | Amino acids 67 - 98 in SEQ ID NO. 121 | 67 - 98 | | 32 |
| CDR-H 3 | GGELGFAY | 99 - 106 | 85 | 8 |
| HFR4 | Amino acids 107 - 117 in SEQ ID NO. 121 | 107 - 117 | | 11 |

### MAb047-L

SEQ ID NO. 86 Amino Acid Sequence: (105aa) 
DIQMTQSPASLSVSVGETVTITCRASENIYSNLAWYQQKQGKSPQLLVYAAINLVEGVPSRF
SGSGSGTQYSLKINSLQSEDFGSYFCQHFWGLPYTFGGGTKLE
SEQ ID NO. 87 > 3626-mAb047-19-76C9F6-L (321 bp)

GACATTCAGATGACTCAGTCTCCAGCCCCCTATCTGTATCTGTATCTGGAGAAACTGT
CACCATGTGTCGAGAAAACTGTCAAAGTGTGGAAAGTGGTATCAGTAGAAAATTGGGTAT
CAGTAGATGGGAAAATCTCCTCAGTGGCATCAGATGGTGTGGCATCAGATGGATGTGGCA
TCAGATGGATGTGGCATCAGATGGGGGGGGGGGGGGGGGGGGGGGGGGGGGGGGGGGG
GGGGGGGGGGGGGGGGGGGGGGGGGGGGGGGGGGGGGGGGGGGGGGGGGGGGGGGGGG
GGGGGGGGGGGACATTTTGGGTCTTCCGTACACGTTCGGAGGGGACCAAGCTGGAAAT

| Region | Sequence fragment | Position in SEQ ID NO. 126 | Sequence Number SEQ ID NO. | Length |
|---|---|---|---|---|
| LFR1 | Amino acids 1-23 in SEQ ID NO. 126 | 1 - 23 | | 23 |
| CDR-L 1 | RASENIYSNLA | 24 - 34 | 88 | 11 |
| LFR2 | Amino acids 35 - 49 in SEQ ID NO. 126 | 35 - 49 | | 15 |
| CDR-L 2 | AAINLVE | 50 - 56 | 89 | 7 |
| LFR3 | Amino acids 57 - 88 in SEQ ID NO. 126 | 57 - 88 | | 32 |
| CDR-L 3 | QHFWGLPYT | 89 - 97 | 90 | 9 |
| LFR4 | Amino acids 98 - 105 in SEQ ID NO. 126 | 98 - 105 | | 8 |

### MAb055-H

SEQ ID NO. 91 Amino Acid Sequence: (112aa) 
QVQLKQSGPGLLQPSQSLSITCTVSGFSLDEYGVHWFRQSPGKGLEWLGVIWSGGSTDYNA
AFISRLSIRKDNSKSQIFFNMNSLQADDTAIYYCVREEWEYWGQGTTLTVS
SEQ ID NO. 92 > 3626-mAb055-24-83D3H2-H (339 bp)

CAGGTGCAGCTGAAGCAGTCAGGACCTGCCTACTGCAGCCTACTGCACAGAGCCTGTC
CTTCACGTCTCTGCACAGTCTCTGTTAGATGAGTATGGTTTCTGTAGATGAGTGTGTGTG
TGTGTGTGTGGAGTGGAGTGGAGTGGAGTGGAGTGGAGTGGAGTGGAGTGGAGTGGAGTG
GAGTGGAGTGGAGTGGGAGTGGAGTGGAGTGGAGTGCACAGACAGGACAATTCCAAGAG
ACAATTCCAAATTTCTTTAATATGACAGATGATGATGATGATGATGATGATGATGATGATG
ATGATGATGATGATCACAGCCATATTACTGTGTCAGAGAGGAATGGGAATATTGGGGCCA
AGGCACACTCTCCACAGTCTCCTCA

| Region | Sequence fragment | Position in SEQ ID NO. 141 | Sequence Number SEQ ID NO. | Length |
|---|---|---|---|---|
| HFR1 | Amino acids 1-30 in SEQ ID NO. 141 | 1 - 30 | | 30 |
| CDR-H1 | EYGVH | 31 - 35 | 93 | 5 |
| HFR2 | Amino acids 36 - 49 in SEQ ID NO. 141 | 36 - 49 | | 14 |
| CDR-H2 | VIWSGGSTDYNAAFIS | 50 - 65 | 94 | 16 |
| HFR3 | Amino acids 66 - 97 in SEQ ID NO. 141 | 66 - 97 | | 32 |
| CDR-H3 | EEWEY | 98 - 102 | 95 | 5 |
| HFR4 | Amino acids 103-112 in SEQ ID NO. 141 | 103 - 112 | | 10 |

### MAb055-L

SEQ ID NO. 96 Amino Acid Sequence: (107aa) 
DIQMTQTTSSLSASLGDRVTISCRPSQDIDNYLNWFQQRPDGTIKLLIYYTSRLHSGVPSRFS
GSGSGTDYSLTINNLEQEDIATYFCQQAHTLPYTFGGGTKLEIK
SEQ ID NO. 97 DNA sequence:

GATATCCAGATGACACAGACTACATCCCCCTGTCTGCCTCTCTGGAGACAGAGTCACA
GTTGCAGCCAAGTTGCAGGACAGTATTTAAACTTGGTTTCAGCAGAGAGAAACTGGTTGTT
TCAGCAGAGAGACAGATTCCTGGCATGATGATGATGGGTGTGGCATGATGGGTGTGTGTGT
GTGTGTGTGTGTGATGTGTGTGATGTGTGTGATGTGTGTGATGTGTGTGGCACTGATTGTGT
GATGTGTGATGTGTGATGTGTGATGTGTGATGTGTGTGATGTGTGTGTGTGTGTGTGTGT
GTGCTCATACGCTTCCGTACACGTTCGGAGGGGGACCAACTGGAAATAAAA

| Region | Sequence fragment | Position in SEQ ID NO.146 | Sequence Number SEQ ID NO. | Length |
|---|---|---|---|---|
| LFR1 | Amino acids 1-23 in SEQ ID NO. 146 | 1 - 23 | | 23 |
| CDR-L 1 | RPSQDIDNYLN | 24 - 34 | 98 | 11 |
| LFR2 | Amino acids 35 - 49 in SEQ ID NO. 146 | 35 - 49 | | 15 |
| CDR-L 2 | YTSRLHS | 50 - 56 | 99 | 7 |
| LFR3 | Amino acids 57 - 88 in SEQ ID NO. 146 | 57 - 88 | | 32 |
| CDR-L 3 | QQAHTLPYT | 89 - 97 | 100 | 9 |
| LFR4 | Amino acids 98-107 in SEQ ID NO. 146 | 98 - 107 | | 10 |

### Humab035-24-VH

SEQ ID NO. 101 Amino Acid Sequence (116aa) 
QVQLVQSGAEVKKPGASVKVSCKASDYTFTDYEMHWVRQAPGQGLEWIGAIDPETG
GTAYNQKFKDRVTMTADTSTSTVYMELSSLRSEDTAVYYCAKTGYQFDYWGQGTTVTVS
S
SEQ ID NO. 102 DNA sequence (348 bp)

CAGGTGCAGCTGGTGCAGTCAGGAGAGAGAGAAGCCAGGCGCAAGCGGAAGGTC
TTGCAAGGCCAGCGACTAGAGATGCATTGCCGGGACTAGAGATGCATTGGGGATTGGG
ATTGGGATTGCACGGGATTGGGATTGACCGAGGAGAGAGAGAGAGAGAGAGAGAGAG
AGAGAGAGAGAGAGAGAGAGAGAGAGAGAGAGAGAGAGAGAGAGAGAGAGAGAGAGA
GAGAGAGAGAGAGAGAGAGAGAGAGAGAGAGAGAGAGAGAGAGAGAGAGAGAGAGAG
AGCGAGGACACCGCCGTGTACTATTGCGCCAAGACCGGCTACCAGGGCTACCAGTTCG
ACTATTGGGGGCCAGGAACCAGTGACAGTGTCCAGC

### Humab035-24-VL

SEQ ID NO. 103 Amino Acid Sequence (107aa) 
DIQMTQSPSSVSASVGDRVTITCKASQNVGSNVAWYQQKPGKAPKALIYSASYRYSG
VPSRFSGSGSGTDFTLTISSLQPEDFATYYCQQYNSHPYTFGQGTKLEIK
SEQ ID NO. 104 DNA sequence (321 bp)

GACATTCACAGATGACCCAGACCCTAGACCCTAGCAGCGTGCCGCTAGCGGGAG
AGTGACCGCGCGGGAGAGTGCCAGCCAGGGCTTGGGCAGCAGGGCTTGGTATCAGCA
GAGGCCTAGGGAGGCCTAGGGAGGCCTAGGGGGGGGGGGGGGGGGGGGGGGGGG
GGGGGGGGGGGGGGGGGGGGGGGGGGGGGGGGGGGGGGGGGGGGGGGGGG
GGGGGGGGGGGGGGGGGGGGGGGGGGGGGGGGGGGGGGGGGGGGGGGGGG
GGGGGGTACAACAGCCACCCCCTACACTTCGGACAGGGCACAGCTGGAGATCAAG

## Claims

1. A heavy chain variable region of an antibody having a complementarity determining region CDR selected from the group consisting of:
a VH-CDR1 as shown in SEQ ID NO.10n+3,
a VH-CDR2 as shown in SEQ ID NO.10n+4, and
a VH-CDR3 as shown in SEQ ID NO.10n+5;
wherein each n is independently 0, 1, 2, 3, 4, 5, 6, 7, 8 or 9;
wherein any one of the above amino acid sequences further includes a derivative sequence that is optionally added, deleted, modified, and/or substituted for at least one amino acid and can retain the binding affinity of OX40.

2. A heavy chain of an antibody having the heavy chain variable region of claim 1.

3. A light chain variable region of an antibody having a complementarity determining region CDR selected from the group consisting of:
a VL-CDR1 as shown in SEQ ID NO.10n+8,
a VL-CDR2 as shown in SEQ ID NO.10n+9, and
a VL-CDR3 as shown in SEQ ID NO.10n+10;
wherein each n is independently 0, 1, 2, 3, 4, 5, 6, 7, 8 or 9;
wherein any one of the above amino acid sequences further includes a derivative sequence that is optionally added, deleted, modified, and/or substituted for at least one amino acid and can retain the binding affinity of OX40.

4. A light chain of an antibody having the light chain variable region of claim 3.

5. An antibody having:
(1) a heavy chain variable region of claim 1; and/or
(2) a light chain variable region of claim 3;
or the antibody has the heavy chain of claim 2; and/or the light chain of claim 4,
wherein any one of the above amino acid sequences further includes a derivative sequence that is optionally added, deleted, modified, and/or substituted for at least one amino acid and can retain the binding affinity of OX40.

6. The antibody of claim 5, wherein the antibody has a heavy chain variable region of claim 1 and a light chain variable region of claim 3; wherein the heavy chain variable region and the light chain variable region comprise a CDR selected from the group consisting of:
| VH-CDR1 Sequence Number | VH-CDR2 Sequence Number | VH-CDR3 Sequence Number | VL-CDR1 Sequence Number | VL-CDR2 Sequence Number | VL-CDR3 Sequence Number |
|---|---|---|---|---|---|
| 3 | 4 | 5 | 8 | 9 | 10 |
| 13 | 14 | 15 | 18 | 19 | 20 |
| 23 | 24 | 25 | 28 | 29 | 30 |
| 33 | 34 | 35 | 38 | 39 | 40 |
| 43 | 44 | 45 | 48 | 49 | 50 |
| 53 | 54 | 55 | 58 | 59 | 60 |
| 63 | 64 | 65 | 68 | 69 | 70 |
| 73 | 74 | 75 | 78 | 79 | 80 |
| 83 | 84 | 85 | 88 | 89 | 90 |
| 93 | 94 | 95 | 98 | 99 | 100 |
wherein any one of the above amino acid sequences also includes a derivative sequence that is optionally added, deleted, modified, and/or substituted for at least one amino acid and can retain the binding affinity of OX40.

7. The antibody of claim 5, wherein the heavy chain variable region of the antibody contains the amino acid sequence as shown in SEQ ID NO. 101, 1, 11, or 21; and/or the light chain variable region of the antibody contains the amino acid sequence as shown in SEQ ID NO. 103, 6, 16 or 26.

8. The antibody of claim 6, wherein the antibody is selected from the group consisting of:
| Antibody number | Clone | VH sequence Number | VL Sequence Number |
|---|---|---|---|
| 1 | Humab035-24 | 101 | 103 |
| 2 | Mab035 | 1 | 6 |
| 3 | Mab053 | 11 | 16 |
| 4 | Mab041 | 21 | 26 |
| 5 | Mab058 | 31 | 36 |
| 6 | Mab034 | 41 | 46 |
| 7 | Mab049 | 51 | 56 |
| 8 | Mab064 | 61 | 66 |
| 9 | Mab033 | 71 | 76 |
| 10 | Mab047 | 81 | 86 |
| 11 | Mab055 | 91 | 96 |

9. A recombinant protein comprising:
(i) a heavy chain variable region of claim 1, a heavy chain of claim 2, a light chain variable region of claim 3, a light chain of claim 4, or an antibody of any one of claims 5-8; and
(ii) an optional tag sequence to assist in expression and/or purification.

10. A polynucleotide encoding a polypeptide selected from the group consisting of:
(1) a heavy chain variable region of claim 1, a heavy chain of claim 2, a light chain variable region of claim 3, a light chain of claim 4, or an antibody of any one of claims 5-8; and
(2) the recombinant protein of claim 9.

11. The polynucleotide of claim 10, wherein the polynucleotide encoding the variable region of the heavy chain is shown in SEQ ID NO. 102, 2, 12, 22, 32, 42, 52, 62, 72, 82, or 92; and/or, the polynucleotide encoding the variable region of the light chain is shown in SEQ ID NO. 104, 7, 17, 27, 37, 47, 57, 67, 77, 87 or 97.

12. The polynucleotide of claim 11, wherein the polynucleotide encoding the heavy chain variable region sequence and the polynucleotide encoding the light chain variable region sequence are selected from the group consisting of:
| Clone | Sequence numbering of polynucleotide encoding VH | Sequence numbering of polynucleotide encoding VL |
|---|---|---|
| Humab035-24 | 102 | 104 |
| Mab035 | 2 | 7 |
| Mab053 | 12 | 17 |
| Mab041 | 22 | 27 |
| Mab058 | 32 | 37 |
| Mab034 | 42 | 47 |
| Mab049 | 52 | 57 |
| Mab064 | 62 | 67 |
| Mab033 | 72 | 77 |
| Mab047 | 82 | 87 |
| Mab055 | 92 | 97 |

13. A vector comprising the polynucleotide of any one of claims 10-12.

14. A genetically engineered host cell comprising the vector of claim 13 or the genome integrated with the polynucleotide of any one of claims 10-12.

15. An antibody conjugate comprising:
(a) an antibody portion, which is selected from the group consisting of a heavy chain variable region of claim 1, a heavy chain of claim 2, a light chain variable region of claim 3, a light chain of claim 4, or an antibody of any one of claims 5-8, and a combination thereof; and
(b) a coupling portion coupled to the antibody portion, the coupling portion is selected from the group consisting of a detectable marker, drug, toxin, cytokine, radionuclide, enzyme, and a combination thereof.

16. An immune cell that expresses or is exposed outside the cell membrane with the antibody of any one of claims 5-8.

17. A pharmaceutical composition comprising:
(i) an active ingredient, the active ingredient is selected from the group consisting of: the heavy chain variable region of claim 1, the heavy chain of claim 2, the light chain variable region of claim 3, the light chain of claim 4, or the antibody of any one of claims 5-8, the recombinant protein of claim 9, the antibody conjugate of claim 15, the immune cell of claim 16, and a combination thereof; and
(ii) a pharmaceutically acceptable carrier.

18. Use of an active ingredient selected from the group consisting of the heavy chain variable region of claim 1, and the heavy chain of claim 2, the light chain variable region of claim 3, the light chain of claim 4, or the antibody of any one of claims 5-8, the recombinant protein of claim 9, the antibody conjugate of claim 15, the immune cell of claim 16, and a combination thereof for (a) preparing a diagnostic reagent or kit; and/or (b) preparing a drug for preventing and/or treating diseases related to abnormal expression or function of OX40.


sequence listing

<110>  SHANGHAI PHARMAEXPLORER CO., LTD.
       PHARMAEXPLORER LIMITED

<120>  OX40 ANTIBODY, PREPARATION METHOD THEREOF AND USE THEREOF

<130>  XU11915PCEP

<140>  PCT/CN2019/119515
<141>  2019-11-19

<150>  201811386274.3
<151>  2018-11-20

<160>  104

<170>  PatentIn version 3.5

<210>  1
<211>  116
<212>  PRT
<213>  Artificial sequence

<220>
<223>  mAb035 VH

<400>  1

Gln Val Gln Leu Gln Gln Ser Gly Ala Glu Leu Val Arg Pro Gly Ala
1               5                   10                  15

Ser Val Thr Leu Ser Cys Lys Ala Ser Asp Tyr Thr Phe Thr Asp Tyr
            20                  25                  30

Glu Met His Trp Val Tyr Gln Thr Pro Val His Gly Leu Glu Trp Ile
        35                  40                  45

Gly Ala Ile Asp Pro Glu Thr Gly Gly Thr Ala Tyr Asn Gln Lys Phe
    50                  55                  60

Lys Asp Lys Ala Ile Leu Thr Ala Asp Lys Ser Ser Asn Thr Ala Tyr
65                  70                  75                  80

Met Glu Leu Arg Ser Leu Thr Ser Glu Asp Ser Ala Val Tyr Phe Cys
                85                  90                  95

Thr Lys Thr Gly Tyr Gln Phe Asp Tyr Trp Gly Gln Gly Thr Thr Leu
            100                 105                 110

Thr Val Ser Ser
        115

<210>  2
<211>  348

<212>  DNA
<213>  Artificial sequence

<220>
<223>  mAb035 VH

<400>  2
caggttcaac tgcagcagtc tggggctgag ctggtgaggc ctggggcttc agtgacgctg      60

tcctgcaagg cttcggacta cacatttact gactatgaaa tgcactgggt gtaccagaca     120

cctgtacatg gcctggaatg gattggagct attgatcctg aaactggtgg tactgcctac     180

aatcagaagt tcaaggacaa ggccatactg actgcagaca aatcctccaa cacagcctac     240

atggagctcc gcagcctgac atctgaggac tctgccgtct atttctgtac aaaaactggg     300

taccagtttg actactgggg ccaaggcacc actctcacag tctcctca                  348


<210>  3
<211>  5
<212>  PRT
<213>  Artificial sequence

<220>
<223>  mAb035 VH-CDR1

<400>  3

Asp Tyr Glu Met His
1               5


<210>  4
<211>  17
<212>  PRT
<213>  Artificial sequence

<220>
<223>  mAb035 VH-CDR2

<400>  4

Ala Ile Asp Pro Glu Thr Gly Gly Thr Ala Tyr Asn Gln Lys Phe Lys
1               5                   10                  15


Asp



<210>  5
<211>  7
<212>  PRT
<213>  Artificial sequence

<220>
<223>  mAb035 VH-CDR3

<400>  5

Thr Gly Tyr Gln Phe Asp Tyr

1               5

<210>  6
<211>  107
<212>  PRT
<213>  Artificial sequence

<220>
<223>  mAb035 VL

<400>  6

Asp Ile Val Met Thr Gln Ser Gln Lys Phe Met Ser Thr Ser Val Gly
1               5                   10                  15

Asp Arg Val Ser Val Thr Cys Lys Ala Ser Gln Asn Val Gly Ser Asn
            20                  25                  30

Val Ala Trp Tyr Gln Gln Lys Pro Gly Gln Ser Pro Lys Ala Leu Ile
        35                  40                  45

Tyr Ser Ala Ser Tyr Arg Tyr Ser Gly Val Pro Asp Arg Phe Ala Gly
    50                  55                  60

Ser Gly Ser Gly Thr Asp Phe Thr Leu Thr Ile Ser Asn Val Gln Ser
65                  70                  75                  80

Glu Asp Leu Ala Asp Tyr Phe Cys Gln Gln Tyr Asn Ser His Pro Tyr
                85                  90                  95

Thr Phe Gly Gly Gly Thr Lys Leu Glu Ile Lys
            100                 105

<210>  7
<211>  321
<212>  DNA
<213>  Artificial sequence

<220>
<223>  mAb035 VL

<400>  7
gacattgtga tgacccagtc tcaaaaattc atgtccacat cagtaggaga cagggtcagc      60

gtcacctgca aggccagtca gaatgtgggt agtaatgtag cctggtatca acagaaacca     120

ggacaatctc ctaaagcact gatttattcg gcatcctacc ggtacagtgg agtccctgat     180

cgcttcgcag gcagtggatc tgggacagat ttcactctca ccatcagcaa tgtgcagtct     240

gaagacctgg cagattattt ctgtcagcaa tacaacagcc atccgtacac gttcggaggg     300

gggaccaagc tggaaataaa a                                               321

<210>  8
<211>  11
<212>  PRT
<213>  Artificial sequence

<220>
<223>  mAb035 VL-CDR1

<400>  8

Lys Ala Ser Gln Asn Val Gly Ser Asn Val Ala
1               5                   10


<210>  9
<211>  7
<212>  PRT
<213>  Artificial sequence

<220>
<223>  mAb035 VL-CDR2

<400>  9

Ser Ala Ser Tyr Arg Tyr Ser
1               5


<210>  10
<211>  9
<212>  PRT
<213>  Artificial sequence

<220>
<223>  mAb035 VL-CDR3

<400>  10

Gln Gln Tyr Asn Ser His Pro Tyr Thr
1               5


<210>  11
<211>  112
<212>  PRT
<213>  Artificial sequence

<220>
<223>  mAb053 VH

<400>  11

Gln Val Gln Leu Lys Gln Ser Gly Pro Gly Leu Val Gln Pro Ser Gln
1               5                   10                  15


Ser Leu Ser Ile Ile Cys Thr Val Ser Gly Phe Ser Leu Ser Asp Tyr
            20                  25                  30


Gly Val Met Trp Val Arg Gln Ser Pro Gly Lys Gly Leu Glu Trp Leu
        35                  40                  45

Gly Val Ile Trp Ser Gly Gly Ser Thr Asp Tyr Asn Ala Ala Phe Thr
    50                  55                  60

Ser Arg Leu Ser Ile Ser Arg Asp Asn Ser Lys Arg Gln Val Ile Phe
65                  70                  75                  80

Lys Met Asn Ser Leu Gln Ala Glu Asp Thr Ala Ile His Tyr Cys Val
                85                  90                  95

Arg Glu Thr Met Asp Tyr Trp Gly Gln Gly Thr Ser Val Thr Val Ser
            100                 105                 110

<210>  12
<211>  339
<212>  DNA
<213>  Artificial sequence

<220>
<223>  mAb053 VH

<400>  12
caggtgcagc tgaagcagtc aggacctggc ctagtgcagc cctcacagag cctgtccatc      60

atatgcacag tatctggttt ctcattaagt gactatggtg taatgtgggt tcgccagtct     120

ccaggaaagg gtctggagtg gctgggagtg atatggagtg gtggaagtac agactataat     180

gcagctttca catccagact gagcatcagc agggacaatt ccaagagaca agtcatcttt     240

aaaatgaaca gtctacaagc tgaggacaca gccatacatt actgtgtcag agaaacgatg     300

gactactggg gtcaaggaac ctcagtcacc gtctcctca                            339

<210>  13
<211>  5
<212>  PRT
<213>  Artificial sequence

<220>
<223>  mAb053 VH-CDR1

<400>  13

Asp Tyr Gly Val Met
1               5

<210>  14
<211>  16
<212>  PRT
<213>  Artificial sequence

<220>
<223>  mAb053 VH-CDR2

<400>  14

Val Ile Trp Ser Gly Gly Ser Thr Asp Tyr Asn Ala Ala Phe Thr Ser
1               5                   10                  15

<210>  15
<211>  5
<212>  PRT
<213>  Artificial sequence

<220>
<223>  mAb053 VH-CDR3

<400>  15

Glu Thr Met Asp Tyr
1               5

<210>  16
<211>  107
<212>  PRT
<213>  Artificial sequence

<220>
<223>  mAb053 VL

<400>  16

Asp Ile Gln Met Thr Gln Thr Thr Ser Ser Leu Ser Ala Ser Leu Gly
1               5                   10                  15

Asp Arg Val Thr Ile Ser Cys Arg Ala Ser Gln Asp Ile Ser Asn Tyr
            20                  25                  30

Leu Asn Trp Tyr Gln Gln Arg Pro Asp Gly Thr Val Lys Arg Leu Ile
        35                  40                  45

Tyr Tyr Thr Ser Arg Leu His Ser Gly Val Pro Ser Arg Phe Ser Gly
    50                  55                  60

Ser Gly Ser Gly Thr Asp Tyr Ser Leu Thr Ile Ser Ser Leu Glu Gln
65                  70                  75                  80

Glu Asp Ile Ala Thr Tyr Phe Cys Gln Gln Ala His Thr Leu Pro Trp
                85                  90                  95

Thr Phe Gly Gly Gly Thr Lys Leu Glu Ile Lys
            100                 105

<210>  17
<211>  321
<212>  DNA
<213>  Artificial sequence

<220>
<223>  mAb053 VL

<400>  17
gatatccaga tgacacagac tacatcctcc ctgtctgcct ctctgggaga cagagtcacc      60

atcagttgca gggcaagtca ggacattagc aattatttaa actggtatca gcagagacca     120

gatggaactg ttaaacgcct aatctactac acatcaagat tacactcagg agtcccatca     180

aggttcagtg gcagtgggtc tggaacagat tattctctca ccattagcag cctggagcaa     240

gaagacattg ccacttactt ttgccaacag gctcatacgc ttccgtggac gttcggtgga     300

ggcaccaagt tggagatcaa a                                               321


<210>  18
<211>  11
<212>  PRT
<213>  Artificial sequence

<220>
<223>  mAb053 VL-CDR1

<400>  18

Arg Ala Ser Gln Asp Ile Ser Asn Tyr Leu Asn
1               5                   10


<210>  19
<211>  7
<212>  PRT
<213>  Artificial sequence

<220>
<223>  mAb053 VL-CDR2

<400>  19

Tyr Thr Ser Arg Leu His Ser
1               5


<210>  20
<211>  9
<212>  PRT
<213>  Artificial sequence

<220>
<223>  mAb053 VL-CDR3

<400>  20

Gln Gln Ala His Thr Leu Pro Trp Thr
1               5


<210>  21
<211>  112
<212>  PRT
<213>  Artificial sequence

<220>

<223>  mAb041 VH

<400>  21

Glu Val Gln Leu Val Glu Ser Gly Gly Gly Leu Val Lys Pro Gly Gly
1               5                   10                  15

Ser Leu Lys Leu Ser Cys Ala Val Ser Gly Phe Thr Phe Ser Asn Ser
            20                  25                  30

Ala Met Ser Trp Val Arg Gln Thr Pro Glu Lys Arg Leu Glu Trp Val
        35                  40                  45

Ala Thr Ile Thr Asp Gly Gly Asp Tyr Thr Tyr Tyr Ser Asp Asn Val
    50                  55                  60

Lys Gly Arg Phe Thr Ile Ser Arg Asp Asn Ala Lys Asn Asn Leu Tyr
65                  70                  75                  80

Leu Gln Met Ser His Leu Lys Ser Glu Asp Thr Ala Met Tyr Tyr Cys
                85                  90                  95

Val Arg Gly Ile Ser Tyr Trp Gly Gln Gly Thr Leu Val Thr Val Ser
            100                 105                 110

<210>  22
<211>  339
<212>  DNA
<213>  Artificial sequence

<220>
<223>  mAb041 VH

<400>  22
gaagtgcagc tggtggagtc tgggggaggc ttagtgaagc ctggagggtc cctgaaactc      60

tcctgtgcag tctctggatt cactttcagt aactctgcca tgtcttgggt tcgccagact     120

ccggaaaaga ggctggagtg ggtcgcaacc attactgatg gtggtgatta cacctactat     180

tcagacaatg taaagggccg attcaccatc tccagagaca atgccaagaa caacctgtac     240

ctgcaaatga gccatctgaa gtctgaggac acagccatgt attactgtgt aagagggatt     300

tcttactggg gccaagggac tctggtcact gtctctgca                            339

<210>  23
<211>  5
<212>  PRT
<213>  Artificial sequence

<220>
<223>  mAb041 VH-CDR1

<400>  23

Asn Ser Ala Met Ser
1               5

<210>  24
<211>  17
<212>  PRT
<213>  Artificial sequence

<220>
<223>  mAb041 VH-CDR2

<400>  24

Thr Ile Thr Asp Gly Gly Asp Tyr Thr Tyr Tyr Ser Asp Asn Val Lys
1               5                   10                  15

Gly

<210>  25
<211>  4
<212>  PRT
<213>  Artificial sequence

<220>
<223>  mAb041 VH-CDR3

<400>  25

Gly Ile Ser Tyr
1

<210>  26
<211>  111
<212>  PRT
<213>  Artificial sequence

<220>
<223>  mAb041 VL

<400>  26

Asp Ile Val Leu Thr Gln Ser Pro Ala Ser Leu Ala Val Ser Leu Gly
1               5                   10                  15

Gln Arg Ala Thr Ile Ser Cys Arg Ala Ser Lys Ser Val Ser Thr Ser
            20                  25                  30

Ser Asp Ser Tyr Met His Trp Tyr Gln Gln Lys Pro Gly Gln Pro Pro
        35                  40                  45

Lys Leu Leu Ile Lys Tyr Ala Ser Asn Leu Glu Ser Gly Val Pro Val
    50                  55                  60

Arg Phe Ser Gly Ser Gly Ser Gly Thr Asp Phe Thr Leu Asn Ile His
65                  70                  75                  80

Pro Val Glu Glu Glu Asp Ala Ala Thr Tyr Tyr Cys Gln His Ser Arg
                85                  90                  95

Glu Phe Pro Trp Thr Phe Gly Gly Gly Thr Lys Leu Glu Ile Lys
            100                 105                 110

<210>  27
<211>  333
<212>  DNA
<213>  Artificial sequence

<220>
<223>  mAb041 VL

<400>  27
gacattgtgc tgacacagtc tcctgcttcc ttagctgtat ctctggggca gagggccacc      60

atctcctgca gggccagcaa aagtgtcagt acatctagcg atagttacat gcactggtac      120

caacagaaac caggacagcc gcccaaactc ctcatcaagt atgcatccaa cctagaatct      180

ggggtccctg tcaggttcag tggcagtggg tctgggacag acttcaccct caacatccat      240

cctgtggagg aggaggatgc tgcaacatat tattgtcagc acagtaggga gtttccgtgg      300

acgttcggtg gaggcaccaa gctggaaatc aaa                                   333

<210>  28
<211>  15
<212>  PRT
<213>  Artificial sequence

<220>
<223>  mAb041 VL-CDR1

<400>  28

Arg Ala Ser Lys Ser Val Ser Thr Ser Ser Asp Ser Tyr Met His
1               5                   10                  15

<210>  29
<211>  7
<212>  PRT
<213>  Artificial sequence

<220>
<223>  mAb041 VL-CDR2

<400>  29

Tyr Ala Ser Asn Leu Glu Ser
1               5

<210>  30

<211>  9
<212>  PRT
<213>  Artificial sequence

<220>
<223>  mAb041 VL-CDR3

<400>  30

Gln His Ser Arg Glu Phe Pro Trp Thr
1               5


<210>  31
<211>  116
<212>  PRT
<213>  Artificial sequence

<220>
<223>  mAb058 VH

<400>  31

Glu Val Gln Leu Gln Gln Ser Gly Pro Val Leu Val Lys Pro Gly Ala
1               5                   10                  15


Ala Val Lys Met Ser Cys Lys Ala Ser Gly Tyr Ile Phe Thr Asp Tyr
            20                  25                  30


Tyr Ile His Trp Val Lys Leu Ser Arg Gly Glu Ser Leu Glu Trp Ile
        35                  40                  45


Gly Leu Ile Asn Pro Tyr Asn Gly Gly Thr Asn Tyr Asn Gln Gln Phe
    50                  55                  60


Thr Gly Lys Ala Thr Leu Thr Val Asp Lys Ser Ser Ser Thr Ala Tyr
65                  70                  75                  80


Val Glu Leu Asn Ser Leu Thr Ser Gly Asp Ser Ala Val Tyr Tyr Cys
                85                  90                  95


Val Arg Arg Thr Gly Tyr Phe Asp Tyr Trp Gly Gln Gly Thr Thr Leu
            100                 105                 110


Thr Val Ser Ser
        115


<210>  32
<211>  348
<212>  DNA
<213>  Artificial sequence

<220>
<223>  mAb058 VH

<400>  32
gaggtccagc tgcaacagtc tggacctgta ctggtgaagc ctggggctgc agtgaagatg      60

tcctgtaagg cttctggata catattcact gactactata tacactgggt gaagctgagc     120

cgtggagaga gccttgagtg gattggactt attaatcctt acaacggtgg tactaactac     180

aaccaacagt tcacgggcaa ggccacattg actgttgaca agtcctccag cacagcctac     240

gtggagctca acagcctgac atctggggac tctgcagtct attattgtgt acggaggact     300

ggatactttg actactgggg ccaaggcacc actctcacag tctcctca                  348

<210>  33
<211>  5
<212>  PRT
<213>  Artificial sequence

<220>
<223>  mAb058 VH-CDR1

<400>  33

Asp Tyr Tyr Ile His
1               5

<210>  34
<211>  17
<212>  PRT
<213>  Artificial sequence

<220>
<223>  mAb058 VH-CDR2

<400>  34

Leu Ile Asn Pro Tyr Asn Gly Gly Thr Asn Tyr Asn Gln Gln Phe Thr
1               5                   10                  15

Gly

<210>  35
<211>  7
<212>  PRT
<213>  Artificial sequence

<220>
<223>  mAb058 VH-CDR3

<400>  35

Arg Thr Gly Tyr Phe Asp Tyr
1               5

<210>  36
<211>  112
<212>  PRT

<213>  Artificial sequence

<220>
<223>  mAb058 VL

<400>  36

Asp Val Val Met Thr Gln Thr Pro Leu Ser Leu Pro Val Ser Leu Gly
1               5                   10                  15

Asp Gln Ala Ser Ile Ser Cys Arg Ser Ser Gln Ser Leu Val His Ser
            20                  25                  30

Asn Gly Asn Thr Tyr Leu His Trp Tyr Leu Gln Lys Pro Gly Gln Ser
        35                  40                  45

Pro Lys Leu Leu Ile Tyr Lys Val Ser Asn Arg Phe Ser Gly Val Pro
    50                  55                  60

Asp Arg Phe Ser Gly Ser Gly Ser Gly Thr Asp Phe Thr Leu Lys Ile
65                  70                  75                  80

Ser Arg Val Glu Ala Glu Asp Leu Gly Val Tyr Phe Cys Ser Gln Thr
                85                  90                  95

Thr His Val Pro Tyr Thr Phe Gly Gly Gly Thr Lys Leu Glu Ile Lys
            100                 105                 110

<210>  37
<211>  336
<212>  DNA
<213>  Artificial sequence

<220>
<223>  mAb058 VL

<400>  37
gatgttgtga tgacccaaac tccactctcc ctgcctgtca gtcttggaga tcaagcctcc      60

atctcttgca gatctagtca gagccttgta cacagtaatg gaaacaccta tttacattgg     120

tacctgcaga agccaggcca gtctccaaag ctcctgatct acaaagtttc caaccgattt     180

tctggggtcc ccgacaggtt cagtggcagt ggatcaggga cagatttcac actcaagatc     240

agcagagtgg aggctgagga tctgggagtt tatttctgct ctcaaactac acatgttccg     300

tacacgttcg gaggggggac caagctggaa ataaaa                               336

<210>  38
<211>  16
<212>  PRT
<213>  Artificial sequence

<220>

<223>  mAb058 VL-CDR1

<400>  38

Arg Ser Ser Gln Ser Leu Val His Ser Asn Gly Asn Thr Tyr Leu His
1               5                   10                  15


<210>  39
<211>  7
<212>  PRT
<213>  Artificial sequence

<220>
<223>  mAb058 VL-CDR2

<400>  39

Lys Val Ser Asn Arg Phe Ser
1               5


<210>  40
<211>  9
<212>  PRT
<213>  Artificial sequence

<220>
<223>  mAb058 VL-CDR3

<400>  40

Ser Gln Thr Thr His Val Pro Tyr Thr
1               5


<210>  41
<211>  119
<212>  PRT
<213>  Artificial sequence

<220>
<223>  mAb034 VH

<400>  41

Glu Val Gln Leu Gln Gln Ser Gly Pro Val Leu Val Lys Pro Gly Ala
1               5                   10                  15


Ser Val Lys Met Ser Cys Lys Ala Ser Gly Tyr Thr Phe Thr Asp Phe
            20                  25                  30


Tyr Met Asn Trp Met Arg Gln Ser His Gly Lys Ser Leu Glu Trp Ile
        35                  40                  45


Gly Val Ile Asp Pro Tyr Asn Gly Asn Thr Ser Tyr Asn Gln Lys Phe
    50                  55                  60


Lys Gly Lys Ala Thr Leu Thr Val Asp Lys Ser Ser Ser Thr Ala Tyr

65                  70                  75                  80

Met Glu Leu Asn Ser Leu Thr Ser Glu Asp Ser Ala Val Tyr Tyr Cys
                85                  90                  95

Ala Arg Gly Asn Tyr Tyr Tyr Tyr Ala Met Asp Tyr Trp Gly Gln Gly
            100                 105                 110

Thr Ser Val Thr Val Ser Ser
        115

<210>  42
<211>  357
<212>  DNA
<213>  Artificial sequence

<220>
<223>  mAb034 VH

<400>  42
gaggtccagc tgcaacagtc tggacctgtg ctggtgaagc ctggggcttc agtgaagatg      60

tcctgtaagg cttctggata cacattcact gacttctata tgaactggat gaggcagagc     120

catggaaaga gccttgagtg gattggagtt attgatcctt acaacggtaa tactagctac     180

aaccagaagt tcaagggcaa ggccacattg actgttgaca agtcctccag cacagcctac     240

atggagctca acagcctgac atctgaggac tctgcagtct attactgtgc aagaggtaac     300

tattattact atgctatgga ctactggggt caaggaactt cagtcaccgt ctcctca        357

<210>  43
<211>  5
<212>  PRT
<213>  Artificial sequence

<220>
<223>  mAb034 VH-CDR1

<400>  43

Asp Phe Tyr Met Asn
1               5

<210>  44
<211>  17
<212>  PRT
<213>  Artificial sequence

<220>
<223>  mAb034 VH-CDR2

<400>  44

Val Ile Asp Pro Tyr Asn Gly Asn Thr Ser Tyr Asn Gln Lys Phe Lys
1               5                   10                  15

Gly

<210> 45
<211> 10
<212> PRT
<213> Artificial sequence

<220>
<223> mAb034 VH-CDR3

<400> 45

Gly Asn Tyr Tyr Tyr Tyr Ala Met Asp Tyr
1               5                   10

<210> 46
<211> 111
<212> PRT
<213> Artificial sequence

<220>
<223> mAb034 VL

<400> 46

Asp Ile Val Leu Thr Gln Ser Pro Ala Ser Leu Ala Val Ser Leu Gly
1               5                   10                  15

Gln Arg Ala Thr Ile Ser Cys Arg Ala Ser Glu Ser Val Ser Ile His
            20                  25                  30

Gly Thr His Leu Met Tyr Trp Tyr Gln Gln Lys Pro Gly Gln Pro Pro
        35                  40                  45

Lys Leu Leu Ile Tyr Ala Ala Ser Asn Leu Glu Ser Gly Val Pro Ala
    50                  55                  60

Arg Phe Ser Gly Ser Gly Ser Glu Thr Asp Phe Thr Leu Asn Ile His
65                  70                  75                  80

Pro Val Glu Glu Glu Asp Ala Ala Thr Phe Phe Cys Gln Gln Ser Ile
                85                  90                  95

Glu Asp Pro Pro Thr Phe Gly Gly Gly Thr Lys Leu Glu Ile Lys
            100                 105                 110

<210> 47
<211> 333
<212> DNA
<213> Artificial sequence

<220>
<223>  mAb034 VL

<400>  47
gacattgtgc tgacccaatc tccagcttct ttggctgtgt ctctagggca gagggccacc      60

atctcctgca gagccagtga aagtgtcagt attcatggta ctcatttaat gtactggtac     120

caacagaaac caggacagcc acccaaactc ctcatctatg ctgcatccaa cctagaatct     180

ggagtccctg ccaggttcag tggcagtggg tctgagacag acttcaccct caacatccat     240

cctgtggagg aggaggatgc tgcaaccttt ttctgtcagc aaagtattga ggatcctccc     300

acgttcggag gggggaccaa gctggaaata aaa                                  333


<210>  48
<211>  15
<212>  PRT
<213>  Artificial sequence

<220>
<223>  mAb034 VL-CDR1

<400>  48

Arg Ala Ser Glu Ser Val Ser Ile His Gly Thr His Leu Met Tyr
1               5                   10                  15


<210>  49
<211>  7
<212>  PRT
<213>  Artificial sequence

<220>
<223>  mAb034 VL-CDR2

<400>  49

Ala Ala Ser Asn Leu Glu Ser
1               5


<210>  50
<211>  9
<212>  PRT
<213>  Artificial sequence

<220>
<223>  mAb034 VL-CDR3

<400>  50

Gln Gln Ser Ile Glu Asp Pro Pro Thr
1               5


<210>  51
<211>  117
<212>  PRT
<213>  Artificial sequence

<220>
<223>  mAb049 VH

<400>  51

Glu Val Gln Leu Gln Gln Ser Gly Pro Glu Leu Val Lys Pro Gly Ala
1               5                   10                  15

Ser Val Lys Ile Ser Cys Lys Ala Ser Gly His Thr Leu Thr Asp Tyr
            20                  25                  30

Tyr Met Asn Trp Val Lys Gln Ser His Gly Lys Ser Pro Glu Trp Ile
        35                  40                  45

Gly Asp Ile Asn Pro Asn Asn Asp Gly Ile Asp Tyr Asn Gln Lys Phe
    50                  55                  60

Arg Gly Lys Ala Ile Leu Thr Val Asp Lys Ser Ser Ser Ser Phe Tyr
65                  70                  75                  80

Met Glu Leu Arg Ser Leu Thr Ser Glu Asp Ser Ala Val Tyr Tyr Cys
                85                  90                  95

Val Gly Phe Val Arg Gly Tyr Phe Val Ser Trp Gly Gln Gly Thr Thr
            100                 105                 110

Leu Thr Val Ser Ser
        115

<210>  52
<211>  351
<212>  DNA
<213>  Artificial sequence

<220>
<223>  mAb049 VH

<400>  52
gaggtccagc tgcaacaatc tggacctgag ctggtgaagc ctggggcttc agtgaagatt      60

tcctgtaagg cttctggaca cacgctcact gactactaca tgaactgggt gaagcagagc     120

catggaaaga gccctgagtg gattggagat attaatccta ataatgatgg tattgactac     180

aaccagaagt tcaggggcaa ggccatattg actgtagaca agtcctccag ctcattttat     240

atggaactcc gcagtctgac atctgaggac tctgcagtct attattgtgt ggggttcgta     300

cgaggctact ttgtctcctg gggccaaggc accactctca cagtctcctc a              351

<210>  53
<211>  5
<212>  PRT

<213>  Artificial sequence

<220>
<223>  mAb049 VH-CDR1

<400>  53

Asp Tyr Tyr Met Asn
1               5


<210>  54
<211>  17
<212>  PRT
<213>  Artificial sequence

<220>
<223>  mAb049 VH-CDR2

<400>  54

Asp Ile Asn Pro Asn Asn Asp Gly Ile Asp Tyr Asn Gln Lys Phe Arg
1               5                   10                  15


Gly




<210>  55
<211>  8
<212>  PRT
<213>  Artificial sequence

<220>
<223>  mAb049 VH-CDR3

<400>  55

Phe Val Arg Gly Tyr Phe Val Ser
1               5


<210>  56
<211>  106
<212>  PRT
<213>  Artificial sequence

<220>
<223>  mAb049 VL

<400>  56

Asp Ile Gln Met Thr Gln Thr Thr Ser Ser Leu Ser Ala Ser Leu Gly
1               5                   10                  15


Asp Arg Val Thr Ile Asn Cys Arg Ala Ser Gln Asp Ile Ser Asn Tyr
            20                  25                  30


Leu Asn Trp Tyr Gln Gln Lys Pro Asp Gly Thr Val Lys Leu Leu Ile
        35                  40                  45

Tyr Tyr Thr Ser Arg Leu Arg Ser Gly Val Pro Ser Arg Phe Ser Gly
    50                  55                  60

Ser Gly Ser Gly Thr Asp Tyr Ser Leu Thr Ile Asn Asn Leu Asp Gln
65                  70                  75                  80

Glu Asp Phe Ala Thr Tyr Phe Cys Gln Gln Gly Asn Thr Leu Pro Trp
                85                  90                  95

Thr Phe Gly Gly Gly Thr Lys Leu Glu Ile
            100                 105

<210>  57
<211>  321
<212>  DNA
<213>  Artificial sequence

<220>
<223>  mAb049 VL

<400>  57
gatatccaga tgacacagac tacatcctcc ctgtctgcct ctctgggaga cagagtcacc      60

atcaattgca gggcaagtca ggacattagc aattatttaa actggtatca gcagaaacca     120

gatggaactg ttaaactcct gatctactac acatcaagat tacgctcagg agtcccatca     180

aggttcagtg gcagtgggtc tggaacagat tattctctta ccattaacaa cctggaccaa     240

gaagattttg ccacttactt ttgtcaacag ggtaatacgc ttccgtggac gttcggtgga     300

ggcaccaagc tggaaatcaa a                                               321

<210>  58
<211>  11
<212>  PRT
<213>  Artificial sequence

<220>
<223>  mAb049 VL-CDR1

<400>  58

Arg Ala Ser Gln Asp Ile Ser Asn Tyr Leu Asn
1               5                   10

<210>  59
<211>  7
<212>  PRT
<213>  Artificial sequence

<220>
<223>  mAb049 VL-CDR2

<400>  59

Tyr Thr Ser Arg Leu Arg Ser
1               5

<210>  60
<211>  9
<212>  PRT
<213>  Artificial sequence

<220>
<223>  mAb049 VL-CDR3

<400>  60

Gln Gln Gly Asn Thr Leu Pro Trp Thr
1               5

<210>  61
<211>  116
<212>  PRT
<213>  Artificial sequence

<220>
<223>  mAb064 VH

<400>  61

Gln Val Gln Leu Lys Glu Ser Gly Pro Gly Leu Val Ala Pro Ser Gln
1               5                   10                  15

Thr Leu Ser Ile Thr Cys Ser Val Ser Gly Phe Ser Leu Thr Asn Tyr
            20                  25                  30

Ala Val Ser Trp Val Arg Gln Pro Pro Gly Lys Gly Leu Glu Trp Leu
        35                  40                  45

Gly Val Met Trp Gly Asp Gly Asn Thr Asn Tyr His Ser Thr Leu Ile
    50                  55                  60

Ser Arg Leu Ser Ile Ser Lys Asp Asn Ser Lys Ser Gln Val Phe Leu
65                  70                  75                  80

Lys Leu Asn Ser Leu Gln Thr Asp Asp Thr Ala Thr Tyr Tyr Cys Ala
                85                  90                  95

Arg Ser Ser Gly Trp Tyr Phe Asp Val Trp Gly Thr Gly Thr Thr Val
            100                 105                 110

Thr Val Ser Ser
        115

<210>  62
<211>  348

<212>  DNA
<213>  Artificial sequence

<220>
<223>  mAb064 VH

<400>  62
caggtgcagc tgaaggagtc aggacctggc ctggtggcgc cctcacagac cctgtccatc      60

acatgctctg tctcaggttt ctcattaacc aactatgctg taagctgggt tcgccagcct     120

ccaggaaagg gtctggagtg gctgggagta atgtggggtg acgggaacac aaattatcat     180

tcaactctca tatccagact gagcattagt aaggataact ccaagagcca agttttctta     240

aaactgaaca gtctacaaac tgatgacaca gccacgtact actgtgcccg tagtagcggc     300

tggtacttcg atgtctgggg cacagggacc acggtcaccg tctcctca                  348


<210>  63
<211>  5
<212>  PRT
<213>  Artificial sequence

<220>
<223>  mAb064 VH-CDR1

<400>  63

Asn Tyr Ala Val Ser
1               5


<210>  64
<211>  16
<212>  PRT
<213>  Artificial sequence

<220>
<223>  mAb064 VH-CDR2

<400>  64

Val Met Trp Gly Asp Gly Asn Thr Asn Tyr His Ser Thr Leu Ile Ser
1               5                   10                  15


<210>  65
<211>  8
<212>  PRT
<213>  Artificial sequence

<220>
<223>  mAb064 VH-CDR3

<400>  65

Ser Ser Gly Trp Tyr Phe Asp Val
1               5


<210>  66

<211>  107
<212>  PRT
<213>  Artificial sequence

<220>
<223>  mAb064 VL

<400>  66

Asp Ile Gln Met Thr Gln Thr Thr Pro Ser Leu Ser Val Thr Leu Gly
1               5                   10                  15

Asp Arg Val Thr Ile Arg Cys Arg Ala Ser Gln Asp Ile Asn Asn Tyr
            20                  25                  30

Leu Asn Trp Tyr Gln Gln Lys Pro Asp Gly Thr Val Lys Leu Leu Ile
        35                  40                  45

Tyr Tyr Thr Ser Glu Ser Arg Ser Gly Val Pro Ser Arg Phe Ser Gly
    50                  55                  60

Ser Gly Ser Gly Thr Asp Tyr Ser Leu Thr Ile Ser Asn Leu Glu Gln
65                  70                  75                  80

Glu Asp Ile Ala Thr Tyr Phe Cys Gln Gln Gly Tyr Ala Leu Pro Pro
                85                  90                  95

Ala Phe Gly Gly Gly Thr Lys Leu Glu Ile Lys
            100                 105


<210>  67
<211>  321
<212>  DNA
<213>  Artificial sequence

<220>
<223>  mAb064 VL

<400>  67
gatatccaga tgacacagac tacaccctcc ctgtctgtga ctctgggaga cagagtcacc      60

atcagatgca gggcaagtca ggacattaac aattatttaa attggtatca gcagaaacca     120

gatggaactg ttaaactcct gatctactat acatcagaat cacgttcagg agtcccatca     180

aggttcagtg gcagtgggtc tggaacagat tattctctca ccattagcaa cctggaacaa     240

gaagatattg ccacttactt ttgccaacag ggttatgcgc ttcctccggc gttcggtgga     300

ggcaccaagc tggaaataaa a                                               321


<210>  68
<211>  11
<212>  PRT
<213>  Artificial sequence

<220>
<223>  mAb064 VL-CDR1

<400>  68

Arg Ala Ser Gln Asp Ile Asn Asn Tyr Leu Asn
1               5                   10


<210>  69
<211>  7
<212>  PRT
<213>  Artificial sequence

<220>
<223>  mAb064 VL-CDR2

<400>  69

Tyr Thr Ser Glu Ser Arg Ser
1               5


<210>  70
<211>  9
<212>  PRT
<213>  Artificial sequence

<220>
<223>  mAb064 VL-CDR3

<400>  70

Gln Gln Gly Tyr Ala Leu Pro Pro Ala
1               5


<210>  71
<211>  118
<212>  PRT
<213>  Artificial sequence

<220>
<223>  mAb033 VH

<400>  71

Gln Val Gln Leu Gln Gln Ser Gly Ala Glu Leu Val Arg Pro Gly Thr
1               5                   10                  15


Ala Val Arg Val Ser Cys Lys Ala Ser Gly Tyr Ala Phe Thr Asn Tyr
            20                  25                  30


Phe Ile Glu Trp Val Lys Gln Arg Pro Gly Gln Gly Leu Glu Trp Ile
        35                  40                  45


Gly Val Ile Asn Pro Gly Ser Gly Asp Thr Tyr Tyr Ser Glu Lys Phe
    50                  55                  60

Gln Gly Lys Ala Thr Leu Thr Ala Asp Lys Ser Ser Ser Thr Ala Tyr
65                  70                  75                  80

Met Gln Leu Ser Ser Leu Thr Ser Glu Asp Ser Ala Val Tyr Phe Cys
                85                  90                  95

Ala Arg Glu Asp Asp Tyr Ser Trp Phe Thr Tyr Trp Gly Gln Gly Thr
            100                 105                 110

Leu Val Thr Val Ser Ala
        115

<210>  72
<211>  354
<212>  DNA
<213>  Artificial sequence

<220>
<223>  mAb033 VH

<400>  72
caggtccagc tgcagcagtc tggagctgag ttggtaaggc ctgggactgc agtgagggtg      60

tcctgcaagg cttctggata cgccttcact aattacttta tagagtgggt aaagcagagg     120

cctggacagg gccttgagtg gattggagtg attaatcctg gaagtggtga tacttactac     180

agtgagaagt tccagggcaa ggcaacactg actgcagaca aatcctccag cactgcctac     240

atgcagctca gcagtctgac atctgaggac tctgcggtct atttctgtgc aagagaggat     300

gattactcct ggtttactta ctggggccaa gggactctgg tcactgtctc tgca           354

<210>  73
<211>  5
<212>  PRT
<213>  Artificial sequence

<220>
<223>  mAb033 VH-CDR1

<400>  73

Asn Tyr Phe Ile Glu
1               5

<210>  74
<211>  17
<212>  PRT
<213>  Artificial sequence

<220>
<223>  mAb033 VH-CDR2

<400>  74

Val Ile Asn Pro Gly Ser Gly Asp Thr Tyr Tyr Ser Glu Lys Phe Gln
1               5                   10                  15

Gly

<210>  75
<211>  9
<212>  PRT
<213>  Artificial sequence

<220>
<223>  mAb033 VH-CDR3

<400>  75

Glu Asp Asp Tyr Ser Trp Phe Thr Tyr
1               5

<210>  76
<211>  111
<212>  PRT
<213>  Artificial sequence

<220>
<223>  mAb033 VL

<400>  76

Asp Ile Val Leu Thr Gln Ser Pro Ala Ser Leu Ala Val Ser Leu Gly
1               5                   10                  15

Gln Arg Ala Thr Ile Ser Cys Arg Ala Ser Glu Asn Val Asp Asn Tyr
            20                  25                  30

Gly Ile Ser Phe Met His Trp Tyr Arg Gln Lys Pro Gly Gln Pro Pro
        35                  40                  45

Lys Leu Leu Ile Tyr Arg Ala Ser Asn Leu Glu Ser Gly Ile Pro Ala
    50                  55                  60

Arg Phe Ser Gly Ser Gly Ser Arg Thr Asp Phe Thr Leu Thr Ile Asn
65                  70                  75                  80

Pro Val Glu Thr Asp Asp Val Ala Thr Tyr Tyr Cys Gln Gln Ser Asn
                85                  90                  95

Lys Asp Pro Tyr Thr Phe Gly Gly Gly Thr Lys Leu Glu Ile Lys
            100                 105                 110

<210>  77
<211>  333
<212>  DNA

<213>  Artificial sequence

<220>
<223>  mAb033 VL

<400>  77
gacattgtgc tgacccaatc tccagcttct ttggctgtgt ctctagggca gagggccacc      60

atctcctgca gagccagcga aaatgttgat aattatggca ttagttttat gcactggtac      120

cggcagaaac caggacagcc acccaaactc ctcatctatc gtgcatccaa cctagaatct      180

gggatccctg ccaggttcag tggcagtggg tctaggacag acttcaccct caccattaat      240

cctgtggaga ctgatgatgt tgcaacctat tactgtcagc aaagtaataa ggatccgtac      300

acgttcggag gggggaccaa actggaaata aaa                                   333


<210>  78
<211>  15
<212>  PRT
<213>  Artificial sequence

<220>
<223>  mAb033 VL-CDR1

<400>  78

Arg Ala Ser Glu Asn Val Asp Asn Tyr Gly Ile Ser Phe Met His
1               5                   10                  15


<210>  79
<211>  7
<212>  PRT
<213>  Artificial sequence

<220>
<223>  mAb033 VL-CDR2

<400>  79

Arg Ala Ser Asn Leu Glu Ser
1               5


<210>  80
<211>  9
<212>  PRT
<213>  Artificial sequence

<220>
<223>  mAb033 VL-CDR3

<400>  80

Gln Gln Ser Asn Lys Asp Pro Tyr Thr
1               5


<210>  81
<211>  117

<212>  PRT
<213>  Artificial sequence

<220>
<223>  mAb047 VH

<400>  81

Glu Val Gln Leu Val Glu Ser Gly Gly Gly Leu Val Lys Pro Gly Gly
1               5                   10                  15

Ser Leu Lys Leu Ser Cys Ala Ala Ser Gly Phe Thr Phe Ser Asp Tyr
            20                  25                  30

Gly Met Tyr Trp Ile Arg Gln Ala Pro Glu Lys Gly Leu Glu Trp Val
        35                  40                  45

Ala Tyr Ile Ser Asn Gly Gly Ser Ser Ile Tyr Tyr Ala Asp Thr Val
    50                  55                  60

Lys Gly Arg Phe Thr Val Ser Arg Asp Asn Ala Lys Asn Thr Leu Phe
65                  70                  75                  80

Leu Gln Met Thr Ser Leu Arg Ser Glu Asp Thr Ala Ile Tyr Tyr Cys
                85                  90                  95

Ala Arg Gly Gly Glu Leu Gly Phe Ala Tyr Trp Gly Gln Gly Thr Leu
            100                 105                 110

Val Thr Val Ser Ala
        115


<210>  82
<211>  351
<212>  DNA
<213>  Artificial sequence

<220>
<223>  mAb047 VH

<400>  82
gaggtgcagc tggtggagtc tgggggaggc ttagtgaagc ctggagggtc cctgaaactc      60

tcctgtgcag cctctggatt cactttcagt gactatggaa tgtactggat tcgtcaggct     120

ccagagaagg ggctggagtg ggttgcatac attagtaatg gcggtagttc catctactat     180

gcagacacag tgaagggccg attcaccgtc tccagagaca atgccaagaa caccctgttc     240

ctgcaaatga ccagtctgag gtctgaggac acggccatat attactgtgc aaggggagga     300

gaactcgggt ttgcttactg gggccaaggg actctggtca ctgtctctgc a              351


<210>  83

<211>  5
<212>  PRT
<213>  Artificial sequence

<220>
<223>  mAb047 VH-CDR1

<400>  83

Asp Tyr Gly Met Tyr
1               5


<210>  84
<211>  17
<212>  PRT
<213>  Artificial sequence

<220>
<223>  mAb047 VH-CDR2

<400>  84

Tyr Ile Ser Asn Gly Gly Ser Ser Ile Tyr Tyr Ala Asp Thr Val Lys
1               5                   10                  15


Gly



<210>  85
<211>  8
<212>  PRT
<213>  Artificial sequence

<220>
<223>  mAb047 VH-CDR3

<400>  85

Gly Gly Glu Leu Gly Phe Ala Tyr
1               5


<210>  86
<211>  105
<212>  PRT
<213>  Artificial sequence

<220>
<223>  mAb047 VL

<400>  86

Asp Ile Gln Met Thr Gln Ser Pro Ala Ser Leu Ser Val Ser Val Gly
1               5                   10                  15


Glu Thr Val Thr Ile Thr Cys Arg Ala Ser Glu Asn Ile Tyr Ser Asn
            20                  25                  30

Leu Ala Trp Tyr Gln Gln Lys Gln Gly Lys Ser Pro Gln Leu Leu Val
        35                  40                  45

Tyr Ala Ala Ile Asn Leu Val Glu Gly Val Pro Ser Arg Phe Ser Gly
    50                  55                  60

Ser Gly Ser Gly Thr Gln Tyr Ser Leu Lys Ile Asn Ser Leu Gln Ser
65                  70                  75                  80

Glu Asp Phe Gly Ser Tyr Phe Cys Gln His Phe Trp Gly Leu Pro Tyr
                85                  90                  95

Thr Phe Gly Gly Gly Thr Lys Leu Glu
            100                 105

<210>  87
<211>  321
<212>  DNA
<213>  Artificial sequence

<220>
<223>  mAb047 VL

<400>  87
gacatccaga tgactcagtc tccagcctcc ctatctgtat ctgtgggaga aactgtcacc      60

atcacgtgtc gagcaagtga aaatatttac agtaatctag catggtatca gcagaaacag      120

ggaaaatctc ctcagctcct ggtctatgct gcaataaatt tagtagaagg tgtgccatca      180

aggttcagtg gcagtggatc gggcacgcag tattccctca agatcaacag cctgcagtct      240

gaagattttg ggagttattt ctgtcaacat ttttggggtc ttccgtacac gttcggaggg      300

gggaccaagc tggaaagaaa t                                                321

<210>  88
<211>  11
<212>  PRT
<213>  Artificial sequence

<220>
<223>  mAb047 VL-CDR1

<400>  88

Arg Ala Ser Glu Asn Ile Tyr Ser Asn Leu Ala
1               5                   10

<210>  89
<211>  7
<212>  PRT
<213>  Artificial sequence

<220>
<223>  mAb047 VL-CDR2

<400>  89

Ala Ala Ile Asn Leu Val Glu
1               5


<210>  90
<211>  9
<212>  PRT
<213>  Artificial sequence

<220>
<223>  mAb047 VL-CDR3

<400>  90

Gln His Phe Trp Gly Leu Pro Tyr Thr
1               5


<210>  91
<211>  112
<212>  PRT
<213>  Artificial sequence

<220>
<223>  mAb055 VH

<400>  91

Gln Val Gln Leu Lys Gln Ser Gly Pro Gly Leu Leu Gln Pro Ser Gln
1               5                   10                  15


Ser Leu Ser Ile Thr Cys Thr Val Ser Gly Phe Ser Leu Asp Glu Tyr
            20                  25                  30


Gly Val His Trp Phe Arg Gln Ser Pro Gly Lys Gly Leu Glu Trp Leu
        35                  40                  45


Gly Val Ile Trp Ser Gly Gly Ser Thr Asp Tyr Asn Ala Ala Phe Ile
    50                  55                  60


Ser Arg Leu Ser Ile Arg Lys Asp Asn Ser Lys Ser Gln Ile Phe Phe
65                  70                  75                  80


Asn Met Asn Ser Leu Gln Ala Asp Asp Thr Ala Ile Tyr Tyr Cys Val
                85                  90                  95


Arg Glu Glu Trp Glu Tyr Trp Gly Gln Gly Thr Thr Leu Thr Val Ser
            100                 105                 110


<210>  92
<211>  339
<212>  DNA
<213>  Artificial sequence

<220>
<223>  mAb055 VH

<400>  92
caggtgcagc tgaagcagtc aggacctggc ctactgcagc cttcacagag cctgtccatc      60

acctgcacag tctctggttt ctcgttagat gagtatggtg tacattggtt tcgtcagtct     120

ccaggaaagg gtctggagtg gctgggagtg atatggagtg gtggaagcac agactacaat     180

gcagctttca tatccagact gagcatcaga aaggacaatt ccaagagcca aattttcttt     240

aatatgaaca gtctacaagc tgatgacaca gccatatatt actgtgtcag agaggaatgg     300

gaatattggg gccaaggcac aactctcaca gtctcctca                            339


<210>  93
<211>  5
<212>  PRT
<213>  Artificial sequence

<220>
<223>  mAb055 VH-CDR1

<400>  93

Glu Tyr Gly Val His
1               5


<210>  94
<211>  16
<212>  PRT
<213>  Artificial sequence

<220>
<223>  mAb055 VH-CDR2

<400>  94

Val Ile Trp Ser Gly Gly Ser Thr Asp Tyr Asn Ala Ala Phe Ile Ser
1               5                   10                  15


<210>  95
<211>  5
<212>  PRT
<213>  Artificial sequence

<220>
<223>  mAb055 VH-CDR3

<400>  95

Glu Glu Trp Glu Tyr
1               5


<210>  96
<211>  107
<212>  PRT

<213>  Artificial sequence

<220>
<223>  mAb055 VL

<400>  96

Asp Ile Gln Met Thr Gln Thr Thr Ser Ser Leu Ser Ala Ser Leu Gly
1               5                   10                  15

Asp Arg Val Thr Ile Ser Cys Arg Pro Ser Gln Asp Ile Asp Asn Tyr
            20                  25                  30

Leu Asn Trp Phe Gln Gln Arg Pro Asp Gly Thr Ile Lys Leu Leu Ile
        35                  40                  45

Tyr Tyr Thr Ser Arg Leu His Ser Gly Val Pro Ser Arg Phe Ser Gly
    50                  55                  60

Ser Gly Ser Gly Thr Asp Tyr Ser Leu Thr Ile Asn Asn Leu Glu Gln
65                  70                  75                  80

Glu Asp Ile Ala Thr Tyr Phe Cys Gln Gln Ala His Thr Leu Pro Tyr
                85                  90                  95

Thr Phe Gly Gly Gly Thr Lys Leu Glu Ile Lys
            100                 105

<210>  97
<211>  321
<212>  DNA
<213>  Artificial sequence

<220>
<223>  mAb055 VL

<400>  97
gatatccaga tgacacagac tacatcctcc ctgtctgcct ctctgggaga cagagtcacc      60

atcagttgca ggccaagtca ggacattgac aattatttaa actggtttca gcagagacca     120

gatggaacta ttaaactcct gatctattat acatcaagac tacactcagg agtcccatcg     180

aggttcagtg gcagtgggtc tggaacagat tattctctca ccattaacaa cctggaacaa     240

gaagatattg ccacttactt ttgccaacag gctcatacgc ttccgtacac gttcggaggg     300

gggaccaagc tggaaataaa a                                               321

<210>  98
<211>  11
<212>  PRT
<213>  Artificial sequence

<220>

<223>  mAb055 VL-CDR1

<400>  98

Arg Pro Ser Gln Asp Ile Asp Asn Tyr Leu Asn
1               5                   10

<210>  99
<211>  7
<212>  PRT
<213>  Artificial sequence

<220>
<223>  mAb055 VL-CDR2

<400>  99

Tyr Thr Ser Arg Leu His Ser
1               5

<210>  100
<211>  9
<212>  PRT
<213>  Artificial sequence

<220>
<223>  mAb055 VL-CDR3

<400>  100

Gln Gln Ala His Thr Leu Pro Tyr Thr
1               5

<210>  101
<211>  116
<212>  PRT
<213>  Artificial sequence

<220>
<223>  Humab035-24-VH

<400>  101

Gln Val Gln Leu Val Gln Ser Gly Ala Glu Val Lys Lys Pro Gly Ala
1               5                   10                  15

Ser Val Lys Val Ser Cys Lys Ala Ser Asp Tyr Thr Phe Thr Asp Tyr
            20                  25                  30

Glu Met His Trp Val Arg Gln Ala Pro Gly Gln Gly Leu Glu Trp Ile
        35                  40                  45

Gly Ala Ile Asp Pro Glu Thr Gly Gly Thr Ala Tyr Asn Gln Lys Phe
    50                  55                  60

Lys Asp Arg Val Thr Met Thr Ala Asp Thr Ser Thr Ser Thr Val Tyr

65                  70                  75                  80

Met Glu Leu Ser Ser Leu Arg Ser Glu Asp Thr Ala Val Tyr Tyr Cys
                85                  90                  95

Ala Lys Thr Gly Tyr Gln Phe Asp Tyr Trp Gly Gln Gly Thr Thr Val
            100                 105                 110

Thr Val Ser Ser
        115

<210>  102
<211>  348
<212>  DNA
<213>  Artificial sequence

<220>
<223>  Humab035-24-VH

<400>  102
caggtgcagc tggtgcagtc aggagcagaa gtgaagaagc caggcgcaag cgtgaaggtg        60

tcttgcaagg ccagcgacta caccttcacc gactacgaga tgcattgggt ccggcaggct       120

ccaggacagg gactcgagtg gattggcgct atcgacccag agacaggcgg cacagcctat       180

aaccagaagt tcaaggaccg cgtgaccatg accgccgata caagcaccag caccgtgtac       240

atggagctga gcagcctgag aagcgaggac accgccgtgt actattgcgc caagaccggc       300

taccagttcg actattgggg ccagggaacc acagtgacag tgtccagc                    348

<210>  103
<211>  107
<212>  PRT
<213>  Artificial sequence

<220>
<223>  Humab035-24-VL

<400>  103

Asp Ile Gln Met Thr Gln Ser Pro Ser Ser Val Ser Ala Ser Val Gly
1               5                   10                  15

Asp Arg Val Thr Ile Thr Cys Lys Ala Ser Gln Asn Val Gly Ser Asn
            20                  25                  30

Val Ala Trp Tyr Gln Gln Lys Pro Gly Lys Ala Pro Lys Ala Leu Ile
        35                  40                  45

Tyr Ser Ala Ser Tyr Arg Tyr Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Tyr Asn Ser His Pro Tyr
                85                  90                  95

Thr Phe Gly Gln Gly Thr Lys Leu Glu Ile Lys
            100                 105

<210>  104
<211>  321
<212>  DNA
<213>  Artificial sequence

<220>
<223>  Humab035-24-VL

<400>  104
gacatccaga tgacccagag ccctagcagc gtgtccgcta gcgtgggaga tagagtgacc      60

atcacttgca aggccagcca gaacgtgggc agcaacgtgg cttggtatca gcagaagcca     120

gggaaggccc ctaaggccct gatctacagc gccagctaca gatacagcgg cgtgccttct     180

agattcagcg gcagcggaag cggcaccgat ttcaccctga ccatcagcag cctgcagcca     240

gaggacttcg ccacctacta ctgccagcag tacaacagcc acccctacac cttcggacag     300

ggcaccaagc tggagatcaa g                                               321